# EUROPÄISCHE PATENTSCHRIFT

(11) **EP 1 440 066 B1**
(45) Veröffentlichungstag und Bekanntmachung des Hinweises auf die Patenterteilung: **07.03.2007**
(21) Anmeldenummer: 02772388.1
(22) Anmeldetag: 21.10.2002
(51) Int. Cl.: C07D 403/04, C07D 493/10, C07D 491/10, C07D 231/12, C07D 495/10, C07D 405/12, C07D 495/04, A01N 43/56

(54) **PYRAZOLYLSUBSTITUIERTE HETEROCYCLEN UND IHRE VERWENDUNG ALS SCHÄDLINGSBEKÄMPFUNGSMITTEL**
PYRAZOLYL-SUBSTITUTED HETEROCYCLES AND THEIR USE AS PHYTOSANITARY PRODUCTS
HETEROCYCLES A SUBSTITION PYRAZOLYLE ET LEUR UTILISATION EN TANT QUE PRODUITS PHYTOSANITAIRES

(30) Priorität: 22.10.2001 DE 10152005
(43) Veröffentlichungstag der Anmeldung: 28.07.2004
(73) Patentinhaber: Bayer CropScience AG, 40789 Monheim (DE)
(72) Erfinder: FISCHER, Reiner, 40789 Monheim (DE); ULLMANN, Astrid, 50677 Köln (DE); BRETSCHNEIDER, Thomas, 53797 Lohmar (DE); TRAUTWEIN, Axel, 51467 Bergisch Gladbach (DE); WISCHNAT, Ralf, 51061 Köln (DE); ERDELEN, Christoph, deceased (DE); FEUCHT, Dieter, 65760 Eschborn (DE); DREWES, Mark, Wilhelm, 40764 Langenfeld (DE)
(86) Internationale Anmeldenummer: PCT/EP2002/011745
(87) Internationale Veröffentlichungsnummer: WO 2003/035643

(56) Entgegenhaltungen:
- EP-A- 0 334 133
- EP-A- 0 945 437
- WO-A-00/31063
- WO-A-96/16061
- DE-A- 1 946 370
- MICHAELIS A, SCHMIDT O: "Über 1-Säurederivate des 3-Methyl- und 3-Phenyl-5-chlorpyrazols" CHEMISCHE BERICHTE, Bd. 43, 1910, Seiten 2116-2120, XP009003705 ISSN: 0009-2940

## Beschreibung

Die vorliegende Erfindung betrifft neue pyrazolylsubstituierte Heterocyclen, mehrere Verfahren zu ihrer Herstellung und ihre Verwendung als Schädlingsbekämpfungsmittel, Mikrobizide und Herbizide.

Von Thiophenderivaten, die in DE-A-195 27 190 beschrieben sind, sind insektizide und herbizide Eigenschaften bekannt.

Es wurden nun neue Verbindungen der Formel (I) gefunden 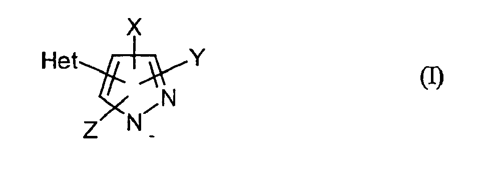 in welcher
- X: für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl steht,
- Y: für Wasserstoff, Halogen oder C₁-C₆-Alkyl steht,
- Z: für C₁-C₆-Alkyl, Halogen, Hydroxy, C₁-C₆-Alkoxy, C₁-C₆-Halogen-alkoxy oder jeweils gegebenenfalls durch C₁-C₆-Alkyl, C₁-C₆-Alkoxy, Halogen, C₁-C₄-Halogenalkyl, C₁-C₆-Halogenalkoxy, Cyano oder Nitro substituiertes Phenyl-C₁-C₂-alkoxy oder gegebenenfalls durch C₁-C₂-Alkyl oder Halogen substituiertes C₃-C₆-Cycloalkyl steht,
- Het: für eine der Gruppen 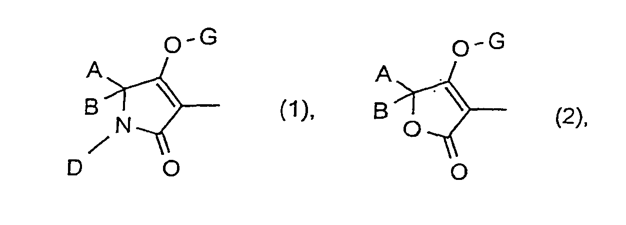 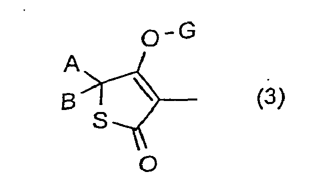 steht
- A: für Wasserstoff oder jeweils gegebenenfalls durch Fluor oder Chlor substituiertes C₁-C₁₂-Alkyl, C₃-C₈-Alkenyl, C₁-C₁₀-Alkoxy-C₁-C₈-alkyl, Poly-C₁-C₈-alkoxy-C₁-C₈-alkyl, C₁-C₁₀-Alkylthio-C₁-C₆-alkyl, gegebenenfalls durch Fluor, Chlor, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes C₆- oder C₁₀-Aryl, oder C₆- oder C₁₀-Aryl-C₁-C₆-alkyl steht,
- B: für Wasserstoff, C₁-C₁₂-Alkyl oder C₁-C₈-Akoxy-C₁-C₆-alkyl steht oder
- A, B: und das Kohlenstoffatom an das sie gebunden sind, für gesättigtes C₃-C₁₀-Cycloalkyl oder ungesättigtes C₅-C₁₀-Cycloalkyl stehen, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
- A, B: und das Kohlenstoffatom, an das sie gebunden sind, für C₅-C₆-Cycloalkyl stehen, welches durch eine gegebenenfalls ein oder zwei nicht direkt benachbarte Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet der gegebenenfalls durch C₁-C₄-Alkyl substituiert sein kann, oder
- A, B: und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
- D: für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl, C₁-C₁₀-Alkylthio-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes Phenyl, Phenyl-C₁-C₆-alkyl stehen oder
- A und D: gemeinsam für jeweils gegebenenfalls substituiertes C₃-C₆-Alkandiyl oder C₃-C₆-Alkendiyl stehen, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cycloalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder Schwefel enthalten kann,
- G: für Wasserstoff (a) oder für eine der Gruppen 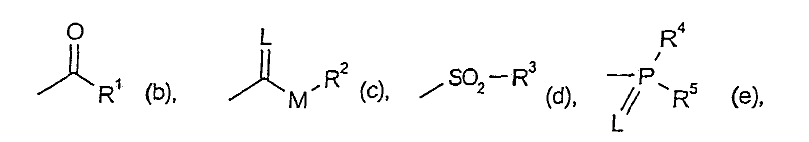 E (f) oder 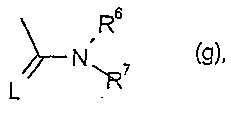 steht in welchen
- E: für ein Metallionäquivalent oder ein Ammoniumion steht,
- L: für Sauerstoff oder Schwefel steht und
- M: für Sauerstoff oder Schwefel steht,
- R¹: für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere nicht direkt benachbarte Ringglieder durch Sauerstoff und/öder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen, C₁-C₆-Alkyl, C₁-C₂-Halogenalkyl oder C₁-C₄-Alkoxy substituiertes 5- oder 6-gliedriges Hetaryl,
für gegebenenfalls durch Halogen oder C₁-C₆-Alkyl substituiertes Phenoxy-C₁-C₆-alkyl oder
für gegebenenfalls durch Halogen, Amino oder C₁-C₆-Alkyl substituiertes 5- oder 6-gliedriges Hetaryloxy-C₁-C₆-alkyl steht,
- R²: für jeweils gegebenenfalls durch Halogen substituiertes C₁-C₂₀-Alkyl, C₂-C₂₀-Alkenyl, C₁-C₈-Akoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl in welchem gegebenenfalls ein Ringatom durch Sauerstoff ersetzt ist, oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
- R³: für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenflkyl, C₁-C₄-Halogenalkoxy, Cyano oder Nitro substituiertes Phenyl oder Benzyl steht,
- R⁴ und R⁵: unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Akylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
- R⁶ und R⁷: unabhängig voneinander für Wasserstoff, für jeweils gegebenenfalls durch Halogen substituiertes C₁-C₈-Alkyl, C₃-C₈-Cycloalkyl, C₁-C₈-Alkoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Akoxy substituiertes Benzyl oder zusammen für einen gegebenenfalls durch C₁-C₄-Alkyl substituierten C₃-C₆-Alkylenrest stehen, in welchem gegebenenfalls ein Kohlenstoffatom durch Sauerstoff oder Schwefel ersetzt ist,
wobei die Verknüpfungsposition am Pyrazolylrest festgelegt wird auf folgende Strukturen:

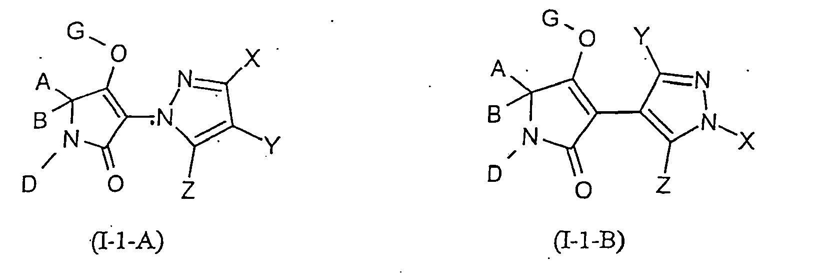

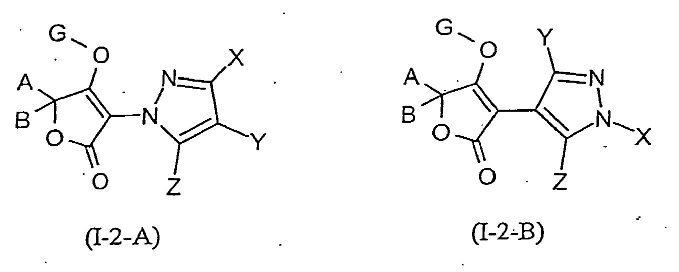

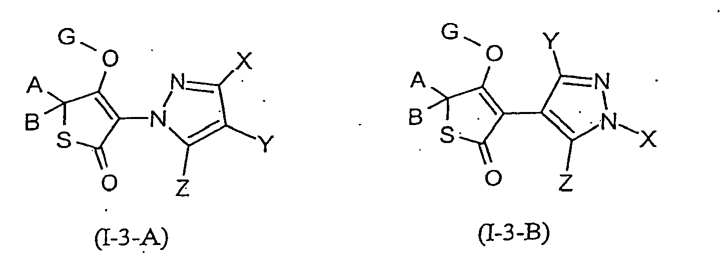

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (3) der Gruppe Het ergeben sich folgende hauptsächliche Strukturen (I-1) bis (I-3): 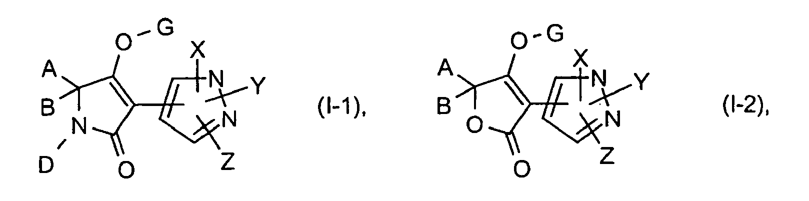 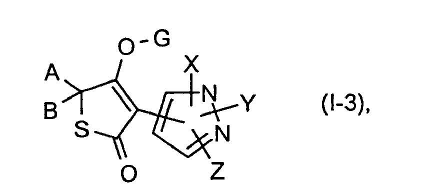 worin
A, B, D, G, X, Y und Z die oben angegebene Bedeutung haben.

Unter Einbeziehung der hauptsächlichen Strukturen (I-1) bis (I-3) und unter Berücksichtigung der möglichen Verknüpfungspositionen am Pyrazolylrest ergeben sich folgende hauptsächliche Strukturen (I-1-A) bis (I-3-B):

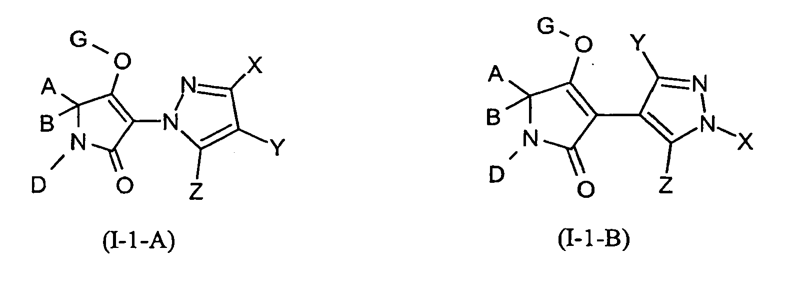

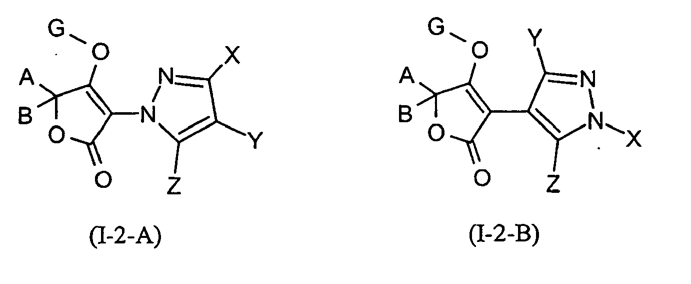

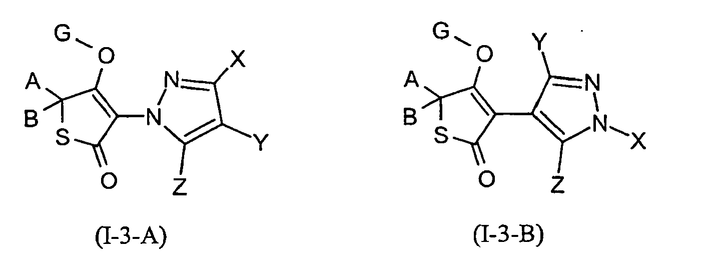

Unter Einbeziehung der verschiedenen Bedeutungen (a), (b), (c), (d), (e), (f) und (g) der Gruppe G ergeben sich folgende hauptsächliche Strukturen (I-1-A-a) bis (I-1-A-g), wenn Het für die Gruppe (1) steht, 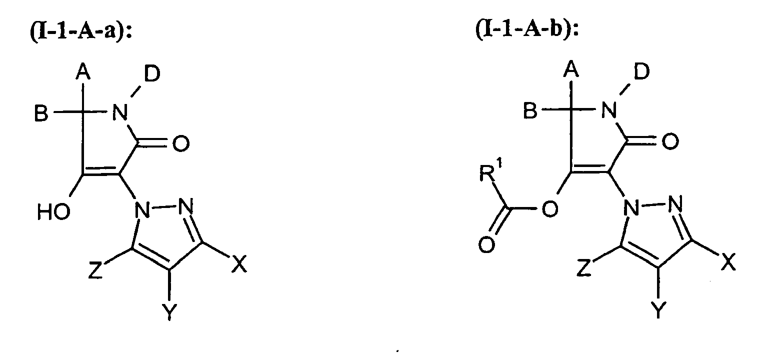 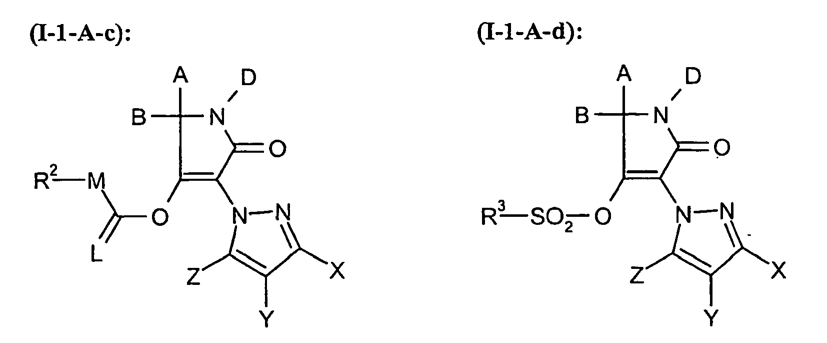 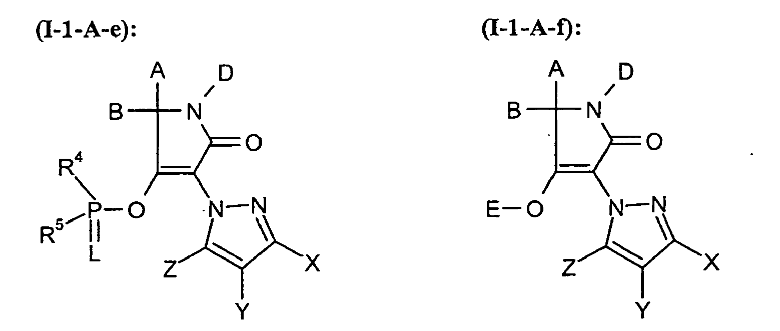 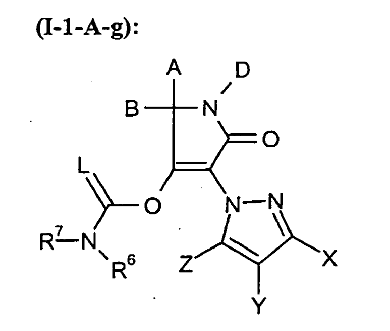 worin
A, B, D, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-1-B-a) bis (I-1-B-g), wenn Het für die Gruppe (1) steht, 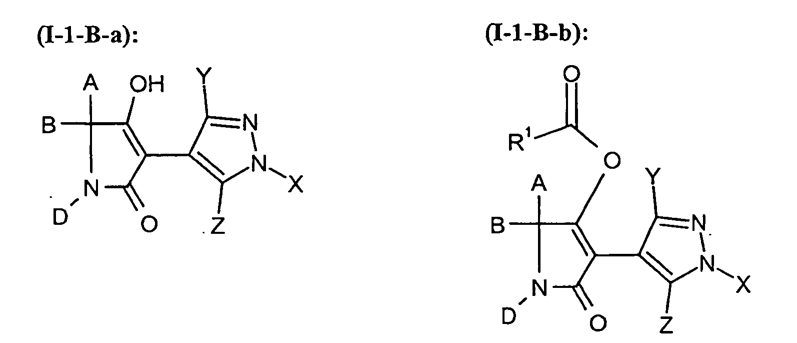 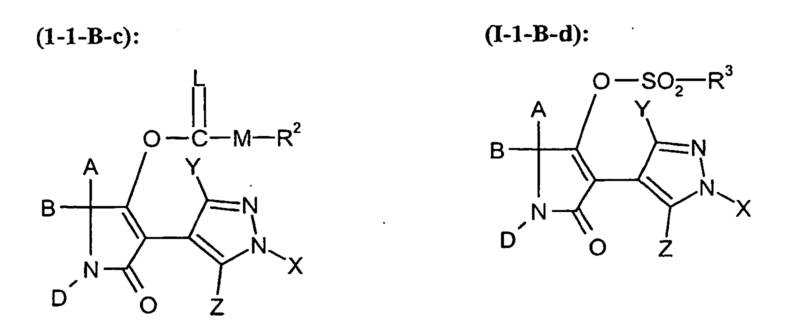 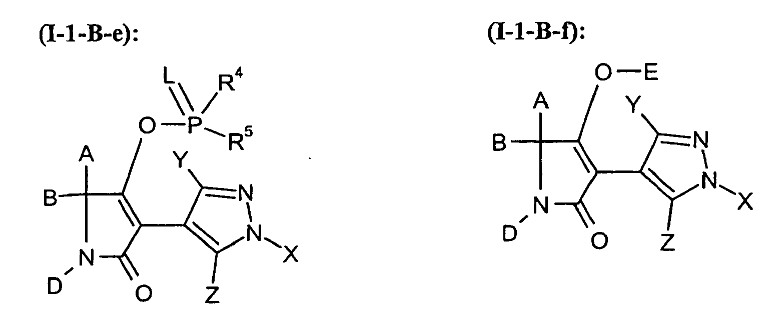 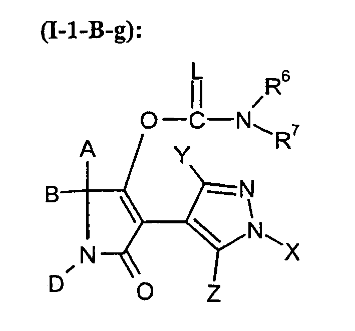 worin
A, B, D, E, L, M, X, Y, Z, R¹, R², R³, R⁴, R⁵, R⁶ und R⁷ die oben angegebene Bedeutung haben.

Unter Einbeziehung der verschiedenen Bedeutungen (a), (b), (c), (d), (e), (f) und (g) der Gruppe G ergeben sich folgende hauptsächliche Strukturen (I-2-A-a) bis (I-2-A-g), wenn Het für die Gruppe (2) steht, 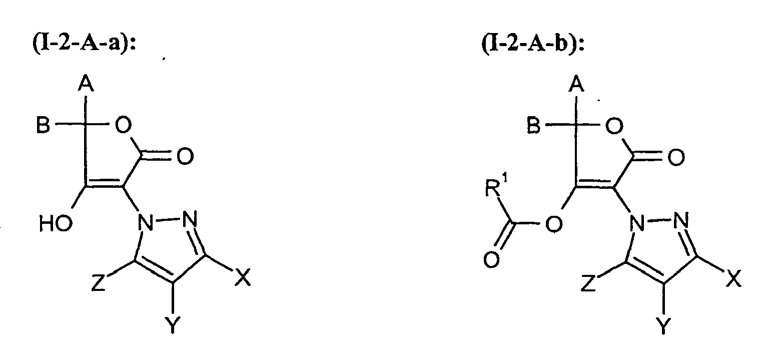 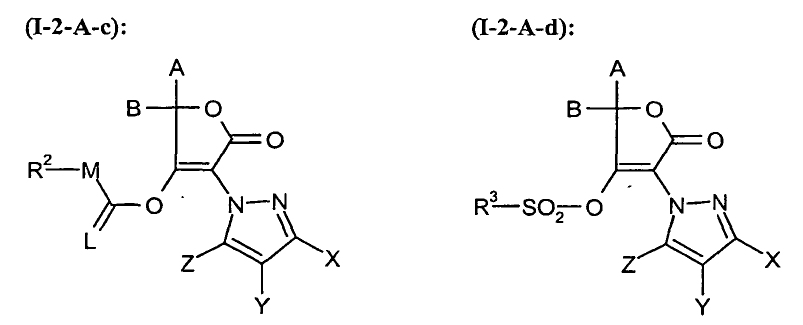 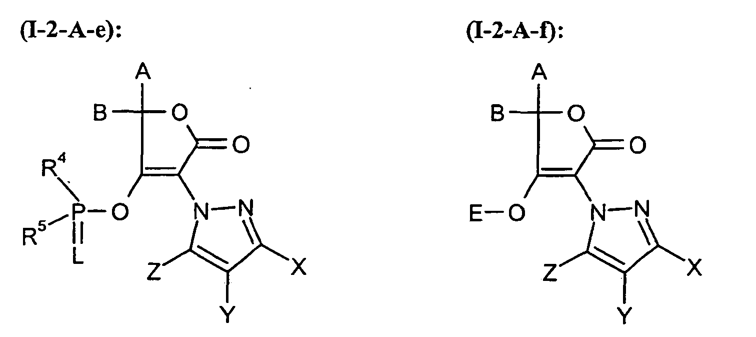 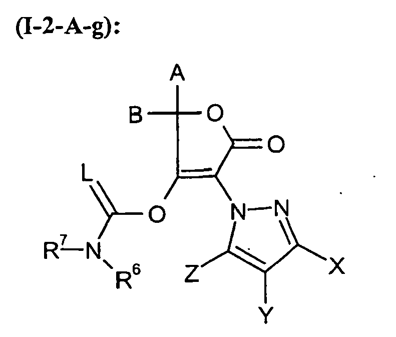 worin
A, B, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-2-B-a) bis (I-2-B-g), wenn Het für die Gruppe (2) steht, 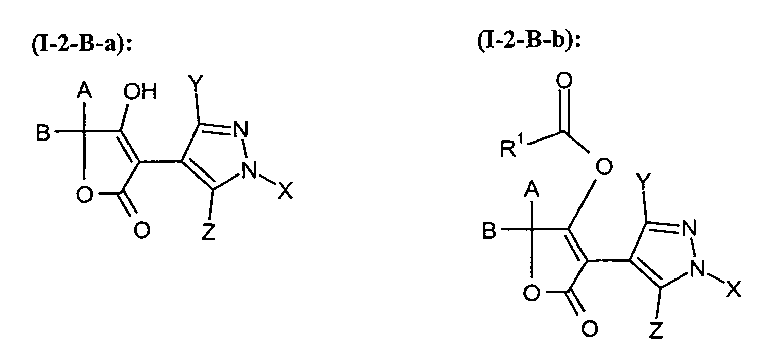 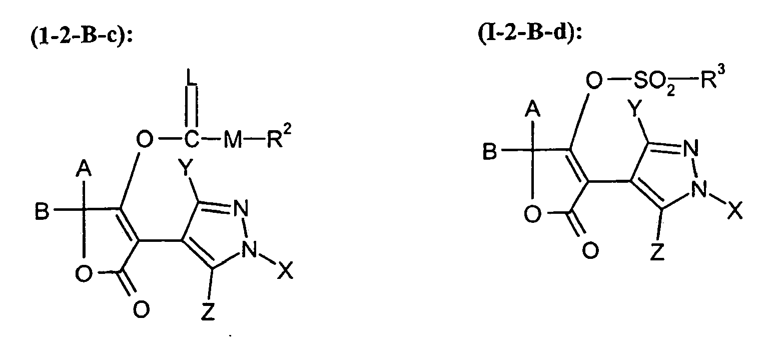 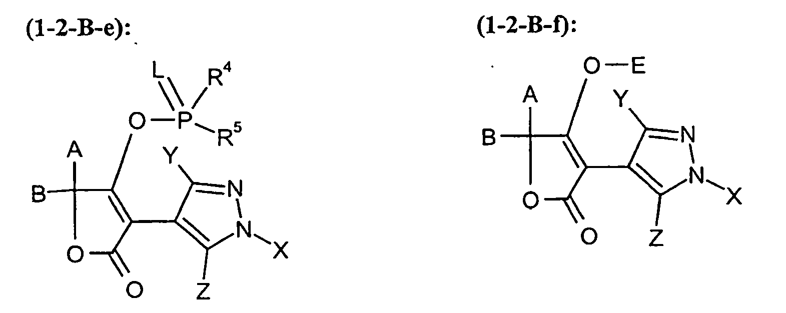 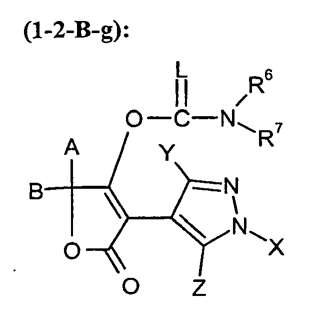 worin
A, B, E, L, M, X, Y, Z, R¹, R², R³, R⁴, R⁵, R⁶ und R⁷ die oben angegebene Bedeutung haben.

Unter Einbeziehung der verschiedenen. Bedeutungen (a), (b), (c), (d), (e), (f) und (g) der Gruppe G ergeben sich folgende hauptsächliche Strukturen (I-3-A-a) bis (I-3-A-g), wenn Het für die Gruppe (3) steht, 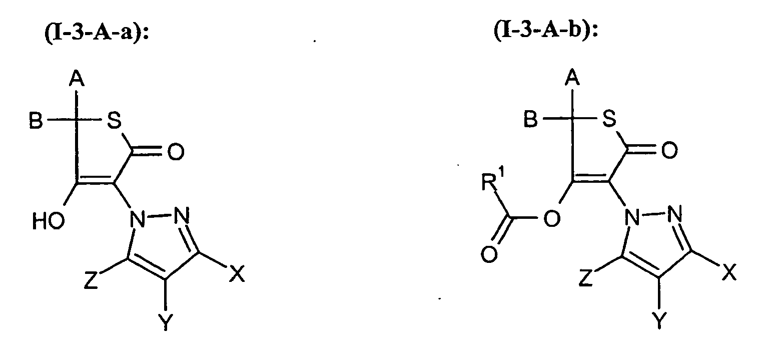 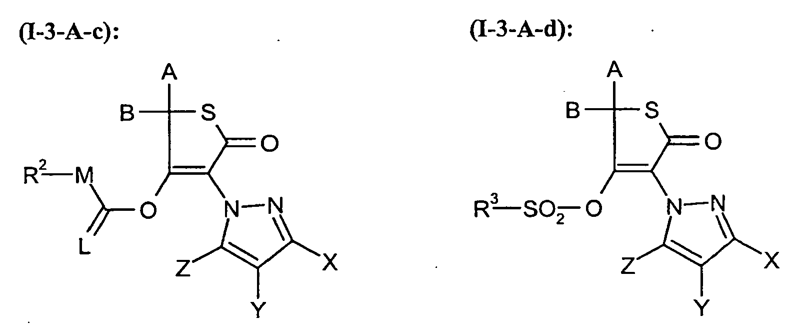 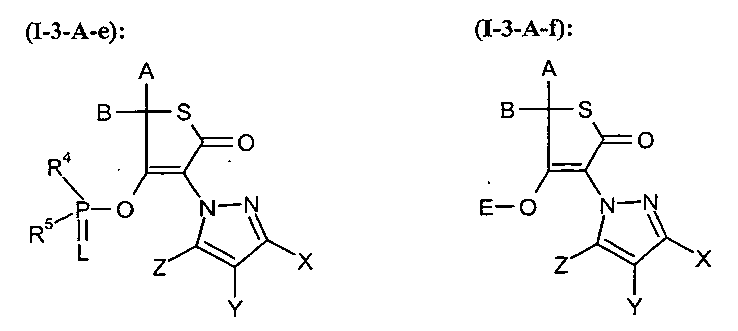 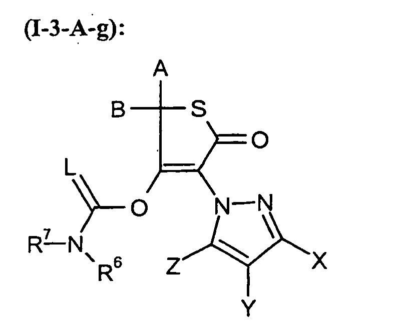 worin
A, B, E, L, M, X, Y, Z, R¹, R², R³, R⁴, R⁵, R⁶ und R⁷ die oben angegebenen Bedeutungen besitzen.

Unter Einbeziehung der verschiedenen Bedeutungen (a), (b), (c), (d), (e), (f) und (g) der Gruppe G ergeben sich folgende hauptsächliche Strukturen (I-3-B-a) bis (I-3-B-g), wenn Het für die Gruppe (3) steht, 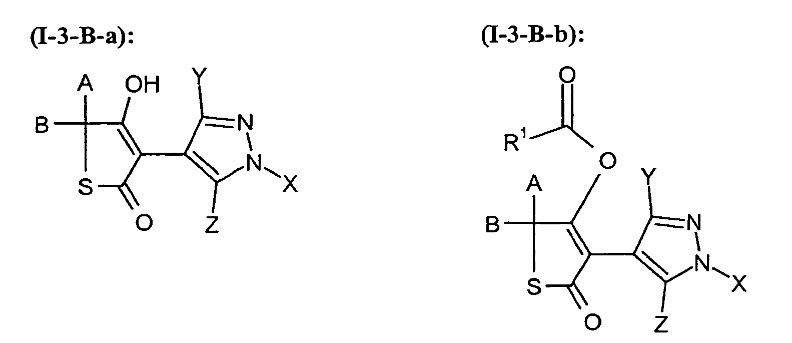 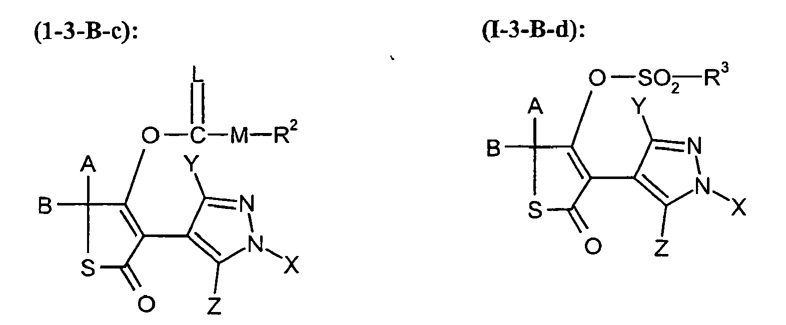 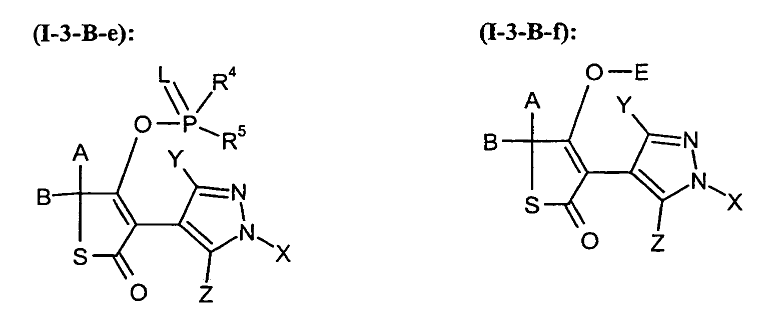 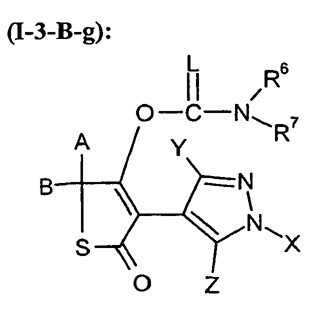 worin
A, B, E, L, M, X, Y, Z, R¹, R², R³, R⁴, R⁵, R⁶ und R⁷ die oben angegebene Bedeutung haben.

Weiterhin wurde gefunden, dass man die neuen Verbindungen der Formel (I) nach einem der im folgenden beschriebenen Verfahren erhält:
(A) Man erhält substituierte 3-Pyrazolyl-pyrrolidin-2,4-dione bzw. deren Enole der Formel (I-1-A-a) und (I-1-B-a) 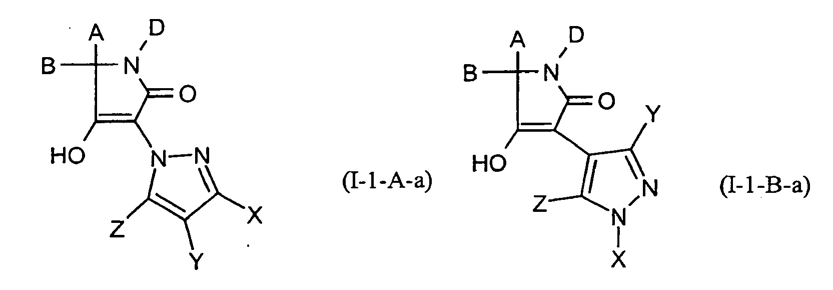 in welcher
   A, B, D, X, Y und Z die oben angegebenen Bedeutungen haben,
   wenn man
   N-Acylaminosäureester der Formel (II-A) und (II-B) 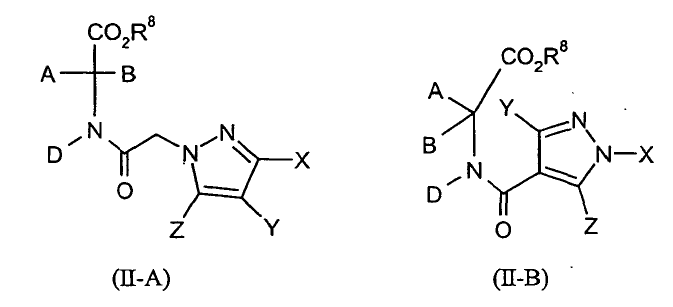 in welcher
   A, B, D, X, Y und Z die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man substituierte 3-Pyrazolyl-4-hydroxy-Δ³-dihydrofuranon-Derivate der Formel (I-2-A-a) und (I-2-B-a) 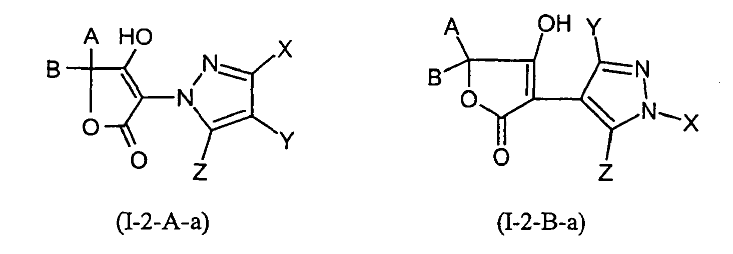 in welcher
   A, B, X, Y und Z die oben angegebenen Bedeutungen haben,
   erhält, wenn man
   Carbonsäureester der Formel (III-A) und (III-B) 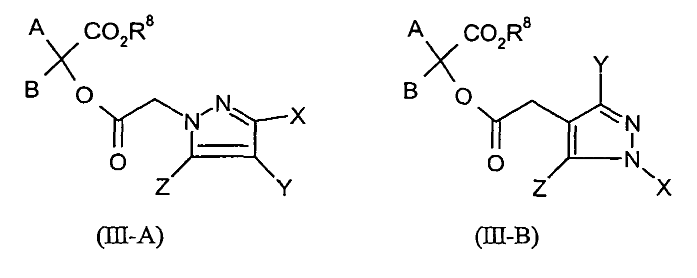 in welcher
   A, B, X, Y, Z und R⁸ die oben angegebenen Bedeutungen haben,
   in Gegenwart eines Verdünnungsmittels und in Gegenwart einer Base intramolekular kondensiert.
(C) Weiterhin wurde gefunden, dass man substituierte 3-Thiazolyl-4-hydroxy-Δ³-dihydrothiophen-2-on-Derivate der Formel (I-3-A-a) und (I-3-B-a) 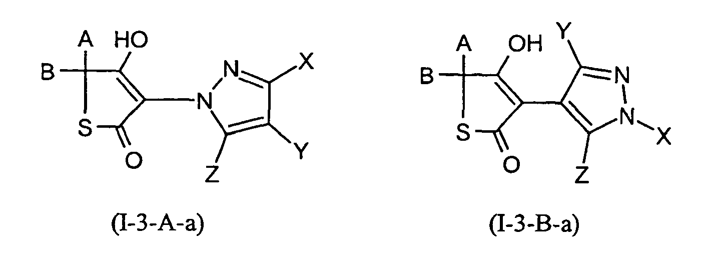 in welcher
   A, B, X, Y und Z die oben angegebenen Bedeutungen haben,
   erhält, wenn man
   β-Ketocarbonsäureester der Formel (IV-A) und (IV-B) 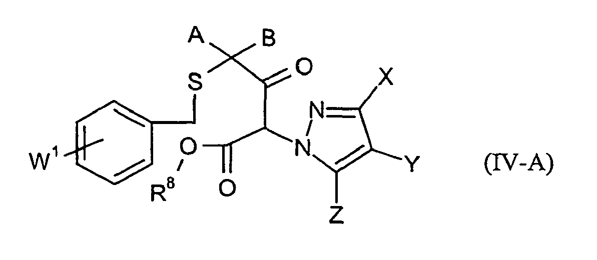 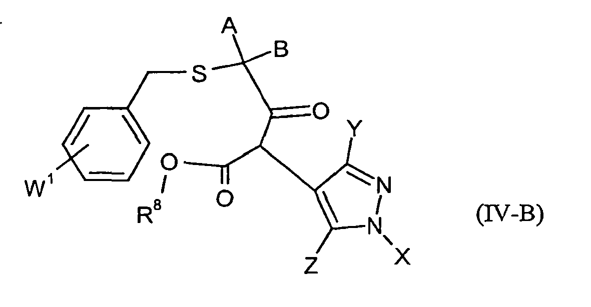 in welcher
   A, B, X, Y, Z und R⁸ die oben angegebenen Bedeutungen haben und
   - W¹: für Wasserstoff, Halogen, Alkyl (bevorzugt C₁-C₆-Alkyl) oder Alkoxy (bevorzugt C₁-C₈-Alkoxy) steht,
   gegebenenfalls in Gegenwart eines Verdünnungsmittels und in Gegenwart einer Säure intramolekular cyclisiert.
   Außerdem wurde gefunden
(D) dass man die Verbindungen der oben gezeigten Formeln (T-1-A-b) bis (I-3-B-b), in welchen A, B, D, R¹, X, Y und Z die oben angegebenen Bedeutungen haben, erhält, wenn man Verbindungen der oben gezeigten Formeln (I-1-A-a) bis (I-3-B-a), in welchen A, B, D, X, Y und Z die oben angegebenen Bedeutungen haben, jeweils
   (α) mit Säurehalogeniden der Formel (V) 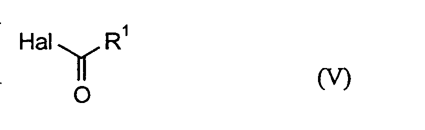 in welcher
      - R¹: die oben angegebene Bedeutung hat und
      - Hal: für Halogen (insbesondere Chlor oder Brom) steht
      oder
   (β) mit Carbonsäureanhydriden der Formel (VI)

      R¹-CO-O-CO-R¹ (VI)
   in welcher
   - R¹: die oben angegebene Bedeutung hat,
   gegebenenfalls in Gegenwart eines Verdünnungsmittels und gegebenenfalls in Gegenwart eines Säurebindemittels umsetzt;
(E) dass man die Verbindungen der oben gezeigten Formeln (I-1-A-c) bis (I-3-B-c), in welchen A, B, D, R², M, X, Y und Z die oben angegebenen Bedeutungen haben und L für Sauerstoff steht, erhält, wenn man Verbindungen der oben gezeigten Formeln (I-1-A-a) bis (I-3-B-a), in welchen A, B, D, X, Y und Z die oben angegebenen Bedeutungen haben, jeweils
   mit Chlorameisensäureestem oder Chlorameisensäurethioestern der Formel (VII)

   R²-M-CO-Cl (VII)

   in welcher
   - R² und M: die oben angegebenen Bedeutungen haben,
   gegebenenfalls in Gegenwart eines Verdünnungsmittels und gegebenenfalls in Gegenwart eines Säurebindemittels umsetzt;
(F) dass man Verbindungen der oben gezeigten Formeln (I-1-A-c) bis (I-3-B-c), in welchen A, B, D, R², M, X, Y und Z die oben angegebenen Bedeutungen haben und L für Schwefel steht, erhält, wenn man Verbindungen der oben gezeigten Formeln (I-1-A-a) bis (I-3-B-a), in welchen A, B, D, X, Y und Z die oben angegebenen Bedeutungen haben, jeweils
   mit Chlormonothioameisensäureestern oder Chlordithioameisensäureestern der Formel (VIII) 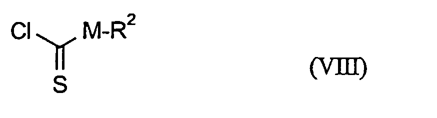 in welcher
   - M und R²: die oben angegebenen Bedeutungen haben,
   gegebenenfalls in Gegenwart eines Verdünnungsmittels und gegebenenfalls in Gegenwart eines Säurebindemittels umsetzt
   und
(G) dass man Verbindungen der oben gezeigten Formeln (I-1-A-d) bis (I-3-B-d), in welchen A, B, D, R³, X, Y und Z die oben angegebenen Bedeutungen haben, erhält, wenn man Verbindungen der oben gezeigten Formeln (I-1-A-a) bis (I-3-B-a), in welchen A, B, D, X, Y und Z die oben angegebenen Bedeutungen haben, jeweils
   mit Sulfonsäurechloriden der Formel (IX)

   R³-SO₂-Cl (IX)

   in welcher
   - R³: die oben angegebene Bedeutung hat,
   gegebenenfalls in Gegenwart eines Verdünnungsmittels und gegebenenfalls in Gegenwart eines Säurebindemittels umsetzt,
(H) dass man Verbindungen der oben gezeigten Formeln (I-1-A-e) bis (I-3-B-e), in welchen A, B, D, L, R⁴, R⁵, X, Y und Z die oben angegebenen Bedeutungen haben, erhält, wenn man Verbindungen der oben gezeigten Formeln (I-1-A-a) bis (I-3-B-a), in welchen A, B, D, X, Y und Z die oben angegebenen Bedeutungen haben, jeweils
   mit Phosphorverbindungen der Formel (X) 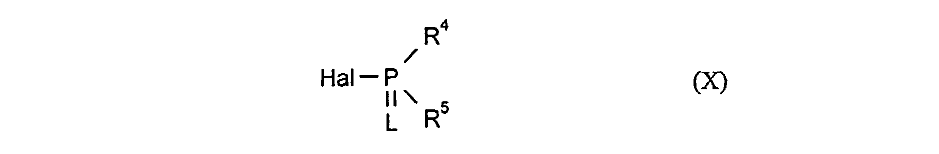 in welcher
   - L, R⁴ und R⁵: die oben angegebenen Bedeutungen haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt,
(I) dass man Verbindungen der oben gezeigten Formeln (I-1-A-f) bis (I-3-B-f), in welchen A, B, D, E, X, Y und Z die oben angegebenen Bedeutungen haben, erhält, wenn man Verbindungen der Formeln (I-1-A-a) bis (I-3-B-a), in welchen A, B, D, X, Y und Z die oben angegebenen Bedeutungen haben, jeweils
   mit Metallverbindungen oder Aminen der Formeln (XI) oder (XII)

   Me(OR¹⁰)ₜ (XI)

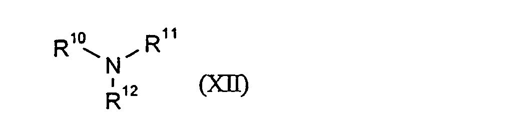

   in welchen
   - Me: für ein ein- oder zweiwertiges Metall (bevorzugt ein Alkali- oder Erd-alkalimetall wie Lithium, Natrium, Kalium, Magnesium oder Calcium),
   - t: für die Zahl 1 oder 2 und
   - R¹⁰, R¹¹, R¹²: unabhängig voneinander für Wasserstoff oder Alkyl (bevorzugt C₁-C₈-Alkyl) stehen,
   gegebenenfalls in Gegenwart eines Verdünnungsmittels umsetzt,
(J) dass man Verbindungen der oben gezeigten Formeln (I-1-A-g) bis (I-3-B-g), in welchen A, B, D, L, R⁶, R⁷, X, Y und Z die oben angegebenen Bedeutungen haben, erhält, wenn man Verbindungen der oben gezeigten Formeln (I-1-A-a) bis (I-3-B-a), in welchen A, B; D, X, Y und Z die oben angegebenen Bedeutungen haben, jeweils
   (α) mit Isocyanaten oder Isothiocyanaten der Formel (XIII)

      R⁶-N=C=L (XIII)

      in welcher
      - R⁶ und L: die oben angegebenen Bedeutungen haben,
      gegebenenfalls in Gegenwart eines Verdünnungsmittels und gegebenenfalls in Gegenwart eines Katalysators umsetzt oder
   (β) mit Carbamidsäurechloriden oder Thiocarbamidsäurechloriden der Formel (XIV) 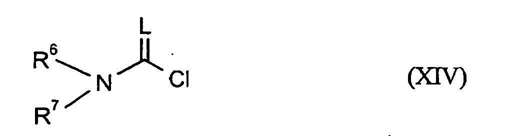 in welcher
      - L, R⁶ und R⁷: die oben angegebenen Bedeutungen haben,
   gegebenenfalls in Gegenwart eines Verdünnungsmittels und gegebenenfalls in Gegenwart eines Säurebindemittels, umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und Herbizide aufweisen.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im folgenden erläutert,
wobei die Verknüpfungspositionen am Pyrazolring entsprechend der Strukturen I-1-A bis I-3-B festgelegt sind:
- X: steht besonders bevorzugt für jeweils gegebenenfalls einfach bis dreifach durch Fluor, Chlor, Brom, C₁-C₄-Alkyl, C₁-C₄-Alkoxy, C₁-C₂-Halogenallcyl, C₁-C₂-Halogenalkoxy, Nitro oder Cyano substituiertes Phenyl,
- Y: steht besonders bevorzugt für Wasserstoff, Chlor, Brom oder C₁-C₄-Alkyl,
- Z: steht besonders bevorzugt für C₁-C₄-Alkyl, Chlor, Brom, C₁-C₄-Alkoxy, C₁-C₄-Halogenalkoxy oder jeweils gegebenenfalls einfach bis zweifach durch C₁-C₄-Alkyl, C₁-C₄-Alkoxy, Fluor, Chlor, Brom, C₁-C₂-Halogenalkyl, C₁-C₂-Halogenalkoxy, Cyano oder Nitro substituiertes Benzyloxy,
- Het: steht besonders bevorzugt für eine der Gruppen 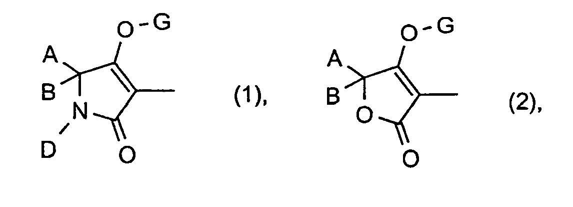 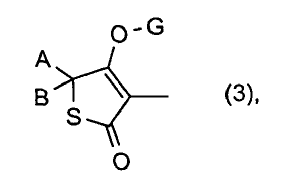
- A: steht besonders bevorzugt für Wasserstoff, jeweils gegebenenfalls durch Fluor substituiertes C₁-C₁₀-Alkyl, C₁-C₈-Alkoxy-C₁-C₆-alkyl, gegebenenfalls durch Fluor, Chlor, C₁-C₄-Alkyl oder C₁-C₄-Akoxy substituiertes C₃-C₇-Cycloalkyl, in welchem gegebenenfalls ein Ringglied durch Sauerstoff oder Schwefel ersetzt ist oder jeweils gegebenenfalls durch Fluor, Chlor, Brom, C₁-C₄-Alkyl, C₁-C₄-Halogenalkyl, C₁-C₄-Alkoxy, Cyano, Nitro oder C₁-C₄-Halogenalkoxy substituiertes Phenyl oder Phenyl-C₁-C₂-alkyl,
- B: steht besonders bevorzugt für Wasserstoff oder C₁-C₁₀-Alkyl oder
- A, B: und das Kohlenstoffatom an das sie gebunden sind, stehen besonders bevorzugt für gesättigtes C₃-C₇-Cycloalkyl oder ungesättigtes C₅-C₇-Cyaoakyl, worin gegebenenfalls ein Ringglied durch Sauerstoff oder Schwefel ersetzt ist und welches gegebenenfalls einfach oder zweifach durch C₁-C₆-Alkyl, C₅-C₈-Cycloalkyl, C₁-C₃-Halogenalkyl, C₁-C₆-Alkoxy, Fluor, Chlor oder Phenyl substituiert ist oder
- A, B: und das Kohlenstoffatom, an das sie gebunden sind, stehen besonders bevorzugt für C₅-C₆-Cycloalkyl, welches durch eine gegebenenfalls ein oder zwei nicht direkt benachbarte Sauerstoff- oder Schwefelatome enthaltende Alkylendiyl- oder durch eine Alkylendioxyl- oder durch eine Alkylendithiolyl-Gruppe substituiert ist, die mit dem Kohlenstoffatom, an das sie gebunden ist, einen weiteren fünf- oder sechsgliedrigen Ring bildet der gegebenenfalls durch C₁-C₃-Alkyl substituiert sein kann, oder
- A, B: und das Kohlenstoffatom, an das sie gebunden sind, stehen besonders bevorzugt für C₅-C₆-Cycloalkyl oder C₅-C₆-Cycloalkenyl, in welchen zwei Substituenten gemeinsam mit den Kohlenstoffatomen, an die sie gebunden sind, für jeweils gegebenenfalls durch C₁-C₅-Alkyl, C₁-C₅-Alkoxy, Fluor, Chlor oder Brom substituiertes C₂-C₄-Alkandlyl, C₂-C₄-Alkendiyl, worin gegebenenfalls eine Methylengruppe durch Sauerstoff oder Schwefel ersetzt ist oder Butadiendiyl stehen,
- D: steht besonders bevorzugt für Wasserstoff, für jeweils gegebenenfalls durch Fluor substituiertes C₁-C₁₀-Alkyl, C₃-C₆-Alkenyl, C₁-C₆-Alkoxy-C₂-C₄-alkyl oder C₁-C₆-Alkylthio-C₂-C₄-alkyl, für gegebenenfalls durch Fluor, C₁-C₄-Alkyl, C₁-C₄-Alkoxy oder C₁-C₂-Halogenakyl substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₄-Alkyl, C₁-C₄-Halogenakyl, C₁-C₄-Alkoxy oder C₁-C₄-Halogenalkoxy substituiertes Phenyl, oder Phenyl-C₁-C₄-alkyl oder
- A und D: stehen gemeinsam besonders bevorzugt für gegebenenfalls substituiertes C₃-C₅-Alkandiyl, in welchem gegebenenfalls eine Methylengruppe durch Sauerstoff oder Schwefel ersetzt sein kann, wobei als Substituenten C₁-C₆-Alkyl oder C₁-C₄-Alkoxy in Frage kommen oder
- A und D: stehen (im Fall der Verbindungen der Formel (I-1)) gemeinsam mit den Atomen, an die sie gebunden sind, für eine der Gruppen AD-1 bis AD-10: 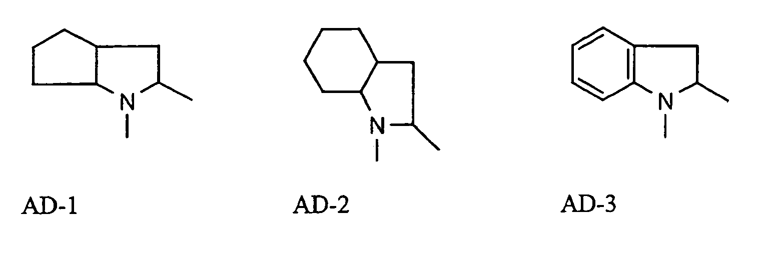 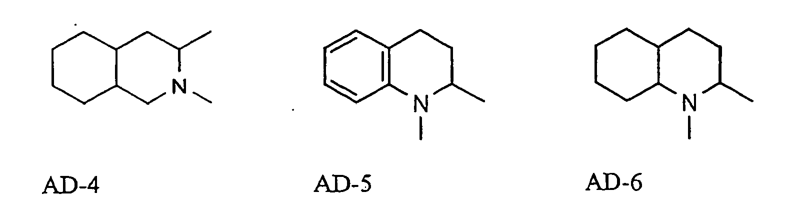 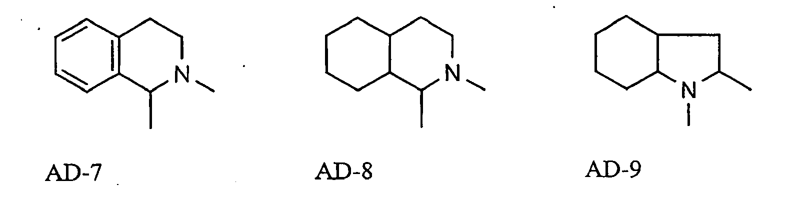 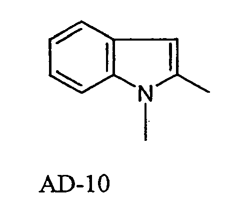
- G: steht besonders bevorzugt für Wasserstoff (a) oder für eine der Gruppen 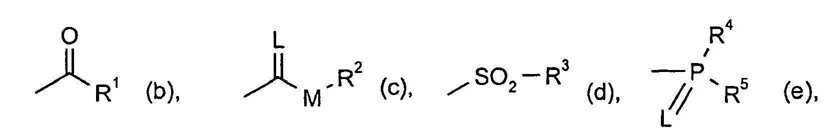 E (f) oder 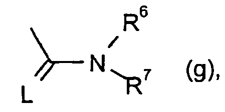 insbesondere für (a), (b), (c) oder (g),
in welchen
E für ein Metallionäquivalent oder ein Ammoniumion steht,
L für Sauerstoff oder Schwefel steht und
M für Sauerstoff oder Schwefel steht,
- R¹: steht besonders bevorzugt für jeweils gegebenenfalls durch Fluor oder Chlor substituiertes C₁-C₁₆-Alkyl, C₂-C₁₆-Alkenyl, C₁-C₆-Alkoxy-C₁-C₄-alkyl, C₁-C₆-Alkylthio-C₁-C₄-alkyl oder gegebenenfalls durch Fluor, Chlor, C₁-C₅-Alkyl oder C₁-C₅-Alkoxy substituiertes C₃-C₇-Cycloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenakyl oder C₁-C₃-Halogenalkoxy substituiertes Phenyl-C₁-C₄-alkyl,
für jeweils gegebenenfalls durch Fluor, Chlor, Brom, C₁-C₄-Alkyl, Trifluormethyl oder C₁-C₂-Alkoxy substituiertes Pyrazolyl, Thiazolyl, Pyridyl, Pyrimidyl, Furanyl oder Thienyl,
- R²: steht besonders bevorzugt für jeweils gegebenenfalls durch Fluor substituiertes C₁-C₁₆-Alkyl, C₂-C₁₆-Alkenyl oder C₁-C₆-Alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder
für jeweils gegebenenfalls durch Fluor, Chlor, Brom, Cyano, Nitro, C₁-C₄-Alkyl, C₁-C₃-Akoxy, C₁-C₂-Halogenalkyl oder C₁-C₂-Halogenalkoxy substituiertes Phenyl oder Benzyl,
- R³: steht besonders bevorzugt für gegebenenfalls durch Fluor substituiertes C₁-C₆-Alkyl oder für jeweils gegebenenfalls durch Fluor, Chlor, Brom, C₁-C₄-Alkyl, C₁-C₄-Alkoxy, C₁-C₃-Halogenalkyl, C₁-C₃-Halogenalkoxy, Cyano oder Nitro substituiertes Phenyl,
- R⁴: steht besonders bevorzugt für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio,
- R⁵: steht besonders bevorzugt für C₁-C₆-Alkoxy oder C₁-C₆-Alkylthio,
- R⁶: steht besonders bevorzugt für C₁-C₆-Alkyl, C₃-C₆-Cycloalkyl, C₁-C₆-Alkoxy, C₃-C₆-Alkenyl, C₁-C₆-Alkoxy-C₁-C₆-alkyl, für gegebenenfalls durch Fluor, Chlor, Brom, C₁-C₃-Halogenalkyl, C₁-C₄-Alkyl oder C₁-C₄-Alkoxy substituiertes Phenyl, für gegebenenfalls durch Fluor, Chlor, Brom, C₁-C₄-Alkyl, C₁-C₃-Halogenalkyl oder C₁-C₄-Alkoxy substituiertes Benzyl,
- R⁷: steht besonders bevorzugt für Wasserstoff, C₁-C₆-Alkyl, C₃-C₆-Alkenyl,
- R⁶ und R⁷: stehen besonders bevorzugt zusammen für einen gegebenenfalls durch Methyl oder Ethyl substituierten C₄-C₅-Alkylenrest, in welchem gegebenenfalls eine Methylengruppe durch Sauerstoff oder Schwefel ersetzt ist,
wobei die Verknüpfungsposition am Pyrazolylrest festgelegt wird auf folgende Strukturen:

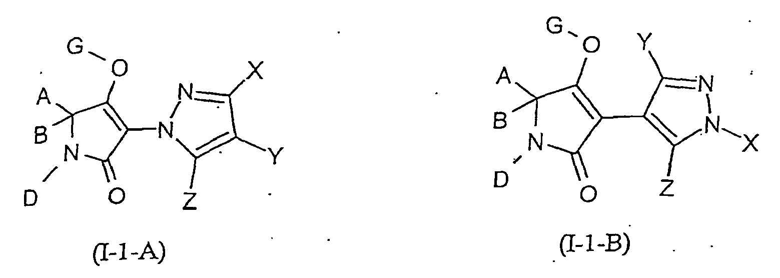

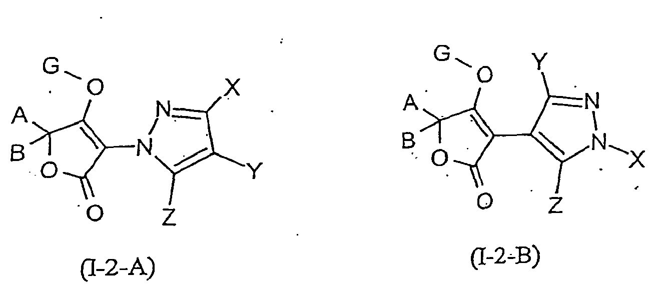

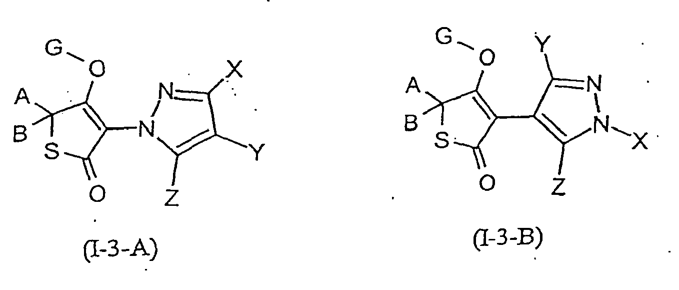

In den als besonders bevorzugt genannten Restedefinitionen steht Halogen für Fluor, Chlor, Brom und Iod, insbesondere für Fluor, Chlor und Brom.
- X: steht ganz besonders bevorzugt für gegebenenfalls einfach bis zweifach durch Fluor, Chlor, Brom, Methyl, Ethyl, Trifluormethyl, Isopropyl, tert.-Butyl, Trifluormethoxy, Methoxy, Ethoxy, Isopropoxy, tert.-Butoxy, Cyano oder Nitro substituiertes Phenyl,
- Y: steht ganz besonders bevorzugt für Wasserstoff, Methyl oder Ethyl,
- Z: steht ganz besonders bevorzugt für Methyl, Ethyl, Propyl, Isopropyl, Chlor, Methoxy, Ethoxy, Propoxy, Isopropoxy, Difluormethoxy oder Trifluorethoxy,
- Het: steht ganz besonders bevorzugt für eine der Gruppen 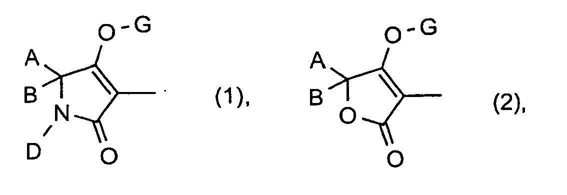 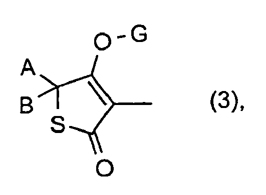
- A: steht ganz besonders bevorzugt für Wasserstoff, für jeweils gegebenenfalls durch Fluor substituiertes C₁-C₈-Alkyl oder C₁-C₆-Alkoxy-C₁-C₄-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder für jeweils gegebenenfalls einfach bis zweifach durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl,
- B: steht ganz besonders bevorzugt für Wasserstoff oder C₁-C₆-Alkyl oder
- A, B: und das Kohlenstoffatom an das sie gebunden sind, stehen ganz besonders bevorzugt für gesättigtes C₃-C₆-Cycloalkyl, in welchem gegebenenfalls ein Ringglied durch Sauerstoff oder Schwefel ersetzt ist und welches gegebenenfalls einfach durch Methyl, Ethyl, Propyl, Isopropyl, Butyl, iso-Butyl, sec.-Butyl, tert.-Butyl, Trifluormethyl, Methoxy, Ethoxy, Propoxy, iso-Propoxy, Butoxy, iso-Butoxy, sek.-Butoxy, tert.-Butoxy, Fluor oder Chlor substituiert ist oder
- A, B: und das Kohlenstoffatom, an das sie gebunden sind, stehen ganz besonders bevorzugt für C₆-Cycloalkyl, welches durch eine gegebenenfalls einfach bis zweifach durch Methyl oder Ethyl substituierte Alkylendioxyl-Gruppe substituiert ist, die mit dem Kohlenstoffatom, an das sie gebunden ist, einen weiteren fünf- oder sechsgliedrigen Ring bildet,
- A, B: und das Kohlenstoffatom, an das sie gebunden sind, stehen ganz besonders bevorzugt für C₅-C₆-Cycloalkyl oder C₅-C₆-Cycloalkenyl, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen,
- D: steht ganz besonders bevorzugt für Wasserstoff, für jeweils gegebenenfalls durch Fluor substituiertes C₁-C₈-Alkyl, C₃-C₄-Alkenyl, C₁-C₆-Alkoxy-C₂-C₄-alkyl, C₁-C₄-Alkylthio-C₂-C₄-alkyl oder für durch Trifluormethyl oder Fluor substituiertes C₃-C₆-Cycloalkyl, in welchem gegebenenfalls eine Methylengruppe durch Sauerstoff oder Schwefel ersetzt ist oder für jeweils gegebenenfalls einfach bis zweifach durch Fluor, Chlor, Methyl, Ethyl, n-Propyl, iso-Propyl, Methoxy, Ethoxy, Trifluormethyl oder Trifluormethoxy substituiertes Phenyl oder Benzyl,
oder
- A und D: stehen gemeinsam ganz besonders bevorzugt für C₃-C₄-Alkandiyl, worin gegebenenfalls ein Kohlenstoffatom durch Schwefel ersetzt ist und welches gegebenenfalls einfach bis zweifach durch Methyl, Ethyl, Methoxy oder Ethoxy substituiert ist oder
- A und D: stehen (im Fall der Verbindungen der Formel (I-1)) gemeinsam mit den Atomen, an die sie gebunden sind, für eine der folgenden Gruppen AD: 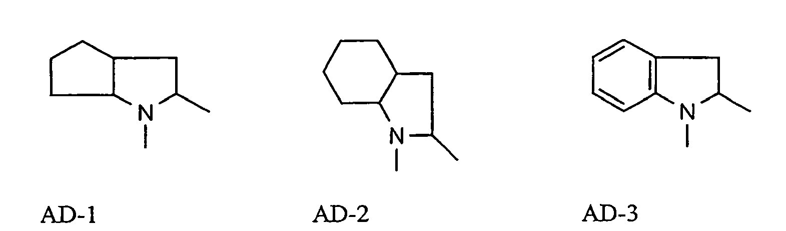 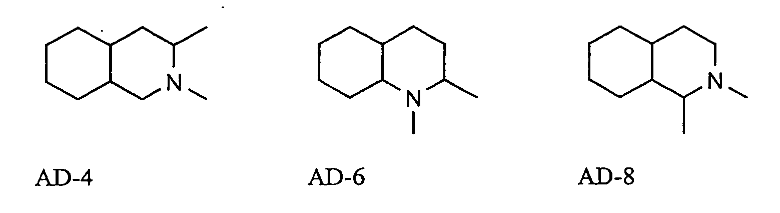 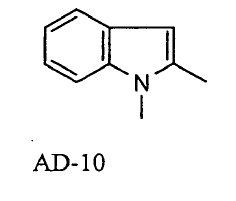
- G: steht ganz besonders bevorzugt für Wasserstoff (a) oder für eine der Gruppen 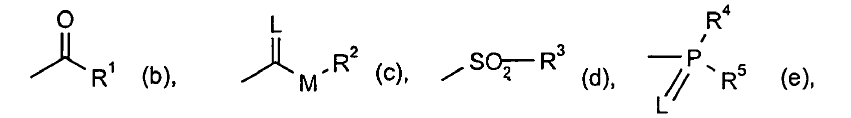 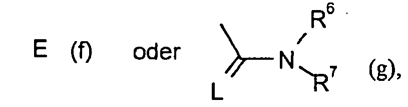 insbesondere für (a), (b) oder (c), in welchen
E für ein Metallionäquivalent oder ein Ammoniumion steht,
L für Sauerstoff oder Schwefel steht und
M für Sauerstoff oder Schwefel steht,
- R¹: steht ganz besonders bevorzugt für jeweils gegebenenfalls durch Fluor oder Chlor substituiertes C₁-C₁₄-Alkyl, C₂-C₁₄-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, Methyl, Ethyl, Propyl, i-Propyl, Butyl, i-Butyl, tert.-Butyl, Methoxy oder Ethoxy, substituiertes C₃-C₆-Cycloalkyl, in welchem gegebenenfalls ein oder zwei nicht direkt benachbarte Ringglieder durch Sauerstoff und/oder Schwefel ersetzt sind,
für gegebenenfalls einfach bis zweifach durch Fluor, Chlor, Brom, Cyano, Nitro, Methyl, Ethyl, n-Propyl, i-Propyl, tert.-Butyl, Methoxy, Ethoxy, i-Propoxy, tert.-Butoxy, Trifluormethyl oder Trifluormethoxy substituiertes Phenyl,
für jeweils gegebenenfalls einfach bis zweifach durch Fluor, Chlor, Brom, Methyl oder Ethyl substituiertes Furanyl, Thienyl oder Pyridyl,
- R²: steht ganz besonders bevorzugt für jeweils gegebenenfalls einfach bis dreifach durch Fluor substituiertes C₁-C₁₄-Alkyl, C₂-C₁₄-Alkenyl oder C₁-C₄-Alkoxy-C₂-C₆-alkyl,
für gegebenenfalls einfach bis zweifach durch Fluor, Chlor, Methyl, Ethyl, n-Propyl, iso-Propyl oder Methoxy substituiertes C₃-C₆-Cycloalkyl,
oder für jeweils gegebenenfalls einfach bis zweifach durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl,
- R³: steht ganz besonders bevorzugt für jeweils gegebenenfalls einfach bis dreifach durch Fluor substituiertes Methyl, Ethyl, n-Propyl oder gegebenenfalls einfach bis zweifach durch Fluor, Chlor, Brom, Methyl, tert.-Butyl, Methoxy, Trifluormethyl, Trifluormethoxy, Cyano oder Nitro substituiertes Phenyl,
- R⁴: steht ganz besonders bevorzugt für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls einfach bis zweifach durch Fluor, Chlor, Brom, Nitro, Cyano, C₁-C₂-Alkoxy, C₁-C₂-Fluoralkoxy, C₁-C₂-Alkylthio, C₁-C₂-Fluorakylthio oder C₁-C₃-Alkyl substituiertes Phenyl, Phenoxy oder Phenylthio,
- R⁵: steht ganz besonders bevorzugt für Methoxy, Ethoxy, Methylthio oder Ethylthio,
- R⁶: steht ganz besonders bevorzugt für C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls einfach bis zweifach durch Fluor, Chlor, Brom, Trifluormethyl, Methyl oder Methoxy substituiertes Phenyl, für gegebenenfalls einfach bis zweifach durch Fluor, Chlor, Brom, Methyl, Trifluormethyl oder Methoxy substituiertes Benzyl,
- R⁷: steht ganz besonders bevorzugt für Wasserstoff, Methyl, Ethyl, Propyl oder Allyl,
- R⁶ und R⁷: stehen ganz besonders bevorzugt zusammen für einen C₅-C₆-Alkylenrest, in welchem gegebenenfalls eine Methylengruppe durch Sauerstoff oder Schwefel ersetzt ist,
wobei die Verknüpfungsposition am Pyrazolylrest festgelegt wird auf folgende Strukturen: 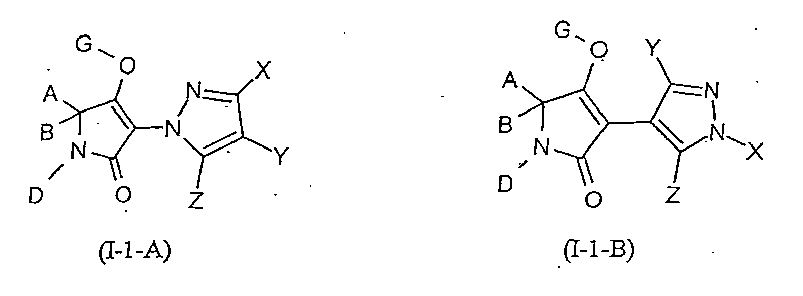 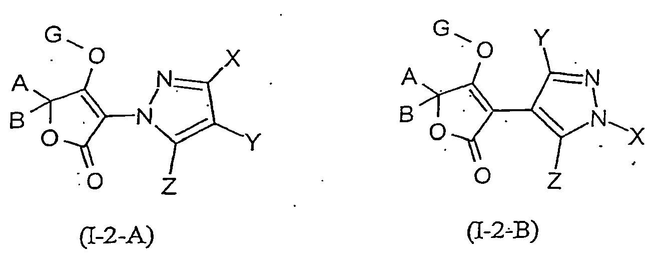 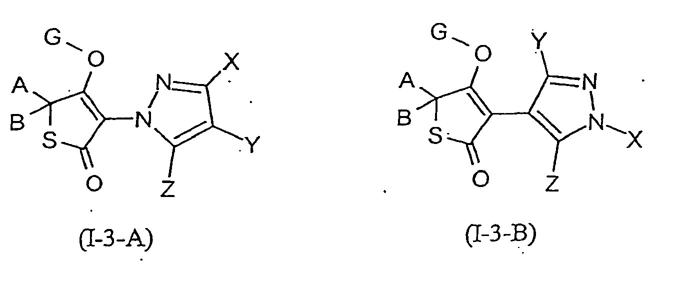
- X: steht hervorgehoben für gegebenenfalls einfach bis zweifach durch Fluor, Chlor, Brom, Methyl, Trifluormethyl, Methoxy oder Trifluormethoxy substituiertes Phenyl,
- Y: steht hervorgehoben für Wasserstoff oder Methyl,
- Z: steht hervorgehoben für Methyl, Ethyl oder Propyl,
- Het: steht hervorgehoben für eine der Gruppen 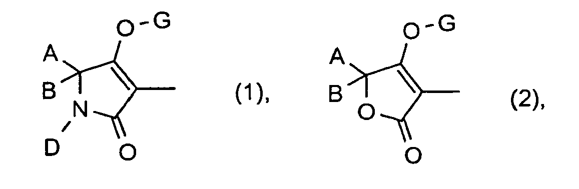
- A: steht hervorgehoben für Wasserstoff oder C₁-C₆-Alkyl,
- B: steht hervorgehoben für Wasserstoff oder C₁-C₆-Alkyl oder
- A, B: und das Kohlenstoffatom an das sie gebunden sind, stehen hervorgehoben für gesättigtes C₅-C₆-Cycloalkyl, in welchem gegebenenfalls ein Ringglied durch Sauerstoff oder Schwefel ersetzt ist und welches gegebenenfalls einfach durch Methyl, Ethyl, Propyl, Isopropyl, Trifluormethyl, Methoxy, Ethoxy, Propoxy, iso-Propoxy, Butoxy oder iso-Butoxy substituiert ist oder
- A, B: und das Kohlenstoffatom an das sie gebunden sind stehen hervorgehoben für C₅-C₆-Cycloalkyl oder C₅-C₆-Cycloalkenyl, worin zwei Substituenten gemeinsam mit den Kohlenstoffatomen, an die sie gebunden sind, für Butadienyl stehen,
- D: steht hervorgehoben für Wasserstoff, Methyl, Ethyl oder Isopropyl oder für durch Trifluormethyl substituiertes Cyclohexyl,
- A und D: stehen gemeinsam hervorgehoben für C₃-C₄-Alkandiyl,
- G: steht hervorgehoben für Wasserstoff (a) oder für eine der Gruppen 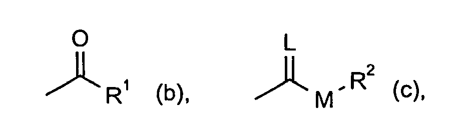 in welchen
L für Sauerstoff steht und
M für Sauerstoff oder Schwefel steht,
- R¹: steht hervorgehoben für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder gegebenenfalls einfach durch Fluor, Chlor, Methyl, Ethyl oder Methoxy substituiertes C₃-C₆-Cycloalkyl, in welchem gegebenenfalls ein Ringglied durch Sauerstoff oder Schwefel ersetzt ist,
für gegebenenfalls einfach durch Fluor, Chlor, Brom, Cyano, Nitro, Methyl, Ethyl, n-Propyl, i-Propyl, tert.-Butyl, Methoxy, Ethoxy, i-Propoxy, tert.-Butoxy, Trifluormethyl oder Trifluormethoxy substituiertes Phenyl,
für jeweils gegebenenfalls einfach durch Fluor, Chlor, Brom, Methyl oder Ethyl substituiertes Thienyl oder Pyridyl,
- R²: steht hervorgehoben für jeweils gegebenenfalls einfach bis dreifach durch Fluor substituiertes C₁-C₆-Alkyl, C₂-C₆-Akenyl oder C₁-C₄-Alkoxy-C₂-C₃-alkyl, für Cyclopentyl oder Cyclohexyl,
oder für jeweils gegebenenfalls einfach durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl,
wobei die Verknüpfungsposition am Pyrazolylrest festgelegt wird auf folgende Strukturen:

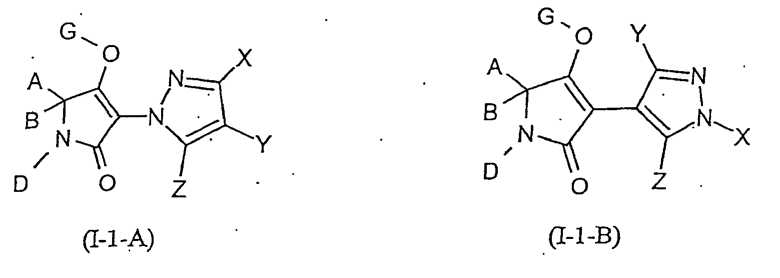

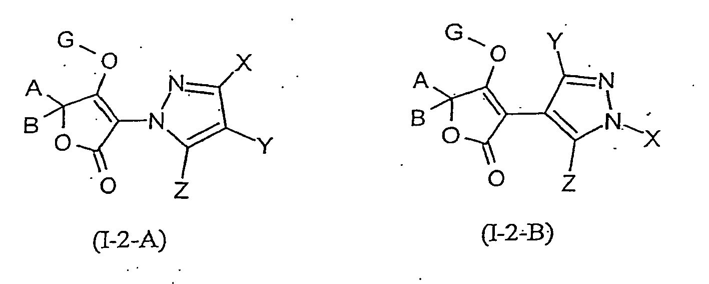

Wenn der Pyrazolrest folgende Verknüpfungsposition hat: 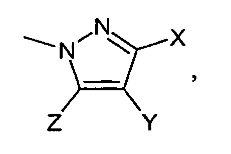 dann steht
- X: insbesondere für gegebenenfalls einfach bis zweifach durch Chlor, Brom oder Trifluormethyl substituiertes Phenyl,
- Y: insbesondere für Wasserstoff,
- Z: insbesondere für Methyl, Ethyl oder Propyl,
- Het: insbesondere für eine der Gruppen 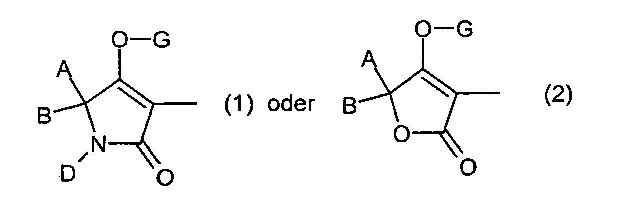
- A: insbesondere für Wasserstoff oder C₁-C₆-Alkyl,
- B: insbesondere für Wasserstoff oder C₁-C₆-Alkyl,
- A, B: und das Kohlenstoffatom, an das sie gebunden sind, insbesondere für gesättigtes C₆-Cycloalkyl, in welchem gegebenenfalls ein Ringglied durch Sauerstoff ersetzt ist oder insbesondere für gesättigtes C₆-Cycloalkyl, welches gegebenenfalls einfach durch Methyl, Trifluormethyl, Methoxy oder Ethoxy substituiert ist,
- A, B: und das Kohlenstoffatom an das sie gebunden sind insbesondere für C₅-C₆-Cycloalkyl worin zwei Substituenten gemeinsam mit den Kohlenstoffatomen an die sie gebunden sind für Butadienyl stehen,
- D: insbesondere für Wasserstoff oder für einfach durch Trifluormethyl substituiertes Cyclohexyl,
- A und D: insbesondere für C₃-C₄-Alkandiyl,
- G: insbesondere für Wasserstoff (a) oder für eine der Gruppen 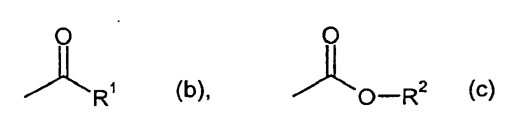 worin
R¹ insbesondere für C₁-C₆-Alkyl, C₃-Cycloalkyl, für jeweils durch Chlor substituiertes Phenyl oder Pyridyl steht,
R² insbesondere für C₁-C₆-Alkyl oder Benzyl steht.

Wenn der Pyrazolrest folgende Verknüpfungsposition hat: 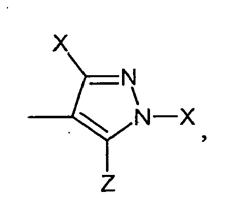 dann steht
- X: insbesondere für einfach durch Chlor oder Trifluormethyl substituiertes Phenyl,
- Y: insbesondere für Methyl,
- Z: insbesondere für Methyl,
- Het: insbesondere für die Gruppe 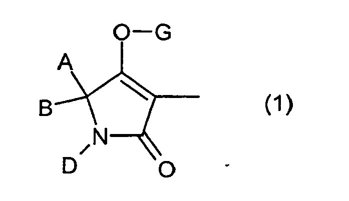
- A: insbesondere für Methyl,
- B: insbesondere für Methyl,
- A, B: und das Kohlenstoffatom, an das sie gebunden sind, insbesondere für C₆-Cycloalkyl, welches einfach durch Methoxy substituiert ist,
- D: insbesondere für Wasserstoff,
- G: insbesondere für Wasserstoff oder für die Gruppe 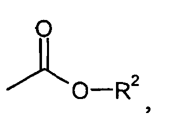
- R²: insbesondere für C₁-C₆-Alkyl.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-A-a) genannt.

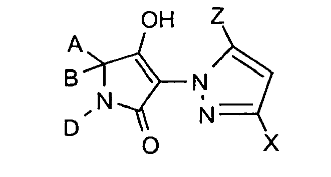

X = 4-Cl-C₆H₄, Z = CH₃.

**Tabelle 1:**

| A | B | D |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 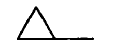 | CH₃ | H |
| 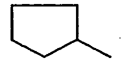 | CH₃ | H |
| 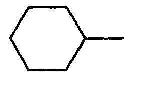 | CH₃ | H |
| -(CH₂)₂- | | H |
| -(CH₂)₄- | | H |
| -(CH₂)₃ | | H |
| -(CH₂)₅- | | H |
| -(CH₂)₆- | | H |
| -(CH₂)₇- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CH-i-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H₇-(CH₂)₂- | | H |
| -(CH₂)₂-CHi-OC₃H₇-(CH₂)₂- | | H |
| -(CH₂)₂-C(CH₃)₂-(CH₂)₂- | | H |
| -CH₂-(CHCH₃)₂-(CH₂)₂- | | H |
| 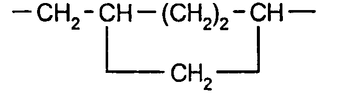 | | H |
| 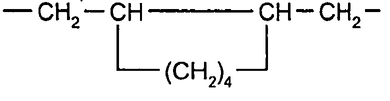 | | H |
| 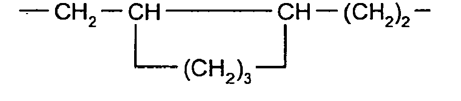 | | H |
| 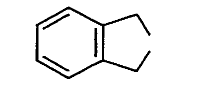 | | H |
| 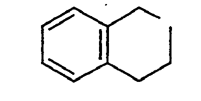 | | H |
| | | |
| A | D | B |
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-S-CH₂- | | H |
| -CH₂-S-(CH₂)₂- | | H |
| -(CH₂)₂-S-CH₂- | | H |
| 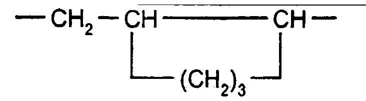 | | H |
| H | CH₃ | H |
| H | C₂H₅ | H |
| H | C₃H₇ | H |
| H | i-C₃H₇ | H |
| H | 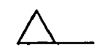 | H |
| H | 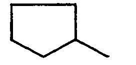 | H |
| H | 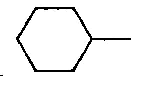 | H |
| CH₃ | CH₃ | H |
| CH₃ | C₂H₅ | H |
| A | D | B |
| CH₃ | C₃H₇ | H |
| CH₃ | i-C₃H₇ | H |
| CH₃ | 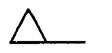 | H |
| CH₃ | 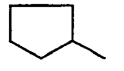 | H |
| CH₃ | 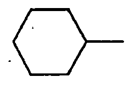 | H |
| C₂H₅ | CH₃ | H |
| C₂H₅ | C₂H₅ | H |

**Tabelle 2:** A, B und D wie in Tabelle 1 angegeben

   X = 4-Cl-C₆H₄, Z = C₂H₅.
**Tabelle 3:** A, B und D wie in Tabelle 1 angegeben

   X = 4-Cl-C₆H₄, Z = C₃H₇.
**Tabelle 4:** A, B und D wie in Tabelle 1 angegeben

   X = 3,4-Cl₂-C₆H₃, Z = CH₃.
**Tabelle 5:** A, B und D wie in Tabelle 1 angegeben

   X = 3,4-Cl₂-C₆H₃, Z = C₂H₅.
**Tabelle 6:** A, B und D wie in Tabelle 1 angegeben

   X = 3,4-Cl₂-C₆H₃, Z = C₃H₇.
**Tabelle 7:** A, B und D wie in Tabelle 1 angegeben

   X = 4-CF₃-C₆H₄, Z = CH₃.
**Tabelle 8:** A, B und D wie in Tabelle 1 angegeben

   X = 4-CF₃-C₆H₄, Z = C₂H₅.
**Tabelle 9:** A, B und D wie in Tabelle 1 angegeben

   X = 4-CF₃-C₆H₄, Z = C₃H₇.
**Tabelle 10:** A, B und D wie in Tabelle 1 angegeben

   X = 4-Br-C₆H₄, Z = CH₃.
**Tabelle 11:** A, B und D wie in Tabelle 1 angegeben

   X = 4-Br-C₆H₄, Z = C₂H₅.
**Tabelle 12:** A, B und D wie in Tabelle 1 angegeben

   X = 4-Br-C₆H₄, Z = C₃H₇.

Im einzelnen seien außer den bei den Herstellungsbeispielen genannten Verbindungen die folgenden Verbindungen der Formel (I-2-A-a) genannt:

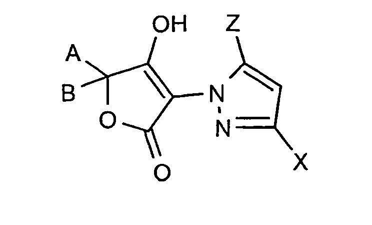

**Tabelle 13: X = 4-Cl-C₆H₄, Z = CH₃.**

| A | B |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 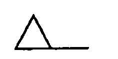 | CH₃ |
| 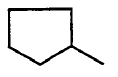 | CH₃ |
| 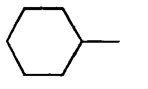 | CH₃ |
| -(CH₂)₂- | |
| -(CH₂)₄- | |
| -(CH₂)₅- | |
| -(CH₂)₆- | |
| -(CH₂)₇- | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH₂)₃- | |
| -(CH₂)₂-CHCH₃-(CH₂)₂- | |
| (CH₂)₂-CHC₂H₅-(CH₂)₂- | |
| -(CH₂)₂-CHC₃H₇-(CH₂)₂- | |
| -(CH₂)₂-CH-i-C₃H₇-(CH₂)₂- | |
| -(CH₂)₂-CHOCH₃-(CH₂)₂- | |
| -(CH₂)₂-CHOC₂H₅-(CH₂)₂- | |
| -(CH₂)₂-CHOC₃H₇-(CH₂)₂- | |
| -(CH₂)₂-CH-i-C₃H₇-(CH₂)₂- | |
| -(CH₂)₂-C(CH₃)₂-(CH₂)2- | |
| -CH₂-(CHCH₃)₂-(CH₂)₂- | |
| 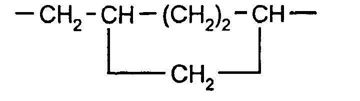 | |
| 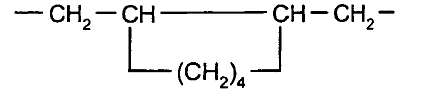 | |
| 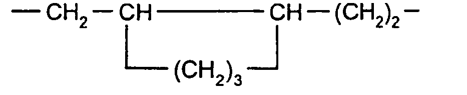 | |
| 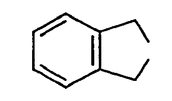 | |
| 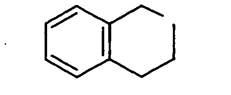 | |

**Tabelle 14:** A und B wie in Tabelle 13 angegeben

   X = 4-Cl-C₆H₄, Z = C₂H₅.
**Tabelle 15:** A und B wie in Tabelle 13 angegeben

   X = 4-Cl-C₆H₄, Z = C₃H₇.
**Tabelle 16:** A und B wie in Tabelle 13 angegeben

   X = 3,4-Cl₂-C₆H₃, Z = CH₃.
**Tabelle 17:** A und B wie in Tabelle 13 angegeben

   X = 3,4-Cl₂-C₆H₃, Z = C₂H₅.
**Tabelle 18:** A und B wie in Tabelle 13 angegeben

   X = 3,4-Cl₂-C₆H₃, Z = C₃H₇.
**Tabelle 19:** A und B wie in Tabelle 13 angegeben

   X = 4-CF₃-C₆H₄, Z = CH₃.
**Tabelle 20:** A und B wie in Tabelle 13 angegeben

   X = 4-CF₃-C₆H₄, Z = C₂H₅.
**Tabelle 21:** A und B wie in Tabelle 13 angegeben

   X = 4-CF₃-C₆H₄, Z = C₃H₇.
**Tabelle 22:** A und B wie in Tabelle 13 angegeben

   X = 4-Br-C₆H₄, Z = CH₃.
**Tabelle 23:** A und B wie in Tabelle 13 angegeben

   X = 4-Br-C₆H₄, Z = C₂H₅.
**Tabelle 24:** A und B wie in Tabelle 13 angegeben

   X = 4-Br-C₆H₄, Z = C₃H₇.

Verwendet man beispielsweise gemäß Verfahren (A) N-[1-(5-Methyl-3-phenyl)-pyrazolylacetyl]-1-amino-cyclohexan-carbonsäureethylester als Ausgangsstoff, so kann der Verlauf des erfindungsgemäßen Verfahrens durch folgendes Reaktionsschema wiedergegeben werden:

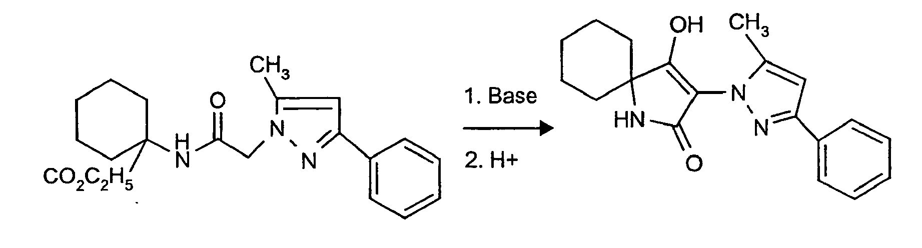

Verwendet man beispielsweise gemäß Verfahren (B) O-[1-(5-Methyl-3-(4-chlor)-phenyl)-pyrazolylacetyl]-2-hydroxyisobuttersäureethylester, so kann der Verlauf des erfindungsgemäßen Verfahrens durch folgendes Reaktionsschema wiedergegeben werden:

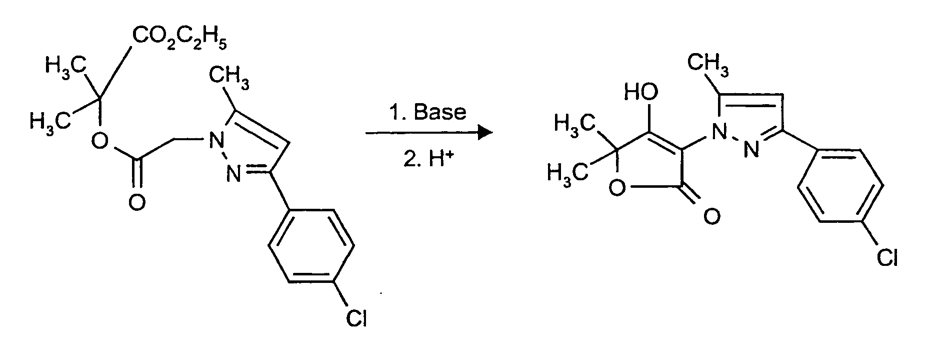

Verwendet man beispielsweise gemäß Verfahren (C) 2-[1-(5-Methyl-3-phenyl)-pyrazolyl]-4-(4-methoxy)-benzylmercapto-4-methyl-3-oxo-valeriansäure-ethylester, so kann der Verlauf des erfmdungsgemäßen Verfahrens durch folgendes Reaktionsschema wiedergegeben werden:

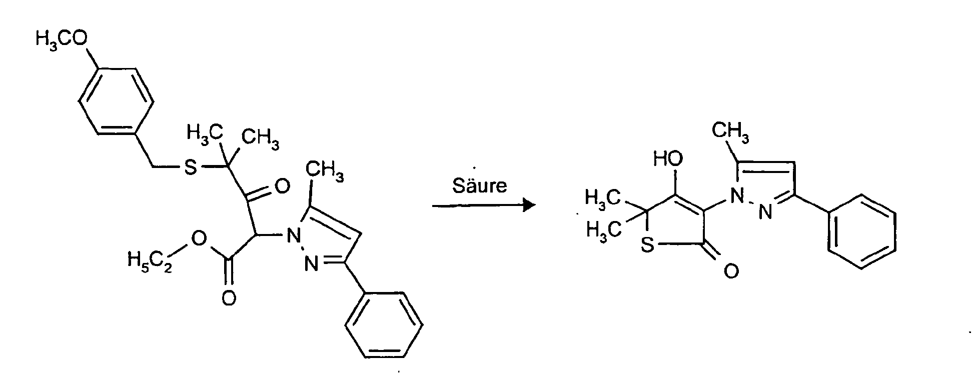

Verwendet man beispielsweise gemäß Verfahren (Dα) 3-[1-(5-Methyl-3-(3-chlorphenyl)-pyrazolyl]-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

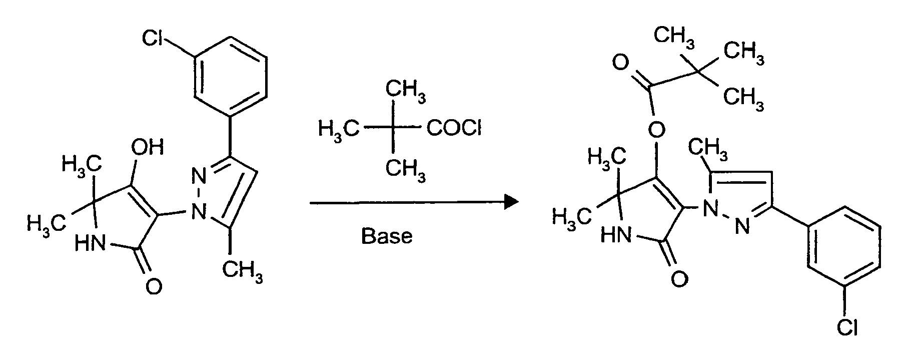

Verwendet man beispielsweise gemäß Verfahren (Dβ) 3-[1-(5-Ethyl-3-(4-chlor-phenyl))-pyrazolyl]-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

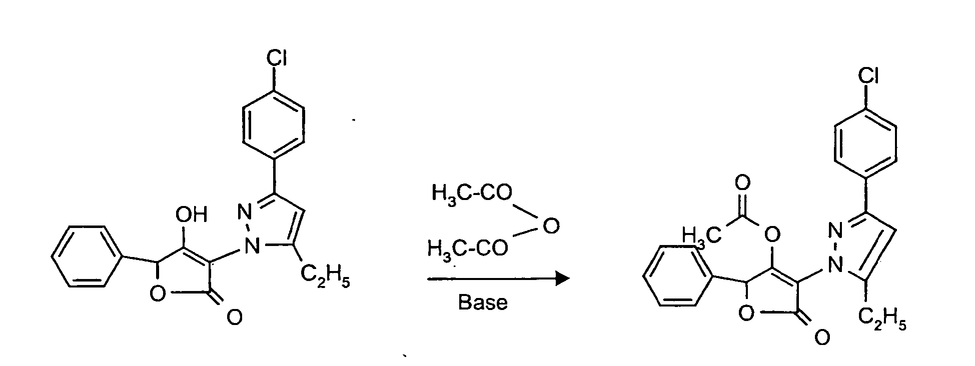

Verwendet man beispielsweise gemäß Verfahren (E) 3-[1-(5-Methyl-3-phenyl)-pyrazolyl]-5,5-dimethylpyrrolidin-2,4-dion und Chlorameisensäureethoxyethylester als Ausgangsverbindungen, so kann der Verlauf des erfindungsgemäßen Verfahrens durch folgendes Reaktionsschema wiedergegeben werden:

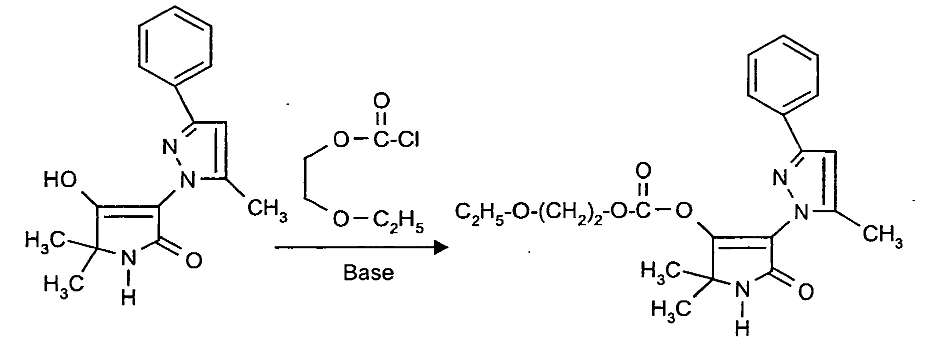

Verwendet man beispielsweise gemäß Verfahren (F), 3-[1-(5-Methyl-3-(4-chlorphenyl))-pyrazolyl]-4-hydroxy-5,5-dimethyl-Δ³-dihydrofuran-2-on und Chlormonothioameisensäuremethylester als Ausgangsprodukte, so kann der Reaktionsverlauf folgendermaßen wiedergegeben werden:

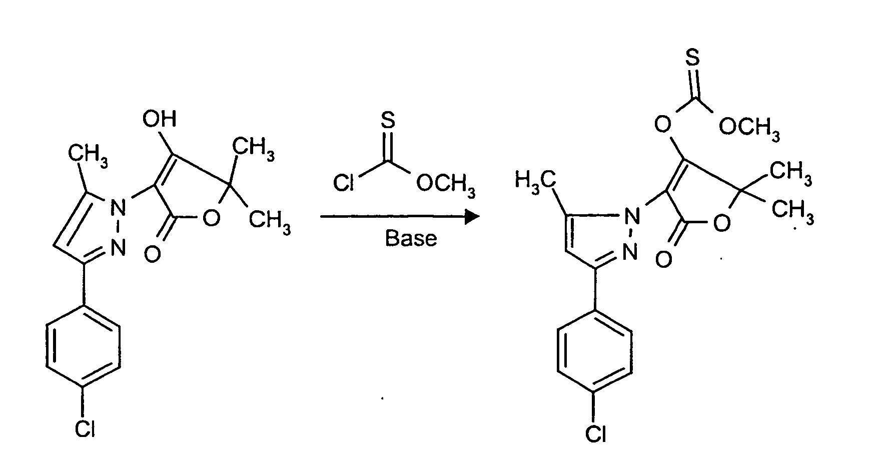

Verwendet man beispielsweise gemäß Verfahren (G) 3-[1-(5-Methyl-3-(4-trifluormethyl-phenyl))-pyrazolyl]-5,5-pentamethylen-pyrrolidin-2,4-dion und Methansulfonsäurechlorid als Ausgangsprodukte, so kann der Reaktionsverlauf durch folgendes Reaktionsschema wiedergegeben werden:

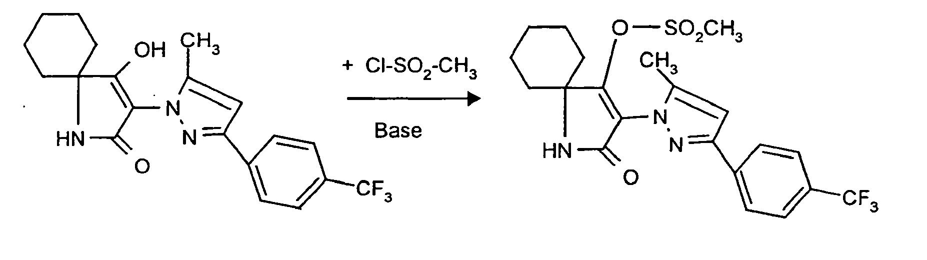

Verwendet man beispielsweise gemäß Verfahren (H) 3-[1-(5-Methyl-3-phenyl)-pyrazolyl]-4-hydroxy-5,5-dirnethyl-Δ³-dihydrofiaran-2-on und Methanthio-phosphonsäurechlorid-(2,2,2-trifluorethylester) als Ausgangsprodukte, so kann der Reaktionsverlauf durch folgendes Reaktionsschema wiedergegeben werden:

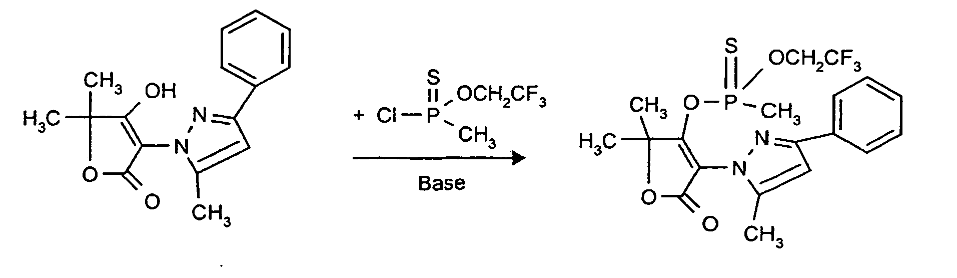

Verwendet man beispielsweise gemäß Verfahren (I) 3-[1-(5-Methyl-3-(4-trifluormethyl-phenyl))-pyrazolyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

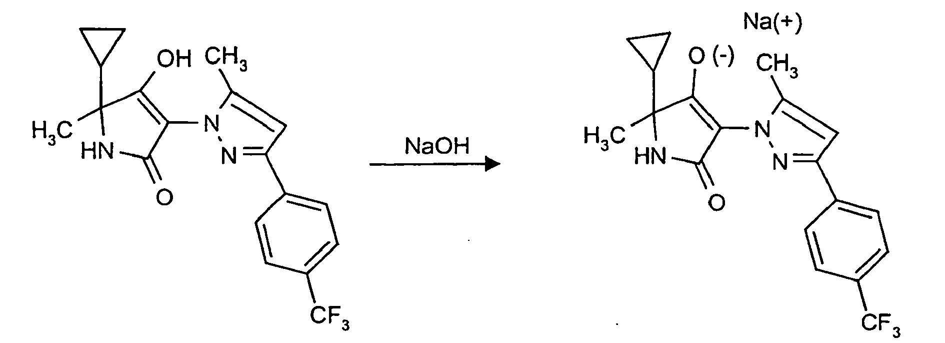

Verwendet man beispielsweise gemäß Verfahren (Jα) 3-[1-(5-Methyl-3-(3-trifluormethyl-phenyl))-pyrazolyl]-4-hydroxy-5-tetramethylen-Δ³-dihydro-furan-2-on und Ethylisocyanat als Ausgangsprodukte, so kann der Reaktionsverlauf durch folgendes Reaktionsschema wiedergegeben werden:

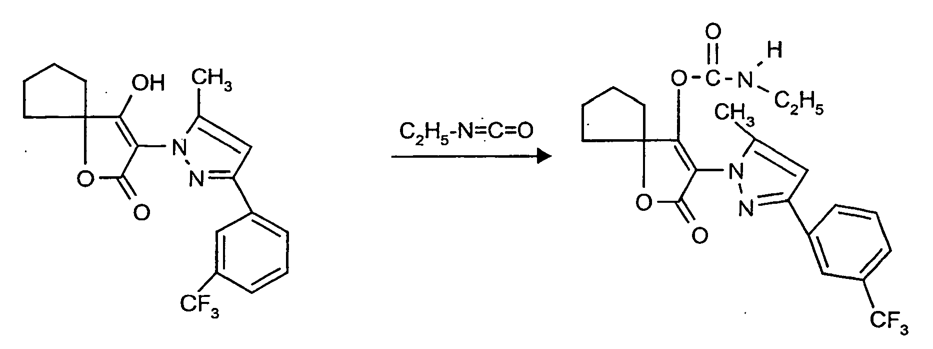

Verwendet man beispielsweise gemäß Verfahren (Jβ) 3-[1-(5-Methyl-3-phenyl)-pyrazolyl]-5-methyl-pyrrolidin-2,4-dion und Dimethylcarbamidsäurechlorid als Ausgangsprodukte, so kann der Reaktionsverlauf durch folgendes Schema wiedergegeben werden:

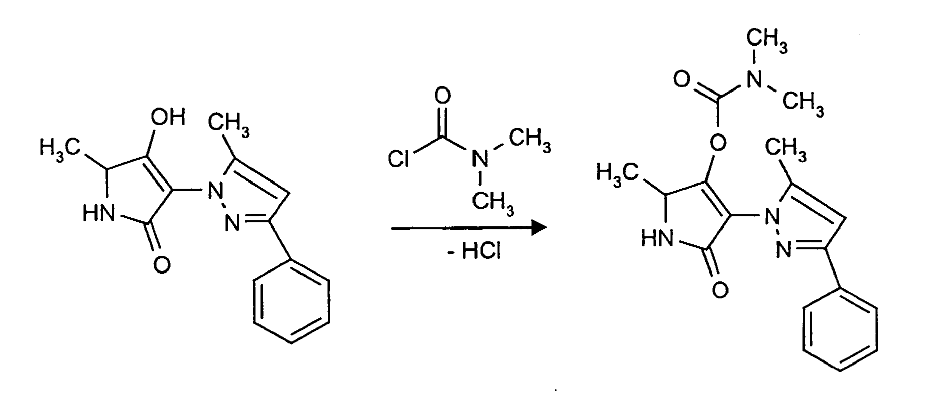

Die beim erfindungsgemäßen Verfahren (A) als Ausgangsstoffe benötigten Verbindungen der Formel (II-A) und (II-B) 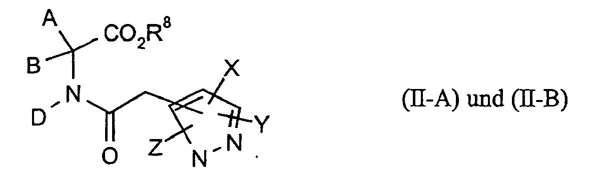 in welcher
A, B, D, X, Y, Z und R⁸ die oben angegebenen Bedeutungen haben,
sind neu.

Man erhält die Acylaminosäureester der Formel (II-A) und (II-B) beispielsweise, wenn man Aminosäurederivate der Formel (XV) 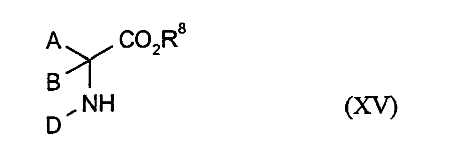 in welcher
- A, B, R⁸ und D: die oben angegebenen Bedeutungen haben,
mit substituierten Pyrazoylessigsäurederivaten der Formel (XVI-A) und (XVI-B) 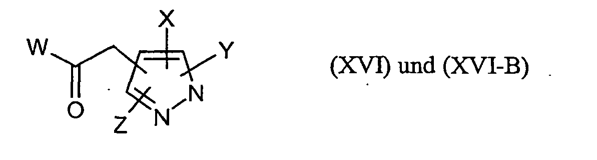 in welcher
- X, Y und Z: die oben angegebenen Bedeutungen haben und
- W: für eine durch Carbonsäureaktivierungsreagenzien wie Carbonyldiimidazol, Carbonyldiimide (wie z.B. Dicyclohexylcarbodiimid), Phosphorylierungsgeagenzien (wie z.B. POCl₃, BOP-Cl), Halogenierungsmittel z.B. Thionylchlorid, Oxalylchlorid, Phosgen oder Chlorameisensäurester eingeführte Abgangsgruppe steht
acyliert (Chem. Reviews 52, 237-416 (1953); Bhattacharya, Indian J. Chem. 6, 341-5, 1968)
oder wenn man Acylaminosäuren der Formel (XVII-A) und (XVII-B) 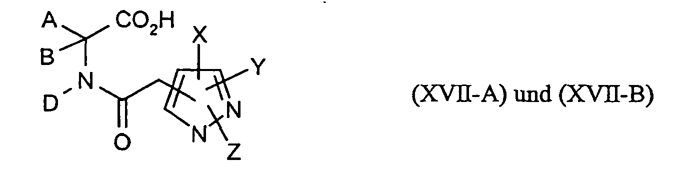 in welcher
A, B, D, X, Y und Z die oben angegebenen Bedeutungen haben,
verestert (Chem. Ind. (London) 1568 (1968)).

Die Verbindungen der Formel (XVII-A) und (XVII-B) 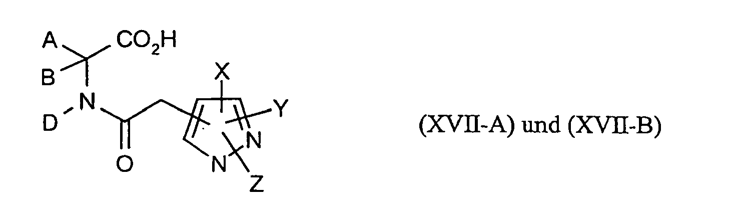 in welcher
A, B, D, X, Y und Z die oben angegebenen Bedeutungen haben,
sind neu.

Man erhält die Verbindungen der Formel (XVII-A) und (XVII-B), wenn man Aminosäuren der Formel (XVIII) 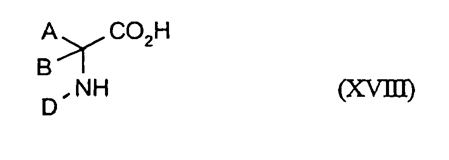 in welcher
A, B und D die oben angegebenen Bedeutungen haben,
mit substituierten Pyrazolylessigsäurederivaten der Formel (XVI-A) und (XVI-B) 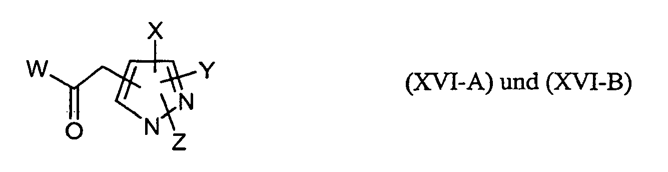 in welcher
- W, X, Y und Z: die oben angegebenen Bedeutungen haben,
beispielsweise nach Schotten-Baumann acyliert (Organikum, VEB Deutscher Verlag der Wissenschaften, Berlin 1977, S. 505).

Die Verbindungen der Formel (XVI-A) sind neu. Die Verbindungen der Formel (XVI-B) sind teilweise neu. Sie lassen sich nach im Prinzip bekannten Verfahren darstellen (EP-A-84822, DE-A-3 203 307, US 4,146,721, DE-A-2 462 459).

Man erhält die Verbindungen der Formel (XVI-A) und (XVI-B) beispielsweise, indem man substituierte Pyrazolylessigsäuren der Formel (XIX-A) und (XIX-B) 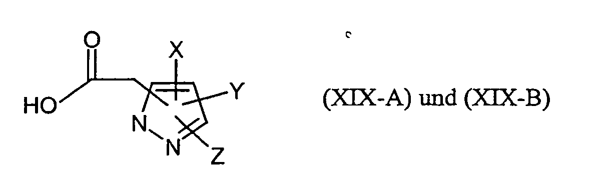 in welcher
- X, Y und Z: die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid) gegebenenfalls in Gegenwart eines Verdünnungsmittels (z.B. gegebenenfalls chlorierten aliphatischen oder aromatischen Kohlenwasserstoffen wie Toluol oder Methylenchlorid) bei Temperaturen von -20°C bis 150°C, bevorzugt von -10°C bis 100°C, umsetzt.

Die Pyrazolylessigsäuren der Formel (XIX-A) sind neu. Sie sind nach im Prinzip bekannten Verfahren herstellbar (Nam, N.L. et. al, Chemistry of Heterocyclic Compounds 34, 382, (1998); L.K. Kulihova, L.V. Cherkesova, Khimiko-Farmatseyticheskii Zhurnal, 8, 18-21, (1974)).

Die Pyrazolylessigsäuren der Formel (XIX-B) sind teilweise käuflich, teilweise bekannt oder lassen sich nach im Prinzip bekannten Verfahren herstellen (US-Patent No. 4,146,721, JP-A-48 028 914, DE-A-1 946 370).

Die Verbindungen der Formel (XV) und (XVIII) sind teilweise käuflich und/oder bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XVIIIa), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen. 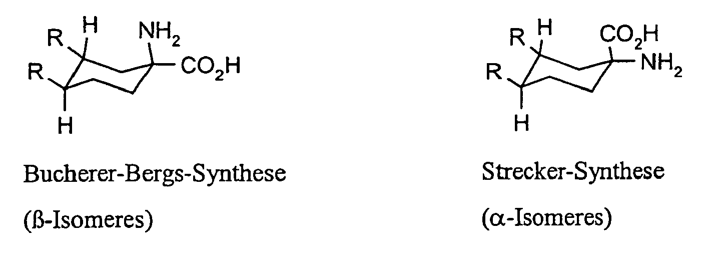 (L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II-A) und (II-B) 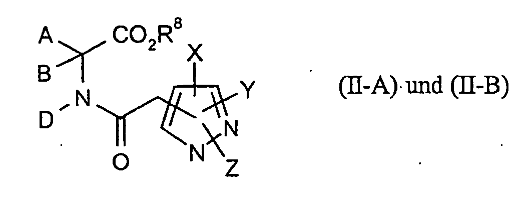 in welcher
- A, B, D, X, Y, Z und R⁸: die oben angegebenen Bedeutungen haben,
herstellen, wenn man Aminonitrile der Formel (XX) 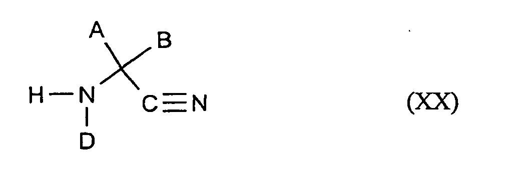 in welcher
- A, B und D: die oben angegebenen Bedeutungen haben,
mit substituierten Pyrazolylessigsäurederivaten der Formel (XVI-A) und (XVI-B) 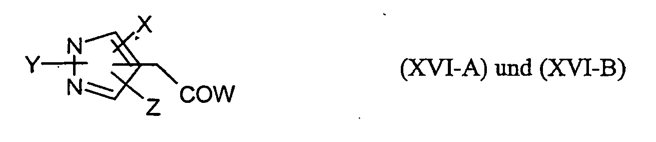 in welcher
- W, X, Y und Z: die oben angegebenen Bedeutungen haben,
zu Verbindungen der Formel (XXI-A) und (XXI-B) 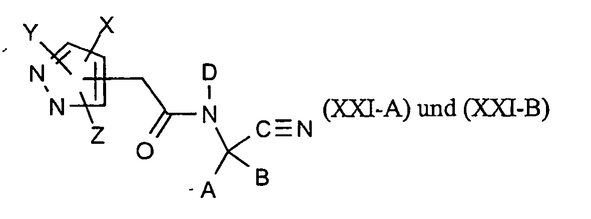 in welcher
A, B, D, X, Y und Z die oben angegebenen Bedeutungen haben,
umsetzt,
und diese anschließend einer sauren Alkoholyse unterwirft.

Die Verbindungen der Formel (XXI-A) und (XXI-B) sind ebenfalls neu.

Die bei dem erfindungsgemäßen Verfahren (B) als Ausgangstoffe benötigten Verbindungen der Formel (III-A) und (III-B) 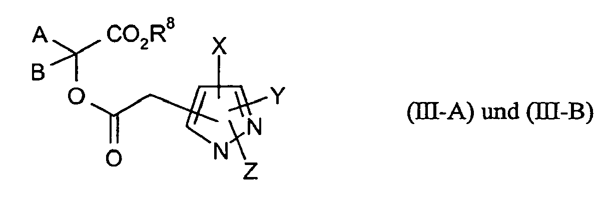 in welcher
A, B, X, Y, Z und R⁸ die oben angegebenen Bedeutungen haben,
sind neu.

Sie lassen sich nach im Prinzip bekannten Methoden herstellen.

So erhält man die Verbindungen der Formel (III-A) und (III-B) beispielsweise, wenn man
2-Hydroxycarbonsäureester der Formel (XXII) 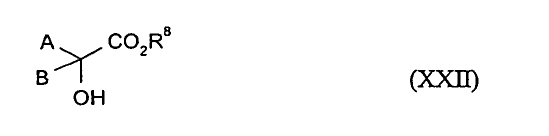 in welcher
A, B und R⁸ die oben angegebenen Bedeutungen haben,
mit substituierten Pyrazolylessigsäurederivaten der Formel (XVI-A) und (XVI-B) 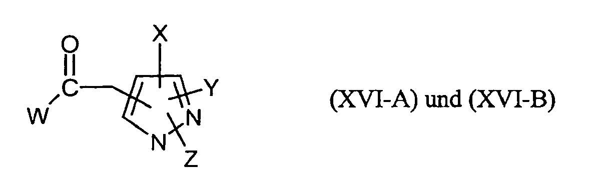 in welcher
- W, X, Y und Z: die oben angegebenen Bedeutungen haben,
acyliert (Chem. Reviews 52, 237-416 (1953).

Weiterhin erhält man Verbindungen der Formel (III-A) und (III-B), wenn man
substituierte Pyrazolylessigsäuren der Formel (XIX-A) und (XIX-B) 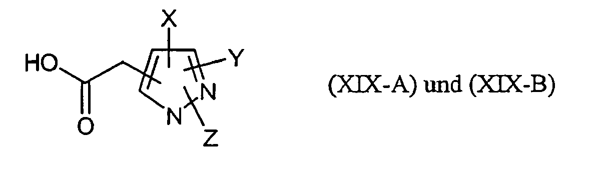 in welcher
- X, Y und Z: die oben angegebenen Bedeutungen haben,
mit α-Halogencarbonsäureestern der Formel (XXIII) 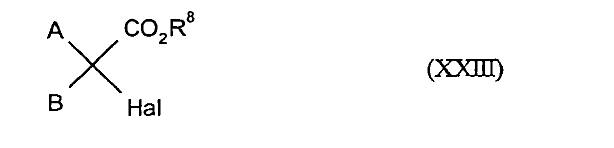 in welcher
- A, B und R⁸: die oben angegebenen Bedeutungen haben und
- Hal: für Chlor oder Brom steht,
alkyliert.

Die bei dem obigen Verfahren (C) als Ausgangsstoffe benötigten Verbindungen der Formel (IV-A) und (IV-B) 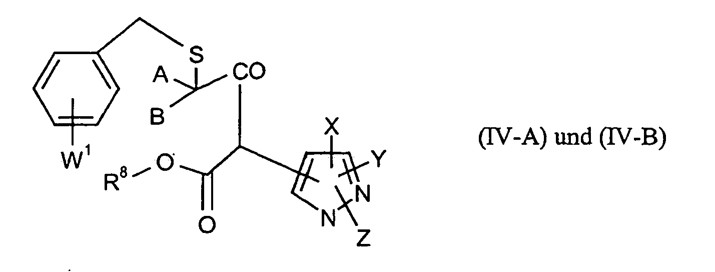 in welcher
A, B, X, Y, Z und R⁸ die oben angegebenen Bedeutungen haben, und
- W¹: für Wasserstoff, Halogen, Alkyl (bevorzugt C₁-C₆-Alkyl) oder Alkoxy (bevorzugt C₁-C₆-Alkoxy) steht,
sind neu.

Sie lassen sich nach im Prinzip bekannten Methoden herstellen.

Man erhält die Verbindungen der Formel (IV) beispielsweise, wenn man
substituierte Pyrazolylessigsäureester der Formel (XXIV-A) und (XXTV-B) 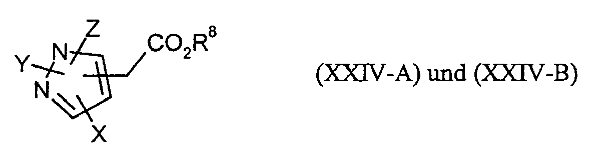 in welcher
- X, Y, Z und R⁸: die oben angegebenen Bedeutungen haben,
mit 2-Benzylthio-carbonsäurehalogeniden der Formel (XXV) 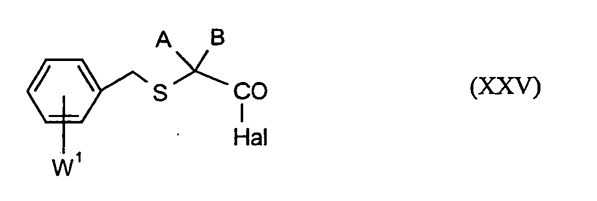 in welcher
- A, B und W¹: die oben angegebenen Bedeutungen haben und
- Hal: für Halogen (insbesondere Chlor oder Brom) steht,
in Gegenwart von starken Basen acyliert (siehe z.B. M.S. Chambers, E.J. Thomas, D.J. Williams, J. Chem. Soc. Chem. Commun., (1987), 1228).

Die Verbindungen der Formel (XXIV-A) sind teilweise neu und lassen sich nach Prinzip bekannten Verfahren herstellen (EP-A-945 437).

Die Verbindungen der Formel (XXIV-B) sind teilweise käuflich, teilweise bekannt und lassen sich nach bekannten Verfahren herstellen (US-Patent-No. 4,146,721, JP-A-48 028 914, DE-A-1 946 370).

Die zur Durchführung der erfindungsgemäßen Verfahren (D), (E), (F), (G), (H), (I) und (J) außerdem als Ausgangsstoffe benötigten Säurehalogenide der Formel (V), Carbonsäureanhydride der Formel (VI), Chlorameisensäureester oder Chlorameisensäurethioester der Formel (VII), Chlormonothioameisensäureester oder Chlordithioameisensäureester der Formel (VIII), Sulfonsäurechloride der Formel (IX), Phosphorverbindungen der Formel (X) und Metallhydroxide, Metallalkoxide oder Amine der Formel (XI) und (XII) und Isocyanate der Formel (XIII) und Carbamidsäurechloride der Formel (XIV) sind allgemein bekannte Verbindungen der Organischen bzw. Anorganischen Chemie.

Die Verbindungen der Formeln (XV), (XVIII), (XX), (XXII), (XXIII) und, (XXV), sind teilweise käuflich, teilweise bekannt und/oder lassen sich nach im Prinzip bekannten Methoden herstellen.

Das Verfahren (A) ist dadurch gekennzeichnet, dass man Verbindungen der Formeln (II-A) oder (II-B), in welcher A, B, D, X, Y, Z und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen -80°C und 180°C, vorzugsweise zwischen -50°C und 120°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formeln (II-A) oder (II-B) und die deprotonierenden Basen im Allgemeinen in etwa doppeltäquimolaren Mengen ein. Es ist jedoch auch möglich, die eine oder andere Komponente in einem größeren Überschuß (bis zu 3 Mol) zu verwenden.

Das Verfahren (B) ist dadurch gekennzeichnet, dass Verbindungen der Formeln (III-A) oder (II-B), in welcher A, B, X, Y, Z und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natriummethylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im Allgemeinen arbeitet man bei Temperaturen zwischen -80°C und 180°C, vorzugsweise zwischen -50°C und 120°C.

Das erfindungsgemäße Verfahren (B) wird im Allgemeinen unter Normaldruck durchgeführt.

Bei der Durchführung des erfindungsgemäßen Verfahrens (B) setzt man die Reaktionskomponenten der Formeln (III-A) oder (III-B) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (C) ist dadurch gekennzeichnet, dass man Verbindungen der Formeln (IV-A) oder (IV-B) in welcher A, B, X, Y, Z und R⁸ die oben angegebene Bedeutung haben, in Gegenwart einer Säure und gegebenenfalls in Gegenwart eines Verdünnungsmittels intramolekular cyclisiert.

Als Verdünnungsmittel können bei dem erfindungsgemäßen Verfahren (C) alle inerten organischen Solventien eingesetzt werden. Vorzugsweise verwendbar sind Kohlenwasserstoffe, wie Toluol und Xylol, ferner halogenierte Kohlenwasserstoffe wie Dichlormethan, Chloroform, Ethylenchlorid, Chlorbenzol, Dichlorbenzol, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Gegebenenfalls kann auch die eingesetzte Säure als Verdünnungsmittel dienen.

Als Säure können bei dem erfindungsgemäßen Verfahren (C) alle üblichen anorganischen und organischen Säuren eingesetzt werden wie z.B. Halogenwasserstoffsäuren, Schwefelsäure, Alkyl-, Aryl- und Haloalkylsulfonsäuren, insbesondere halogenierte Alkylcarbonsäuren wie z.B. Trifluoressigsäure.

Die Reaktionstemperaturen können bei der Durchführung des erfindungsgemäßen Verfahrens (C) innerhalb eines größeren Bereiches variiert werden. Im Allgemeinen arbeitet man bei Temperaturen zwischen -30°C und 250°C, vorzugsweise zwischen 0°C und 150°C.

Das erfindungsgemäße Verfahren (C) wird im Allgemeinen unter Normaldruck durchgeführt.

Bei der Durchführung des erfindungsgemäßen Verfahrens (C) setzt man die Reaktionskomponenten der Formeln (IV-A) oder (IV-B) und die Säure z.B. in äquimolaren Mengen ein. Es ist jedoch gegebenenfalls auch möglich, die Säure als Lösungsmittel oder als Katalysator zu verwenden.

Das Verfahren (D-α) ist dadurch gekennzeichnet, dass man Verbindungen der Formeln (I-1-A-a) bis (I-3-B-a) jeweils mit Carbonsäurehalogeniden der Formel (V) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (D-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylacetamid, Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (D-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (D-α) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (D-α) werden die Ausgangsstoffe der Formeln (I-1-A-a) bis (I-3-B-a) und das Carbonsäurehalogenid der Formel (V) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (D-β) ist dadurch gekennzeichnet, dass man Verbindungen der Formeln (I-1-A-a) bis (I-3-B-a) mit Carbonsäureanhydriden der Formel (VI) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (D-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (D-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (D-β) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (D-β) werden die Ausgangsstoffe der Formeln (I-1-A-a) bis (I-3-B-a) und das Carbonsäureanhydrid der Formel (VI) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuss vorhandenes Carbonsäureanhydrid sowie die entstehende Carbonsäure durch Destillation oder durch Waschen mit einem organischen Lösungsmittel oder mit Wasser entfernt.

Das Verfahren (E) ist dadurch gekennzeichnet, dass man Verbindungen der Formeln (I-1-A-a) bis (I-3-B-a) jeweils mit Chlorameisensäureestern oder Chlorameisensäurethiolestem der Formel (VII) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (E) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (E) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, Nitrile wie Acetonitril und auch stark polare Solventien, wie Dimethylformamid, Dimethylacetamid, Dimethylsulfoxid und Sulfolan.

Die Reaktionstemperaturen können bei der Durchführung des erfindungsgemäßen Verfahrens (E) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (E) wird im Allgemeinen unter Normaldruck durchgeführt.

Bei der Durchführung des erfindungsgemäßen Verfahrens (E) werden die Ausgangsstoffe der Formeln (I-1-A-a) bis (I-3-B-a) und der entsprechende Chlorameisensäureester bzw. Chlorameisensäurethiolester der Formel (VII) im Allgemeinen jeweils in angenähert äquivalenten Mengen verwendet. Es ist jedoch auch möglich, die eine oder andere Komponente in einem größeren Überschuss (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.
Das erfindungsgemäße Verfahren (F) ist dadurch gekennzeichnet, dass man Verbindungen der Formeln (I-1-A-a) bis (I-3-B-a) jeweils mit Verbindungen der Formel (VIII) in Gegenwart eines Verdünnungsmittels und gegebenenfalls in Gegenwart eines Säurebindemittels umsetzt.

Beim Herstellungsverfahren (F) setzt man pro Mol Ausgangsverbindung der Formeln (I-1-A-a) bis (I-3-B-a) ca. 1 Mol Chlormonothioameisensäureester bzw. Chlordithioameisensäureester der Formel (VIII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Nitrile, Ester, Ether, Amide, Sulfone, Sulfoxide, aber auch Halogenalkane.

Vorzugsweise werden Acetonitril, Essigsäureethylester, Dimethylsulfoxid, Tetrahydrofuran, Dimethylformamid oder Methylenchlorid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindungen (I-1-A-a) bis (I-3-B-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgerührt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (G) ist dadurch gekennzeichnet, dass man Verbindungen der Formeln (I-1-A-a) bis (I-3-B-a) jeweils mit Sulfonsäurechloriden der Formel (IX) gegebenenfalls in Gegenwart eines Verdünnungsmittels und gegebenenfalls in Gegenwart eines Säurebindemittels umsetzt.

Beim Herstellungsverfahren (G) setzt man pro Mol Ausgangsverbindung der Formel (I-I-A-a) bis (I-3-B-a) ca. 1 Mol Sulfonsäurechlorid der Formel (IX) bei -20 bis 150°C, vorzugsweise bei 20 bis 70°C um.

Als gegebenenfalls zugesetzte Verdünnungsmittel kommen alle inerten polaren organischen Lösungsmittel in Frage wie Nitrile, Ester, Ether, Amide, Sulfone, Sulfoxide oder halogenierte Kohlenwasserstoffe wie Methylenchlorid.

Vorzugsweise werden Acetonitril, Essigsäureethylester, Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-A-a) bis (1-3-B-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das erfindungsgemäße Verfahren (H) ist dadurch gekennzeichnet, dass man Verbindungen der Formeln (I-1-A-a) bis (I-3-B-a) jeweils mit Phosphorverbindungen der Formel (X) gegebenenfalls in Gegenwart eines Verdünnungsmittels und gegebenenfalls in Gegenwart eines Säurebindemittels umsetzt.

Beim Herstellungsverfahren (H) setzt man zum Erhalt von Verbindungen der Formeln (I-1-A-e) bis (1-3-B-e) auf 1 Mol der Verbindungen (I-1-A-a) bis (I-3-B-a), 1 bis 2, vorzugsweise 1 bis 1,3 Mol der Phosphorverbindung der Formel (X) bei Temperaturen zwischen -40°C und 150°C, vorzugsweise zwischen -10 und 110°C um.

Als gegebenenfalls zugesetzte Verdünnungsmittel kommen alle inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Methylenchlorid eingesetzt.

Als gegebenenfalls zugesetzte Säurebindemittel kommen übliche anorganische oder organische Basen in Frage wie Hydroxide, Carbonate oder Amine. Beispielhaft seien Natriumhydroxid, Natriumcarbonat, Kaliumcarbonat, Pyridin, Triethylamin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Das Verfahren (I) ist dadurch gekennzeichnet, dass man Verbindungen der Formeln (I-1-A-a) bis (I-3-B-a) mit Metallhydroxiden bzw. Metallalkoxiden der Formel (XI) oder Aminen der Formel (XII), gegebenenfalls in Gegenwart eines Verdünnungsmittels, umsetzt.

Als Verdünnungsmittel können bei dem erfindungsgemäßen Verfahren (I) vorzugsweise Ether wie Tetrahydrofuran, Dioxan, Diethylether oder aber Alkohole wie Methanol, Ethanol, Isopropanol, aber auch Wasser eingesetzt werden.

Das erfindungsgemäße Verfahren (I) wird im Allgemeinen unter Normaldruck durchgeführt.

Die Reaktionstemperaturen liegen im Allgemeinen zwischen -20°C und 100°C, vorzugsweise zwischen 0°C und 50°C.

Das erfindungsgemäße Verfahren (J) ist dadurch gekennzeichnet, dass man Verbindungen der Formeln (I-1-A-a) bis (I-3-B-a) jeweils mit Verbindungen der Formel (XIII) (J-α) gegebenenfalls in Gegenwart eines Verdünnungsmittels und gegebenenfalls in Gegenwart eines Katalysators oder (J-β) mit Verbindungen der Formel (XIV) gegebenenfalls in Gegenwart eines Verdünnungsmittels und gegebenenfalls in Gegenwart eines Säurebindemittels umsetzt.

Bei Herstellungsverfahren (J-α) setzt man pro Mol Ausgangsverbindung der Formeln (I-1-A-a) bis (I-3-B-a) ca. 1 Mol Isocyanat der Formel (XIII) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (J-β) setzt man pro Mol Ausgangsverbindung der Formeln (I-1-A-a) bis (I-3-B-a) ca. 1 Mol Carbamidsäurechlorid der Formel (XIV) bei -20 bis 150°C, vorzugsweise bei 0 bis 70°C um.

Als gegebenenfalls zugesetzte Verdünnungsmittel kommen alle inerten polaren organischen Lösungsmittel in Frage wie Nitrile, Ester, Ether, Amide, Sulfone, Sulfoxide oder halogenierte Kohlenwasserstoffe.

Die Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Emtegutes und zur Bekämpfung von tierischen Schädlingen, insbesondere Insekten, Spinnentieren und Nematoden, die in der Landwirtschaft, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp..
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica.
Aus der Ordnung der Dermaptera z.B. Forficula auricularia.
Aus der Ordnung der Isoptera z.B. Reticulitermes spp..
Aus der Ordnung der Phthiraptera z.B. Pediculus humanus corporis, Haematopinus spp., Linognathus spp., Trichodectes spp., Damalinia spp..
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp.,
Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..
Aus der Ordnung der Siphonaptera z.B. Xenopsylla cheopis, Ceratophyllus spp..
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die erfindungsgemäßen Verbindungen können gegebenenfalls in bestimmten Konzentrationen bzw. Aufwandmengen auch als Herbizide und Mikrobizide, beispielsweise als Fungizide, Antimykotika und Bakterizide verwendet werden. Sie lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injezieren und bei Vermehrungsmaterial, insbesondere bei Samen, weiterhin durch ein- oder mehrschichtiges Umhüllen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
**Fungizide:**
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
   Quinconazol, Quintozen (PCNB),
   Schwefel und Schwefel-Zubereitungen,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
   Uniconazol,
   Validamycin A, Vinclozolin, Viniconazol,
   Zarilamid, Zineb, Ziram sowie
   Dagger G,

   OK-8705,
   OK-8801,

   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,

   (5RS,6RS)-6-Hydroxy-2,2,7,7-tetamethyl-5-(1H-1,2,4-triazol-1-yl)-3-octanon, (E)-a-(Methoxyimino)-N-methyl-2-phenoxy-phenylacetamid,

   {2-Methyl-1-[[[1-(4-methylphenyl)-ethyl]-amino]-carbonyl]-propyl}-carbaminsäure-1-isopropylester

   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,2-Dichlor-N-[1-(4-chlorphenyl)-ethyl]-1-ethyl-3-methyl-cyclopropanearboxamid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimetilylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-(1,1-Dimethylethyl)-N-ethyl-N-propyl-1,4-dioxaspiro[4.5]decan-2-methanamin,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarböxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,3-Dichlor-4-hydroxyphenyl)-1-methyl-cyclohexancarboxamid.
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Tricblor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on,
   4-[3,4-Dimethoxyphenyl)-3-(4-fluorphenyl)-acryloyl]-morpholin
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephate, Acetamiprid, Acequinocyl, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Dicofol, Diflubenzuron, Dimethoat, Dimethylvinphos, Dinetofuran, Diofenolan, Disulfoton, Docusat-sodium, Dofenapyn,
   Eflusilanate, Emamectin, Empenthrin, Endosulfan, Entomopfthora spp., Esfenvalerate, Ethiofencarb, Ethion, Ethiprole, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenthion,, Fenvalerate, Fipronil, Fluazinam, Fluazuron, Flubrocythrinate, Flucycloxuron, Flucythrinate, Flufenoxuron, Flumethrin, Flupyrazofos, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Indoxacarb, Isazofos, Isofenphos, Isoxathion, Ivermectin,
   Kernpolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos,
   Ribavirin
   Salithion, Sebufos, Silafluofen, Spinosad, Spirodiclofen, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Theta-cypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii

   YI 5302
   Zeta-cypermethrin, Zolaprofos

   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl-2,2,3,3-tetramethylcyclopropanecarboxylat
   1-[(2-Chlor-5-thiazolyl)methyl]tetrahydro-3,5-dimethyl-N-nitro-1,3,5-triazin-2(1H)-imin
   2-(2-Chlor-6-fluorphenyl)-4-[4-(1,1-dimethylethyl)phenyl]-4,5-dihydro-oxazol
   2-(Acetlyoxy)-3-dodecyl-1,4-naphthalindion
   2-Chlor-N-[[[4-(1-phenylethoxy)-phenyl]-amino]-carbonyl]-benzamid
   2-Chlor-N-[[[4-(2,2-dichlor-1,1-difluorethoxy)-phenyl]-amino]-carbonyl]-benzamid
   3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat
   N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N''-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   N-Cyanomethyl-4-trifluormethyl-nicotinamid
   3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren Safenern bzw. Semichemicals ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acari, (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus. Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen:

Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlomaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches oder ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipät, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid, Triflumuron, Chlothianidin, Spinosad, Tefluthrin,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butylcarbamat, N-Octyl-isothiazolin-3-on und 4,5-Dichlor-N-octylisothiazolin-3-on, sein.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflußkrebse) zusammengefaßt werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindüngsgemäßen Verbindungen allein oder in Kombination mit anderen Wirkstoffen, kann auf den Einsatz von Schwermetallen wie z.B. in Bis-(trialkylzinn)-sulfiden, Tri-*n*-butylzinnlaurat, Tri-*n*-butylzinnchlorid, Kupfer(I)-oxid, Triethylzinnchlorid, Tri-*n*-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-*n*-butylzinnfluorid, Manganethylenbisthiocarbamat, Zinkdimethyldithiocarbamat, Zinkethylenbisthiocarbamat, Zink- und Kupfersalze von 2-Pyridinthiol-1-oxid, Bisdimethyldithiocarbamoylzinkethylenbisthiocarbamat, Zinkoxid, Kupfer(I)-ethylen-bisdithiocarbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert.*-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[*b*]thiophencarbonsäurecyclohexylamid-S,S-dioxid, Dichlofluanid, Fluorfolpet, 3-Iod-2-propinyl-butylcarbamat, Tolylfluanid und Azole wie
   Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb, Fe-chelate;
   oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamoylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylinaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, *Chem. Ind.* 1985, *37*, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wäßrigen System, Vinylchlorid/Vinylacetat-Copolymersysteme in Form wäßriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus.
Aus der Ordnung der Dermaptera z.B. Forficula auricularia.
Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coloptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die erfindungsgemäßen Wirkstoffe können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindemia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe ist jedoch keineswegs auf diese Gattungen beschränkt, sondern erstreckt sich in gleicher Weise auch auf andere Pflanzen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen(-sodium), Aclonifen, Alachlor, Alloxydim(-sodium), Ametryne, Amicarbazone, Amidochlor, Amidosulfuron, Anilofos, Asulam, Atrazine, Azafenidin, Azimsulfuron, Beflubutamid, Benazolin(-ethyl), Benfuresate, Bensulfuron(-methyl), Bentazon, Benzfendizone, Benzobicyclon, Benzofenap, Benzoylprop (-ethyl), Bialaphos, Bifenox, Bispyribac(-sodium), Bromobutide, Bromofenoxim, Bromoxynil, Butachlor, Butafenacil(-allyl), Butroxydim, Butylate, Cafenstrole, Caloxydim, Carbetamide, Carfentrazone(-ethyl), Chlomethoxyfen, Chloramben, Chloridazon, Chlorimuron (-ethyl), Chlornitrofen, Chlorsulfuron, Chlortoluron, Cinidon(-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, -P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Für die Mischungen kommen weiterhin bekannte Safener in Frage, beispielsweise: AD-67, BAS-145138, Benoxacor, Cloquintocet (-mexyl), Cyometrinil, 2,4-D, DKA-24, Dichlormid, Dymron, Fenclorim, Fenchlorazol (-ethyl), Flurazole, Fluxofenim, Furilazole, Isoxadifen (-ethyl), MCPA, Mecoprop (-P), Mefenpyr (-diethyl), MG-191, Oxabetrinil, PPG-1292, R-29148.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Ventunia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Altemaria brassicae;
Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende . Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Wammebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäure-ester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
Fungizide:
   2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarbisopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesil; Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosinesodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxam ide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
   sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxine-copper.
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Allethrin 1R-isomers, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
   Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
   Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifos-methyl, Chlorpyrifos (-ethyl), Chlovaporthrin, Chromafenozide, Cis-Cypermethrin, Cis-Resmethrin, CisPermethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (IR-trans-isomer), Cyromazine,
   DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimethoate, Dimethylvinphos, Dinobuton, Dinocap, Dinotefuran, Diofenolan, Disulfoton, Docusat-sodium, Dofenapyn, DOWCO-439,
   Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
   Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flwnethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
   Gamma-HCH, Gossyplure, Grandlure, Granuloseviren,
   Halfenprox, Halofenozide, HCH, HCN-801, Heptenophos, Hexaflumuron, Hexythiazox, Hydramethylnone, Hydroprene,
   IKA-2002, Imidacloprid, Imiprothrin, Indoxacarb, Iodofenphos, Iprobenfos, Isazofos, Isofenphos, Isoprocarb, Isoxathion, Ivermectin,
   Japonilure,
   Kadethrin, Kempolyederviren, Kinoprene,
   Lambda-Cyhalothrin, Lindane, Lufenuron,
   Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metolcarb, Metoxadiazone, Mevinphos, Milbemectin, Milbemycin, MKI-245, MON-45700, Monocrotophos, Moxidectin, MTI-800,
   Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
   OK-5101, OK-5201, OK-9601, OK-9602, OK-9701, OK-9802, Omethoate, Oxamyl, Oxydemeton-methyl,
   Paecilomyces fumosoroseus, Parathion-methyl, Parathion (-ethyl), Permethrin (cis-, trans-), Petroleum, PH-6045, Phenothrin (IR-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Prallethrin, Profenofos, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
   Quinalphos,
   Resmethrin, RH-5849, Ribavirin, RU-12457, RU-15525,
   S-421, S-1833, Salithion, Sebufos, SI-0009, Silafluofen, Spinosad, Spirodiclofen, Spiromesifen, Sulfluramid, Sulfotep, Sulprofos, SZI-121,
   Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (1R-isomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Triflumuron, Trimethacarb,
   Vamidothion, Vaniliprole, Verbutin, Verticillium lecanii,
   WL-108477, WL-40027,
   YI-5201, YI-5301, YI-5302,
   XMC, Xylylcarb,
   ZA-3274, Zeta-Cypermethrin, Zolaprofos, ZXI-8901,
   die Verbindung 3-Methyl-phenyl-propylcarbamat (Tsumacide Z), die Verbindung 3-(5-Chlor-3-pyridinyl)-8-(2,2,2-trifluorethyl)-8-azabicyclo[3.2.1]-octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfaßbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die Herstellung und die Verwendung der erfindungsgemäßen Wirkstoffe geht aus den nachfolgenden Beispielen hervor.

### Herstellungsbeispiele

### Beispiel-Nr. I-1-A-a-1

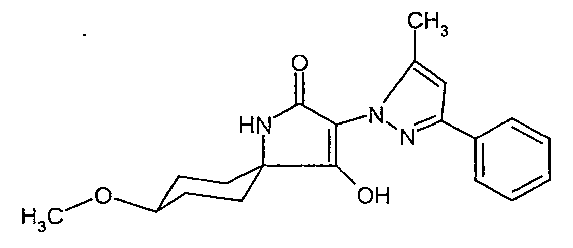

Zu 3,54 g (0,029 mol) Kalium-tert.-butylat in 17 ml wasserfreiem DMF (Dimethylformamid) werden bei 20°C 5,28 g der Verbindung des Beispiels II-A-1 in 11 ml wasserfreiem DMF zugetropft und 1 h bei 40°C nachgerührt.

Die Reaktionslösung rührt man in 150 ml Eiswasser, säuert mit konzentrierter Salzsäure an und dampft das Lösungsmittel ab. Der Rückstand wird zweimal mit 50 ml Methanol ausgekocht und das Methanol im Vakuum eingeengt. Den erhaltenen Rückstand erhitzt man nochmals in 20 ml Methanol, lässt abkühlen und saugt den Niederschlag ab.

Ausbeute: 2,55 g (52 % d. Theorie), Fp. 256°C

In Analogie zu Beispiel (I-1-A-a-1) und gemäß den allgemeinen Angaben zur Herstellung erhält man folgende Verbindungen der Formel (I-1-A-a) und (I-1-B-a)

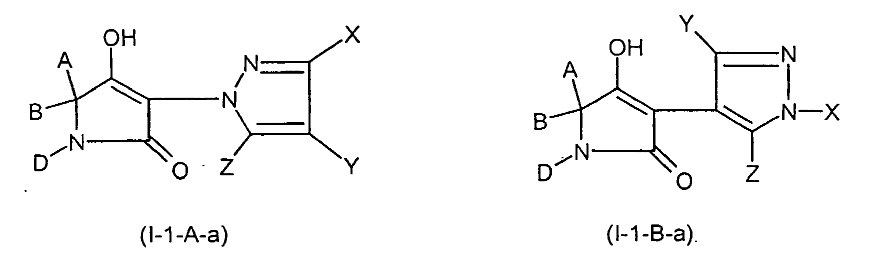

| Bsp.-Nr. | X | Y | Z | D | A | B | Fp.°C | Isomer |
|---|---|---|---|---|---|---|---|---|
| I-1-A-a-2 | 3-Cl-C₆H₄ | H | CH₃ | H | CH₃ | CH₃ | 260 | - |
| I-1-A-a-3 | 3-Cl-C₆H₄ | H | CH₃ | H | -(CH₂)₂-CHOCH₃-(CH₂)₂- | | 270 | β |
| I-1-A-a-4 | 4-Cl-C₆H₄ | H | CH₃ | H | -(CH₂)₂-CHOCH₃-(CH₂)₂- | | >300 | β |
| I-1-A-a-5 | C₆H₅ | H | CH₃ | H | CH₃ | CH₃ | 263 | - |
| I-1-A-a-6 | 4-Cl-C₆H₄ | H | CH₃ | H | -(CH₂)₂-O-(CH₂)₂- | | 371 | - |
| I-1-A-a-7 | 4-Cl-C₆H₄ | H | C₂H₅ | H | -(CH₂)₂-CHOCH₃-(CH₂)₂- | | 245 | β |
| I-1-A-a-8 | 4-Cl-C₆H₄ | H | C₃H₇ | H | -(CH₂)₂-CHOCH₃-(CH₂)₂- | | 375 | β |
| I-1-A-a-9 | 3-CF₃-C₆H₄ | H | CH₃ | H | -(CH₂)₂-CHOCH₃-(CH₂)₂- | | 275 | β |
| I-1-A-a-10 | 3,4-Cl₂-C₆H₃ | H | CH₃ | H | -(CH₂)₂-CHOCH₃-(CH₂)₂- | | 353 | β |
| I-1-A-a-11 | 3-Br-C₆H₄ | H | CH₃ | H | -(CH₂)₂-CHOCH₃-(CH₂)₂- | | 259 | β |
| I-1-A-a-12 | 4-Cl-C₆H₄ | H | CH₃ | 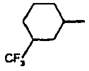 | H | H | 228 | - |
| I-1-A-a-13 | 4-Cl-C₆H₄ | H | CH₃ | -(CH₂)₄- | | H | 164 | - |
| I-1-A-a-14 | 4-Br-C₆H₄ | H | CH₃ | H | -(CH₂)₂-CHOCH₃-(CH₂)₂- | | 241 | β |
| I-1-A-a-15 | 4-Cl-C₆H₄ | H | C₃H₇ | H | CH₃ | CH₃ | 176Z | - |
| I-1-A-a-16 | 4-Cl-C₆H₄ | H | C₂H₅ | H | CH₃ | CH₃ | 260Z | - |
| I-1-A-a-17 | 4-Br-C₆H₄ | H | CH₃ | H | CH₃ | CH₃ | 260Z | - |
| I-1-A-a-18 | 4-Cl-C₆H₄ | H | CH₃ | H | -CH₂-O-(CH₂)₃- | | 250 | - |
| I-1-A-a-19 | 3,4-Cl₂-C₆H₃ | H | CH₃ | H | CH₃ | CH₃ | 254 | - |
| I-1-A-a-20 | 3-CF₃-C₆H₄ | H | CH₃ | H | CH₃ | CH₃ | 228 | - |
| I-1-A-a-21 | 3-Br-C₆H₄ | H | CH₃ | H | CH₃ | CH₃ | 248 | - |
| I-1-B-a-1 | 4-Cl-C₆H₄ | CH₃ | CH₃ | H | -(CH₂)₂-CHOCH₃-(CH₂)₂- | | 315 | β |
| I-1-B-a-2 | 4-Cl-C₆H₄ | CH₃ | CH₃ | H | CH₃ | CH₃ | 279 | - |
| I-1-B-a-3 | 3-CF₃-C₆H₄ | CH₃ | CH₃ | H | -(CH₂)₂-CHOCH₃-(CH₂)₂- | | 245 | β |

| | | | | | | | | |
|---|---|---|---|---|---|---|---|---|
| Z = Zersetzungspunkt | | | | | | | | |

### Beispiel-Nr. I-1-A-b-1

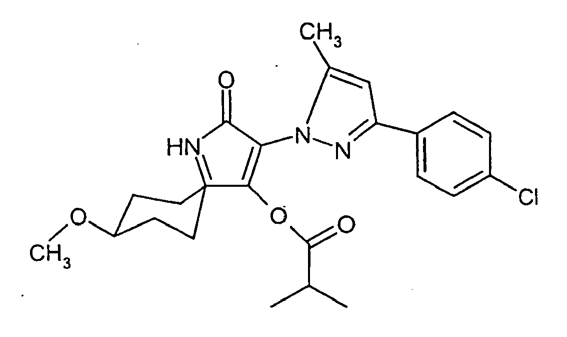

0,78 g der Verbindung gemäß Herstellungsbeispiel I-1-A-a-4 in 30 ml wasserfreiem Essigsäureethylester und 0,3 ml Triethylamin werden unter Rückfluß mit 0,23 g Isobuttersäurechlorid in 2 ml wasserfreiem Essigsäureethylester versetzt. Nach beendeter Reaktion (dünnschichtchromatographische Kontrolle) wird das Lösungsmittel abdestilliert, der Rückstand in 50 ml Dichlormethan aufgenommen, zweimal mit je 10 ml 0,5 N NaOH-Lösung gewaschen, getrocknet und das Lösungsmittel abdestilliert. Es erfolgt säulenchromatographische Reinigung an Kieselgel (Methylenchlorid:Essigsäureethylester, 3: 1).

Ausbeute: 0,65 g (71 % der Theorie, Fp. 226°C

In Analogie zu Beispiel (I-1-A-b-1) und gemäß den allgemeinen Angaben zur Herstellung erhält man folgende Verbindungen der Formel (I-1-A-b) und (I-1-B-b)

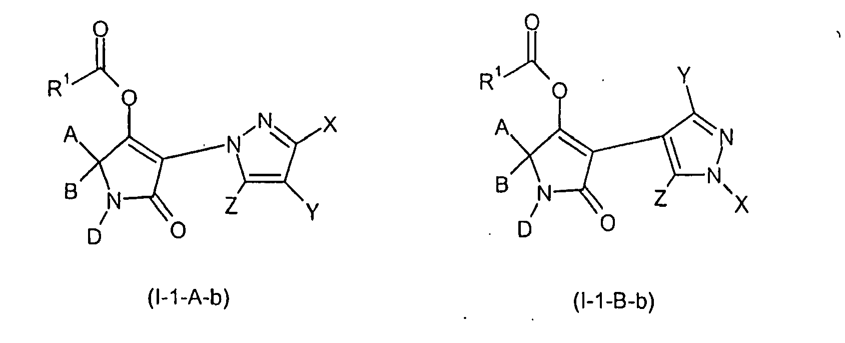

| Bsp.-Nr. | X | Y | Z | D | A | B | R¹ | Fp.°C | Isomer |
|---|---|---|---|---|---|---|---|---|---|
| I-1-A-b-1 | 4-Cl-C₆H₄ | H | CH₃ | H | -(CH₂)₂-CHOCH₃-(CH₂)₂- | | i-C₃H₇ | 226 | β |
| I-1-A-b-2 | 4-Cl-C₆H₄ | H | CH₃ | H | -(CH₂)₂-CHOCH₃-(CH₂)₂- | | 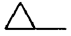 | 209 | β |
| I-1-A-b-3 | 4-Cl-C₆H₄ | H | CH₃ | H | -(CH₂)₂-CHOCH₃-(CH₂)₂- | | 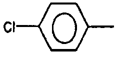 | 226 | β |
| I-1-A-b-4 | 4-Cl-C₆H₄ | H | CH₃ | H | -(CH₂)₂-CHOCH₃-(CH₂)₂- | | 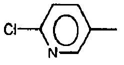 | 231 | β |

### Beispiel-Nr. I-1-A-c-1

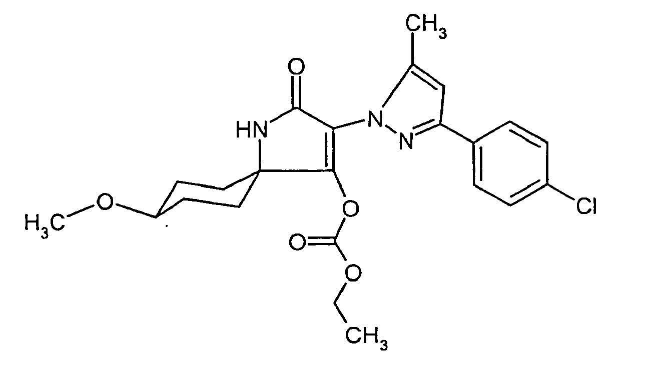

Zu 1,21 g der Verbindung des Beispiels I-1-A-a-4 in 30 ml wasserfreiem Dichlormethan (CH₂Cl₂) gibt man 0,42 ml (3 mmol) Triethylamin. Bei 10 bis 20°C werden 0,3 ml (3 mmol) Chlorameisensäureethylester in 3 ml wasserfreiem CH₂Cl₂ zugetropft.

Es wird bei Raumtemperatur gerührt. Das Reaktionsende wird dünnschichtchromatographisch verfolgt.

Das Lösungsmittel wird einrotiert und der Rückstand in Dichlormethan aufgenommen, zweimal mit 20 ml 0,5 N NaOH gewaschen, getrocknet und einrotiert. Man reinigt säulenchromatographisch über Kieselgel (Dichlormethan/Essigsäureethylester, 3 : 1).

Ausbeute: 0,4 g (29 % d. Theorie), Fp. 174°C

In Analogie zu Beispiel (I-1-A-c-1) und (I-1-B-c-1) und gemäß den allgemeinen Angaben zur Herstellung erhält man folgende Verbindungen der Formel (I-1-A-c) und (I-1-B-c)

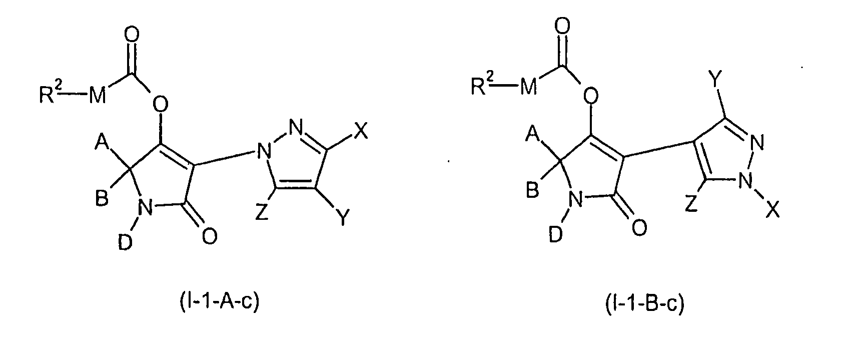

| Bsp.-Nr. | X | Y | Z | D | A | B | M | R² | Fp.°C | Isomer |
|---|---|---|---|---|---|---|---|---|---|---|
| I-1-A-c-2 | 4-Cl-C₆H₄ | H | C₃H₇ | H | -(CH₂)₂-CHOCH₃-(CH₂)₂- | | O | C₂H₅ | 157 | β |
| I-1-A-c-3 | 4-Cl-C₆H₄ | H | CH₃ | H | -(CH₂)₂-O-(CH₂)₂- | | O | C₂H₅ | 156 | - |
| I-1-A-c-4 | 4-Cl-C₆H₄ | H | CH₃ | H | -(CH₂)₂-CHOCH₃-(CH₂)₂- | | O | C₆H₅-CH₂ | 167 | β |
| I-1-A-c-5 | 4-Cl-C₆H₄ | H | CH₃ | H | -(CH₂)₂-CHOCH₃-(CH₂)₂- | | O | C₂H₅ | | β |

### Beispiel-Nr. I-1-B-c-1

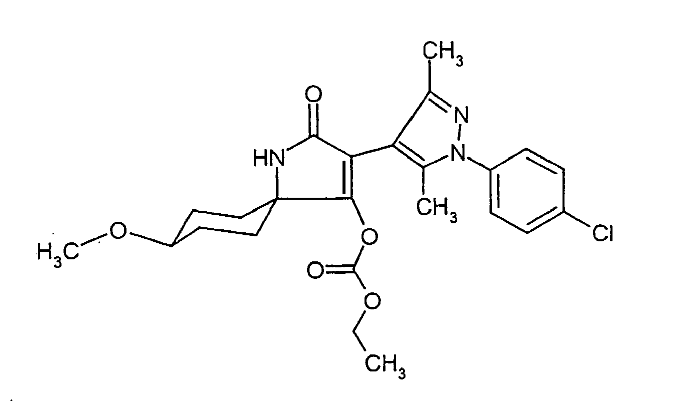

Zu 1,21 g der Verbindung des Beispiels I-1-B-a-1 in 20 ml wasserfreiem CH₂Cl₂ gibt man 0,28 ml (2 mmol) Triethylamin. Bei 10 bis 20°C werden 0,2 ml (2 mmol) Chlorameisensäureethylester in 3 ml wasserfreiem CH₂Cl₂ zugetropft.

Es wird bei Raumtemperatur gerührt. Das Reaktionsende wird dünnschichtchromatographisch verfolgt.

Das Lösungsmittel wird einrotiert und der Rückstand in Dichlormethan aufgenommen, zweimal mit 20 ml 0,5 N NaOH gewaschen, getrocknet und einrotiert. Man reinigt säulenchromatographisch über Kieselgel (Dichlormethan/Essigsäureethylester, 2 : 1).

Ausbeute: 0,84 g (88 % d. Theorie), Fp. 244°C

### Beispiel-Nr. II-A-1

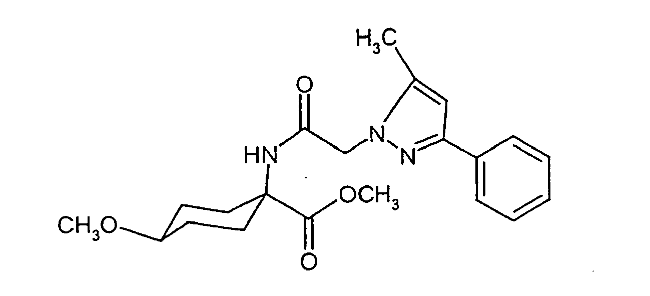

6,08 g 4-Methoxy-1-amino-cyclohexan-carbonsäuremethylester-hydrochlorid in 80 ml absolutem THF und 8 ml Triethylamin werden 5 Minuten gerührt. Dazu gibt man 4,3 g N-(5-Methyl-3-phenyl)-pyrazolylessigsäure und rührt 15 Minuten bei Raumtemperatur. Anschließend gibt man 4,4 ml Triethylamin zu und tropft sofort 1,12 ml Phosphoroxychlorid so zu, dass die Lösung mäßig siedet. Es wird 30 Minuten unter Rückfluss gerührt.

Die Reaktionslösung wird in 400 ml Eiswasser gegeben, mit 7 ml Triethylamin alkalisch gestellt und mit Dichlormethan extrahiert. Es wird getrocknet und einrotiert.

Man reinigt säulenchromatographisch über Kieselgel (Dichlormethan/Aceton 5 : 1)

Ausbeute: 5,28 g (68 % d. Theorie), Fp. 140°C

In Analogie zu Beispiel (II-A-1) und gemäß den allgemeinen Angaben zur Herstellung erhält man folgende Verbindungen der Formel (II-A) und (II-B)

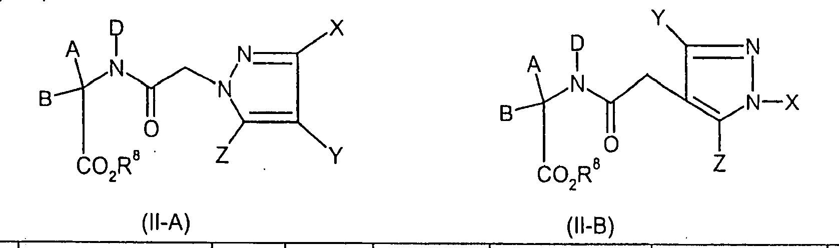

| **Bsp.-Nr.** | **X** | **Y** | **Z** | **D** | **A** | **B** | **R⁸** | **Fp.°C** | **Isomer** |
|---|---|---|---|---|---|---|---|---|---|
| II-A-2 | 3-Cl-C₆H₄ | H | CH₃ | H | CH₃ | CH₃ | CH₃ | 133 | - |
| II-A-3 | 3-Cl-C₆H₄ | H | CH₃ | H | -(CH₂)₂-CHOCH₃-(CH₂)₂- | | CH₃ | 143 | β |
| II-A-4 | C₆H₅ | H | CH₃ | H | CH₃ | CH₃ | CH₃ | 99 | - |
| II-A-5 | 4-Cl-C₆H₄ | H | CH₃ | H | -(CH₂)₂-CHOCH₃-(CH₂)₂- | | CH₃ | 150 | β |
| II-A-6 | 4-Cl-C₆H₄ | H | CH₃ | H | -CH₂-O-(CH₂)₃- | | CH₃ | 155 | - |
| II-A-7 | 4-Cl-C₆H₄ | H | CH₃ | H | -(CH₂)₂-O-(CH₂)₂- | | CH₃ | 169 | - |
| II-A-8 | 4-Cl-C₆H₄ | H | CH₃ | H | CH₃ CH₃ | | CH₃ | | - |
| II-A-9 | 4-Cl-C₆H₄ | H | CH₃ | -(CH₂)₄- | | H | C₂H₅ | 107 | - |
| II-A-10 | 4-Cl-C₆H₄ | H | CH₃ | -(CH₂)₂-S-CH₂- | | H | C₂H₅ | 134 | - |
| II-A-11 | 4-Cl-C₆H₄ | H | CH₃ | i-C₃H₇ | H | H | C₂H₅ | | - |
| II-A-12 | 4-Cl-C₆H₄ | H | C₂H₅ | H -(CH₂)₂-CHOCH₃-(CH₂)₂- | | | CH₃ | 155 | β |
| II-A-13 | 4-Cl-C₆H₄ | H | C₃H₇ | H | -(CH₂)₂-CHOCH₃-(CH₂)₂- | | CH₃ | 127 | β |
| II-A-14 | 3-CF₃-C₆H₄ | H | CH₃ | H | -(CH₂)₂-CHOCH₃-(CH₂)₂- | | CH₃ | 138 | β |
| II-A-15 | 3,4-Cl₂-C₆H₃ | H | CH₃ | H | -(CH₂)₂-CHOCH₃-(CH₂)₂- | | CH₃ | 151 | β |
| II-A-16 | 3-Br-C₆H₄ | H | CH₃ | H | -(CH₂)₂-CHOCH₃-(CH₂)₂- | | CH₃ | 149 | β |
| II-A-17 | 4-Cl-C₆H₄ | H | C₂H₅ | H | CH₃ | CH₃ | CH₃ | 120 | - |
| II-A-18 | 4-Cl-C₆H₄ | H | C₃H₇ | H | CH₃ | CH₃ | CH₃ | 151 | - |
| II-A-19 | 3-CF₃-C₆H₄ | H | CH₃ | H | CH₃ | CH₃ | CH₃ | 268 | - |
| II-A-20 | 3,4-Cl₂-C₆H₃ | H | CH₃ | H | CH₃ | CH₃ | CH₃ | 166 | - |
| II-A-21 | 3-Br-C₆H₄ | H | CH₃ | H | CH₃ | CH₃ | CH₃ | 145 | - |
| II-A-22 | 4-Br-C₆H₄ | H | CH₃ | H | -(CH₂)₂-CHOCH₃-(CH₂)₂- | | CH₃ | 142 | β |
| II-A-23 | 4-Br-C₆H₄ | H | CH₃ | H | CH₃ | CH₃ | CH₃ | 149 | - |
| II-A-24 | 4-Cl-C₆H₄ | H | CH₃ | 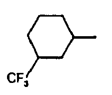 | H | H | C₂H₅ | Öl | - |
| II-B-1 | 4-Cl-C₆H₄ | CH₃ | CH₃ | H | -(CH₂)₂-CHOCH₃-(CH₂)₂- | | CH₃ | 160 | β |
| II-B-2 | 2,4,6-Cl₃-C₆H₂ | CH₃ | CH₃ | H | -(CH₂)₂-CHOCH₃-(CH₂)₂- | | CH₃ | 167 | β |
| II-B-3 | 3-CF₃-C₆H₄ | CH₃ | CH₃ | H | -(CH₂)₂-CHOCH₃-(CH₂)₂- | | CH₃ | 125 | β |
| II-B-4 | 4-Cl-C₆H₄ | CH₃ | CH₃ | H | CH₃ | CH₃ | CH₃ | 155 | - |

### Beispiel-Nr. I-2-A-a-1

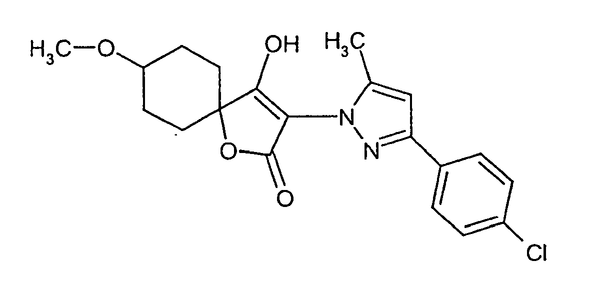

1,9 g (4,5 mmol) der Verbindung gemäß Beispiel III-A-1 werden in 5 ml DMF gelöst. Unter Eiskühlung tropft man 5,35 ml einer 1 molaren Kalium-tert.-butylat-Lösung zu, rührt bei Raumtemperatur 8 h und rotiert das Lösungsmittel ab. Der Rückstand wird in Wasser gelöst, langsam mit konzentrierter HCl angesäuert, abgesaugt und getrocknet.

Ausbeute: 1,01 g (24 % d. Theorie), logP (pH 2,3) 2,05

In Analogie zu Beispiel (I-2-A-a-1) und gemäß den allgemeinen Angaben zur Herstellung erhält man folgende Verbindungen der Formel (I-2-A-a)

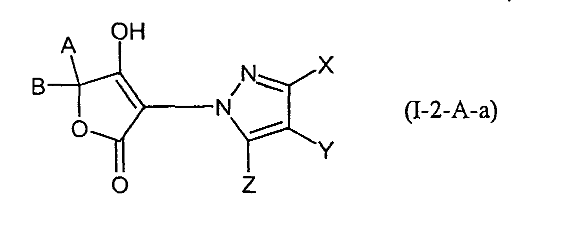

| Bsp.-Nr. | X | Y | Z | A | B | logP (pH 2,3) |
|---|---|---|---|---|---|---|
| I-2-A-a-2 | 4-C₁-C₆H₄ | H | CH₃ | -(CH₂)₂-CHCF₃-(CH₂)₂- | | 2,74 |
| I-2-A-a-3 | 4-Cl-C₆H₄ | H | CH₃ | CH₃ | CH₃ | 1,74 |
| I-2-A-a-4 | 4-Cl-C₆H₄ | H | CH₃ | -(CH₂)₂-CHOC₂H₅-(CH₂)₂- | | 2,46 |
| I-2-A-a-5 | 4-Cl-C₆H₄ | H | CH₃ | 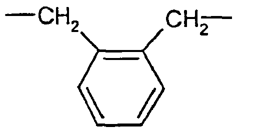 | | 2,35 |
| I-2-A-a-6 | 4-Cl-C₆H₄ | H | CH₃ | -CH₂-O-(CH₂)₃- | | 1,49 |
| I-2-A-a-7 | 4-Cl-C₆H₄ | H | CH₃ | -(CH₂)₂-O-(CH₂)₂- | | 1,55 |
| I-2-A-a-8 | 4-Br-C₆H₄ | H | CH₃ | C₂H₅ | CH₃ | 2,70 |
| I-2-A-a-9 | C₆H₅ | H | CH₃ | i-C₃H₇ | CH₃ | 1,50 |
| I-2-A-a-10 | 3-CF₃-C₆H₄ | H | CH₃ | (CH₃)₃-C-CH₂ | CH₃ | 2,92 |

Die Bestimmung der für die Beispiele (I-2-A-a) angegebenen logP-Werte erfolgte gemäß EEC-Directive 79/831 Annex V.A 8 durch HPLC (High Performance Liquid Chromatography) an einer Phasenumkehrsäule (C 18). Temperatur: 43°C.
(a) Eluenten für die Bestimmung im sauren Bereich: 0,1 % wässrige Phosphorsäure, Acetonitril; linearer Gradient von 10 % Acetonitril bis 90 % Acetonitril.
(b) Eluenten für die Bestimmung im neutralen Bereich: 0,01-molare wässrige Phosphatpuffer-Lösung, Acetonitril; linearer Gradient von 10 % Acetonitril bis 90 % Acetonitril.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Beispiel-Nr. I-2-A-b-1

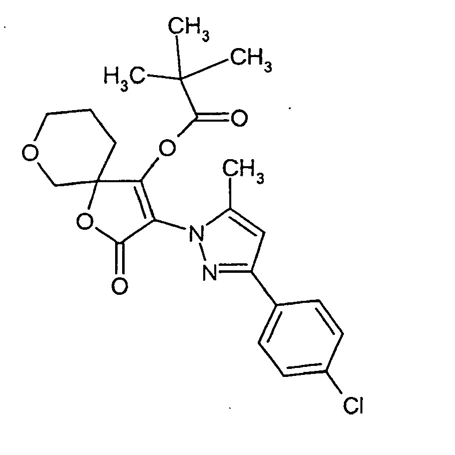

Zu 0,2 g (0,554 mmol) der Verbindung gemäß Herstellungsbeispiel I-2-A-a-6 in 10 ml wasserfreiem Dichlormethan und 0,062 g (0,61 mmol) Triethylamin gibt man unter Eiskühlung 0,074 g (0,61 mmol) Pivalinsäurechlorid und rührt bei Raumtemperatur 8 Stunden.

Die Reaktionslösung wird mit 10 % Zitronensäure und 10 % NaOH-Lösung gewaschen, die organische Phase abgetrennt, getrocknet und das Lösungsmittel abdestilliert.

Ausbeute: 0,141 g (50,4 % d. Theorie), logP (pH 2,3) 4,32.

### Beispiel-Nr. III-A-1

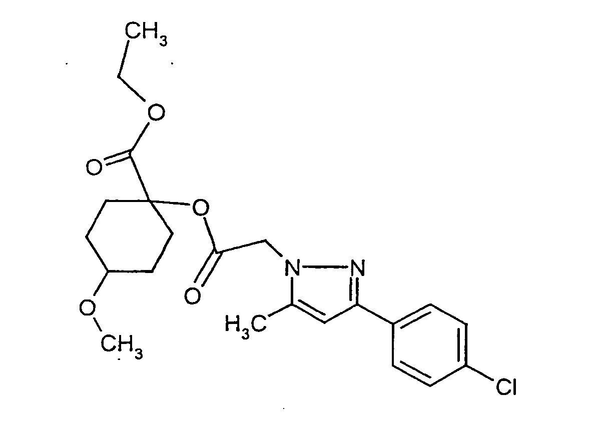

0,9 g (4,5 mmol) 4-Methoxy-1-hydroxy-cyclohexancarbonsäure-ethylester und 1,2 g (4,5 mmol) der Verbindung gemäß Beispiel XVI-A-1 werden 6 h bei 140°C gerührt, abgekühlt und das HCl-Gas mit Argon ausgeblasen.

Ausbeute: 1,93 g (99 % d. Theorie), logP (pH 2,3) 4,11.

In Analogie zu Beispiel (III-A-1) und gemäß den allgemeinen Angaben zur Herstellung erhält man folgende Verbindungen der Formel (III-A)

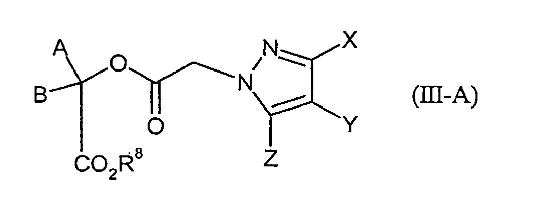

| Bsp.-Nr. | X | Y | Z | A | B | R | Fp.°C |
|---|---|---|---|---|---|---|---|
| III-A-2 | 4-Cl-C₆H₄ | H | CH₃ | -(CH₂)₂-CHCF₃-(CH₂)₂- | | C₂H₅ | *1 |
| III-A-3 | 4-Cl-C₆H₄ | H | CH₃ | CH₃ | CH₃ | C₂H₅ | *1 |
| III-A-4 | 4-Cl-C₆H₄ | H | CH₃ | -(CH₂)₂-CHOC₂H₅-(CH₂)₂- | | C₂H₅ | *1 |
| III-A-5 | 4-Cl-C₆H₄ | H | CH₃ | 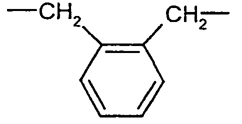 | | C₂H₅ | *1 |
| III-A-6 | 4-Cl-C₆H₄ | H | CH₃ | -CH₂-O-(CH₂)₃- | | C₂H₅ | *1 |
| III-A-7 | 4-Cl-C₆H₄ | H | CH₃ | -(CH₂)₂-O-(CH₂)₂- | | C₂H₅ | *1 |
| III-A-8 | 4-Br-C₆H₄ | H | CH₃ | C₂H₅ | CH₃ | C₂H₅ | *1 |
| III-A-9 | C₆H₅ | H | CH₃ | i-C₃H₇ | CH₃ | C₂H₅ | *1 |
| III-A-10 | 3-CF₃-C₆H₄ | H | CH₃ | (CH₃)₃-C-CH₂ | CH₃ | C₂H₅ | *1 |

| | | | | | | | |
|---|---|---|---|---|---|---|---|
| *1 Die Verbindungen wurden als Rohprodukte zur Herstellung von Verbindungen der Formel (I-2-A-a) verwendet. | | | | | | | |

### Beispiel-Nr. XVI-A-1

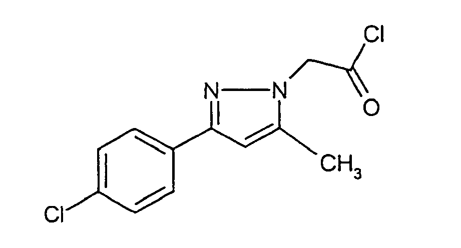

Zu 5,89 g (23 mmol) der Verbindung gemäß Beispiel XIX-A-1 in CH₂Cl₂ wird unter Eiskühlung 4,4 g (34,7 mmol) Oxalsäuredichlorid langsam zugetropft. Man rührt 8 h bei Raumtemperatur und rührt dann 2 h unter Rückfluss. Das Lösungsmittel wird im Vakuum abgezogen.

Ausbeute: 6,22 g (96 % der Theorie)

### Beispiel-Nr. XIX-A-1

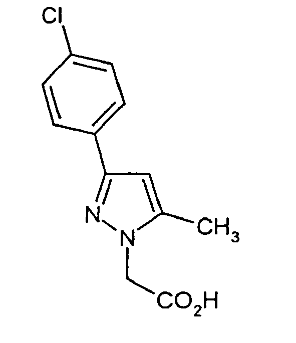

10,7 g (0,04 mol) der Verbindung gemäß Beispiel XXIV-A-1 werden in 20 ml Methanol gelöst und 3,36 g (0,08 mol) Kaliumhydroxid in 13 ml Wasser dazugegeben. Es wird 8 h bei Raumtemperatur gerührt. Das Lösungsmittel wird einrotiert, mit Wasser verrührt und mit konzentrierter Salzsäure auf pH 2 gestellt. Der Niederschlag wird abgesaugt und getrocknet.

Ausbeute: 7,28 g (72 % d. Theorie) Fp. 200°C bis 203°C.

In Analogie zu Beispiel (XIX-A-1) erhält man folgende Verbindungen der Formel (XIX-A)

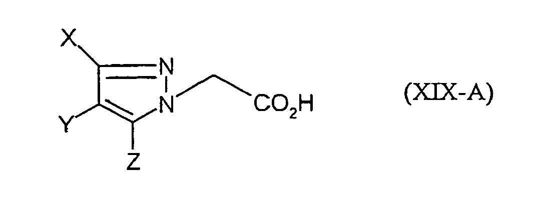

| Bsp.-Nr. | X | Y | Z | Fp.°C |
|---|---|---|---|---|
| XVII-A-2 | 4-Cl-C₆H₄ | H | C₂H₅ | 188 |
| XVII-A-3 | 4-Cl-C₆H₄ | H | C₃H₇ | 181 |
| XVII-A-4 | 3-Br-C₆H₄ | H | CH₃ | 209 |
| XVII-A-5 | 4-Br-C₆H₄ | H | CH₃ | 219 |
| XVII-A-6 | 3-CF₃-C₆H₄ | H | CH₃ | 202 |
| XVII-A-7 | 3,4-Cl₂-C₆H₃ | H | CH₃ | 220 |
| XVII-A-8 | 3-Cl-C₆H₄ | H | CH₃ | 206-207 |
| XVII-A-9 | 3-CF₃-C₆H₄ | H | CH₃ | 200-201 |
| XVII-A-10 | C₆H₅ | H | CH₃ | 173-175 |

### Beispiel-Nr. XXIV-A-1

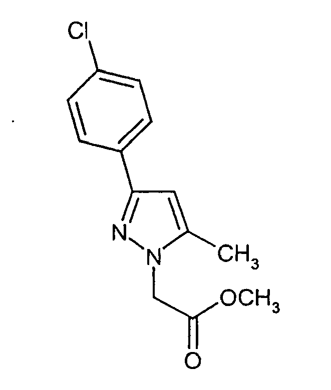

Zu 14 g (0,073mol) 3-(4-Chlorphenyl)-5-methyl-pyrazol und 11,5 g (0,083 mol) Kaliumcarbonat in 209 ml Acetonitril gibt man 12,76 g (0,083 mol) Bromessigsäuremethylester. Man rührt 26 h bei 50°C. Nach dem Abkühlen werden die anorganischen Salze abfiltriert, Methylenchlorid zugegeben und mit gesättigter NaHCO₃-Lösung gewaschen, getrocknet und das Lösungsmittel abrotiert.

Man reinigt säulenchromatographisch über Kieselgel (Dichlormethan/Methanol 99 : 1)

Ausbeute: 10,7 g (55,5 % d. Theorie), Fp. 81 bis 83°C.

In Analogie zu Beispiel (XXIV-A-1) erhält man folgende Verbindungen der Formel (XXIV-A)

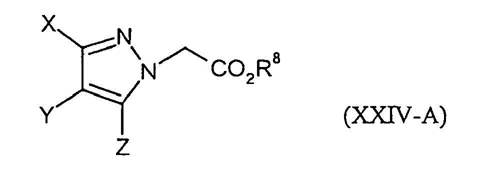

| Bsp.-Nr. | X | Y | Z | R⁸ | Fp.°C |
|---|---|---|---|---|---|
| XXIV-A-2 | 4-Cl-C₆H₄ | H | C₂H₅ | CH₃ | *1 |
| XXIV-A-3 | 4-Cl-C₆H₄ | H | C₃H₇ | CH₃ | *1 |
| XXIV-A-4 | 3-Br-C₆H₄ | H | CH₃ | CH₃ | *1 |
| XXTV-A-5 | 4-Br-C₆H₄ | H | CH₃ | CH₃ | *1 |
| XXIV-A-6 | 3-CF₃-C₆H₄ | H | CH₃ | CH₃ | *1 |
| XXIV-A-7 | 3,4-Cl₂-C₆H₃ | H | CH₃ | CH₃ | *1 |
| XXIV-A-8 | 3-Cl-C₆H₄ | H | CH₃ | CH₃ | Öl |
| XXIV-A-9 | 4-CF₃-C₆H₄ | H | CH₃ | CH₃ | 116 |
| XXIV-A-10 | C₆H₅ | H | CH₃ | CH₃ | Öl |

| | | | | | |
|---|---|---|---|---|---|
| * 1 Die Verbindungen wurden als Rohware zur Herstellung der Verbindungen der Formel (XVII-A) eingesetzt. | | | | | |

Verbindung der Formel XXIV-A-4
   ¹H-NMR 400 MHz (CDCl₃): δ = 2,3 (s, 3H), 3,9 (s, 3H), 4,9 (s, 2H), 6,3 (s, 1H), 7,3 (m, 1H), 7,4 (m, 1H), 8,0 (s, 1H).
Verbindung der Formel XXIV-A-5
   MS (CJ) m/e: 308, 310
Verbindung der Formel XXIV-A-6
   ¹H-NMR 400 MHz, (CDCl₃): δ = 2,1 (s, 3H) 4,9 (s, 2H), 6,4 (s, 1H), 7,75 bis 7,9 (m, 3H), 8,2 (s, 1H).
Verbindung der Formel XXN-A-7
   ¹H-NMR 400 MHz, (CDCl₃): δ = 2,1 (s, 3H), 3,8 (s, 3H), 6,3 (s, 1H), 7,4 (m, 1H), 7,55 (m, 1H), 7,9 (s, 1H).

### Beispiel

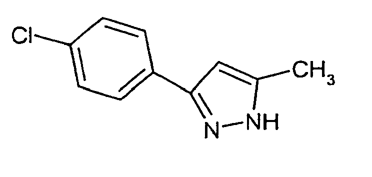

Zu 15 g (0,07 mol) 4-Chlor-benzoyl-aceton in 40 ml Ethanol gibt man 6 g Hydrazin Hydrat und rührt unter Rückfluss 8 h. Der Niederschlag wird abgesaugt, mit Wasser gewaschen und getrocknet.

Ausbeute: 14 g (94 % d. Theorie), Fp. 154 bis 156°C.

### Beispiel A

### Aphis gossypii-Test

| | |
|---|---|
| Lösungsmittel: | 7 Gewichtsteile Dimethylformamid |
| Emulgator: | 2 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Baumwollblätter (Gossypium hirsutum), die stark von der Baumwollblattlaus (Aphis gossypii) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigt z.B. die folgende Verbindung der Herstellungsbeispiele gute Wirksamkeit:

**Tabelle A**

| pflanzenschädigende Insekten | | |
|---|---|---|
| **Aphis gossipii-Test** | | |
| Wirkstoffe | Wirkstoffkonzentration in ppm | Abtötungsgrad in % nach 6^{d} |
| Beispiel I-1-A-a-4 | 500 | 95 |

### Beispiel B

### Myzus-Test

| | |
|---|---|
| Lösungsmittel: | 7 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Bei diesem Test zeigen z.B. die folgenden Verbindungen der Herstellungsbeispiele gute Wirksamkeit:

**Tabelle B**

| pflanzenschädigende Insekten | | |
|---|---|---|
| **Myzus-Test** | | |
| Wirkstoffe | Wirkstoffkonzentration in ppm | Abtötungsgrad in % nach 6^{d} |
| Beispiel I-2-A-a-1 | 500 | 90 |
| Beispiel I-1-A-a-4 | 500 | 90 |

### Beispiel C

### Phaedon-Larven-Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven des Meerrettichblattkäfers (Phaedon cochleariae) besetzt, solange die Blätter noch feucht sind.

Nach der gewünschten Zeit wird die Abtötung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele gute Wirksamkeit:

**Tabelle C**

| pflanzenschädigende Insekten | | |
|---|---|---|
| **Phaedon-Larven-Test** | | |
| Wirkstoffe | Wirkstoffkonzentration in ppm | Abtötungsgrad in % nach 7^{d} |
| Beispiel I-1-A-a-4 | 500 | 100 |
| Beispiel I-1-A-c-1 | 500 | 90 |

### Beispiel D

### Plutella-Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100%, dass alle Raupen abgetötet wurden; 0 % bedeutet, dass keine Raupen abgetötet wurden.

Bei diesem Test zeigt z. B. die folgende Verbindung der Herstellungsbeispiele gute Wirksamkeit:

**Tabelle D**

| pflanzenschädigende Insekten | | |
|---|---|---|
| **Plutella-Test** | | |
| Wirkstoffe | Wirkstoffkonzentration in ppm | Abtötungsgrad in % nach 7^{d} |
| Beispiel I-1-A-a-4 | 500 | 100 |

### Beispiel E

### Spodoptera frugiperda-Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigen z. B. die folgenden Verbindungen der Herstellungsbeispiele gute Wirksamkeit:

**Tabelle E**

| pflanzenschädigende Insekten | | |
|---|---|---|
| **Spodoptera frugiperda -Test** | | |
| Wirkstoffe | Wirkstoffkonzentration in ppm | Abtötungsgrad in % nach 7^{d} |
| Beispiel I-1-A-a-4 | 500 | 100 |
| Beispiel I-I-A-c-1 | 500 | 100 |

### Beispiel F

### Tetranychus-Test (OP-resistent/Tauchbehandlung)

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der Gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele gute Wirksamkeit:

**Tabelle F**

| pflanzenschädigende Milben | | |
|---|---|---|
| **Tetranychus-Test** (OP-resistent/Tauchbehandlung) | | |
| Wirkstoffe | Wirkstoffkonzentration in ppm | Abtötungsgrad in % nach 7^{d} |
| Beispiel I-2-A-a-1 | 100 | 100 |
| Beispiel I-1-A-a-4 | 100 | 95 |
| Beispiel I-1-A-c-1 | 100 | 98 |

### Beispiel G

### Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben, so, dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

### Beispiel H

### Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden mit der Wirkstoffzubereitung bespritzt, so dass die jeweils gewünschten Wirkstoffmengen pro Flächeneinheit ausgebracht werden. Die Konzentration der Spritzbrühe wird so gewählt, dass in 10001 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

| | | | | | | | | |
|---|---|---|---|---|---|---|---|---|
| post-emergence/Gewächshaus | g ai/ha | Alopecurus | Avena fatua | Setaria | Amaranthus | | | |
| Bsp.I-1-B-c-1 | 2000 | 90 | 90 | 95 | 90 | | | |
| | | | | | | | | |
| post-emergence/Gewächshaus | g ai/ha | Alopecurus | Avena fatua | Setaria | Abutilon | Amaranthus | Sinapis | |
| Bsp.I-1-A-c-1 | 250 | 100 | 100 | 100 | 95 | 95 | 80 | |
| | | | | | | | | |
| post-emergence/Gewächshaus | g ai/ha | Zuckerrüben | Alopecurus | Avena fatua | Bromus | Lolium | Setaria | Abutilon |
| Bsp.I-1-A-a-4 | 250 | 10 | 100 | 100 | 100 | 100 | 100 | 80 |
| | | | | | | | | |
| post-emergence/Gewächshaus | g ai/ha | Raps | Alopecurus | Avena fatua | Bromus | Lolium | Setaria | Viola |
| Bsp.I-1-A-a-4 | 125 | 0 | 100 | 100 | 100 | 100 | 100 | 100 |

### Beispiel I

### Grenzkonzentrations-Test / Bodeninsekten - Behandlung transgener Pflanzen

### Testinsekt: Diabrotica balteata - Larven im Boden

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 1 Töpfe und läßt diese bei 20°C stehen.

Sofort nach dem Ansatz werden je Topf 5 vorgekeimte Maiskörner der Sorte YIELD GUARD (Warenzeichen von Monsanto Comp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel J

### Heliothis virescens - Test - Behandlung transgener Pflanzen

| | |
|---|---|
| Lösungsmittel: | 7 Gewichtsteile Aceton |
| Emulgator : | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 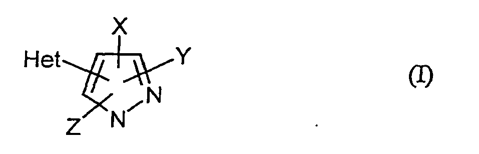 in welcher
X für jeweils gegebenenfalls durch Halogen, C₁-C₆-Alkyl, C₁-C₆-Alkoxy, C₁-C₄-Halogenalkyl, C₁-C₄-Halogenalkoxy, Nitro oder Cyano substituiertes Phenyl steht,
Y für Wasserstoff, Halogen oder C₁-C₆-Alkyl steht,
Z für C₁-C₆-Alkyl, Halogen, Hydroxy, C₁-C₆-Alkoxy, C₁-C₆-Halogenalkoxy oder jeweils gegebenenfalls durch C₁-C₆-Alkyl, C₁-C₆-Alkoxy, Halogen, C₁-C₄-Halogenakyl, C₁-C₆-Halogenalkoxy, Cyano oder Nitro substituiertes Phenyl-C₁-C₂-alkoxy oder gegebenenfalls durch C₁-C₂-Alkyl oder Halogen substituiertes C₃-C₆-Cycloalkyl steht,
Het für eine der Gruppen 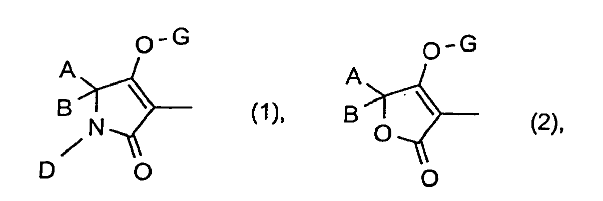 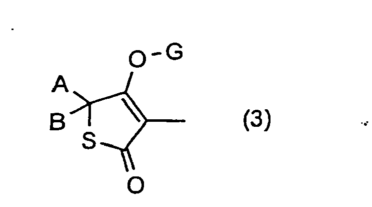 steht
A für Wasserstoff oder jeweils gegebenenfalls durch Fluor oder Chlor substituiertes C₁-C₁₂-Alkyl, C₃-C₈-Akenyl, C₁-C₁₀-Alkoxy-C₁-C₈-alkyl, Poly-C₁-C₈-alkoxy-C₁-C₈-alkyl, C₁-C₁₀-Alkylthio-C₁-C₆-alkyl, gegebenenfalls durch Fluor, Chlor, C₁-C₆-Alkyl oder C₁-C₆-Alkoxy substituiertes C₃-C₈-Cycloalkyl, in welchem gegebenenfalls ein oder zwei nicht direkt benachbarte Ringglieder durch Sauerstoff und/oder Schwefel ersetzt sind oder für jeweils gegebenenfalls durch Halogen, C₁-C₆-Alkyl, C₁-C₆-Halogenalkyl, C₁-C₆-Alkoxy, C₁-C₆-Halogenalkoxy, Cyano oder Nitro substituiertes C₆- oder C₁₀-Aryl, oder C₆- oder C₁₀-Aryl-C₁-C₆-alkyl steht,
B für Wasserstoff, C₁-C₁₂-Alkyl oder C₁-C₈-Alkoxy-C₁-C₆-alkyl steht oder
A, B und das Kohlenstoffatom an das sie gebunden sind, für gesättigtes C₃-C₁₀-Cycloalkyl oder ungesättigtes C₅-C₁₀-Cycloalkyl stehen, worin gegebenenfalls ein Ringglied durch Sauerstoff oder Schwefel ersetzt ist und welche gegebenenfalls einfach oder zweifach durch C₁-C₈-Alkyl, C₃-C₁₀-Cycloalkyl, C₁-C₈-Halogenalkyl, C₁-C₈-Alkoxy, C₁-C₈-Alkylthio, Halogen oder Phenyl substituiert sind oder
A, B und das Kohlenstoffatom, an das sie gebunden sind, für C₅-C₆-Cycloalkyl stehen, welches durch eine gegebenenfalls ein oder zwei nicht direkt benachbarte Sauerstoff- und/oder Schwefelatome enthaltende Alkylendiyl-, oder durch eine Alkylendioxyl- oder durch eine Alkylendithioyl-Gruppe substituiert ist, die mit dem Kohlenstoffatom, an das sie gebunden ist, einen weiteren fünf- bis achtgliedrigen Ring bildet der gegebenenfalls durch C₁-C₄-Alkyl substituiert sein kann, oder
A, B und das Kohlenstoffatom, an das sie gebunden sind, für C₃-C₈-Cycloalkyl oder C₅-C₈-Cycloalkenyl stehen, in welchen zwei Substituenten gemeinsam mit den Kohlenstoffatomen, an die sie gebunden sind, für jeweils gegebenenfalls durch C₁-C₆-Alkyl, C₁-C₆-Alkoxy oder Halogen substituiertes C₂-C₆-Alkandiyl, C₂-C₆-Alkendiyl oder C₄-C₆-Alkandiendiyl stehen, worin gegebenenfalls eine Methylengruppe durch Sauerstoff oder Schwefel ersetzt ist,
D für Wasserstoff, jeweils gegebenenfalls durch Halogen substituiertes C₁-C₁₂-Alkyl, C₃-C₈-Alkenyl, C₃-C₈-Alkinyl, C₁-C₁₀-Alkoxy-C₂-C₈-alkyl, Poly-C₁-C₈-alkoxy-C₂-C₈-alkyl, C₁-C₁₀-Alkylthio-C₂-C₈-alkyl, gegebenenfalls durch Halogen, C₁-C₄-Alkyl, C₁-C₄-Alkoxy oder C₁-C₄-Halogenalkyl substituiertes C₃-C₈-Cycloalkyl, in welchem gegebenenfalls ein Ringglied durch Sauerstoff oder Schwefel ersetzt ist oder jeweils gegebenenfalls durch Halogen, C₁-C₆-Akyl, C₁-C₆-Halogenalkyl, C₁-C₆-Alkoxy, C₁-C₆-Halogenalkoxy, Cyano oder Nitro substituiertes Phenyl, Phenyl-C₁-C₆-alkyl stehen oder
A und D gemeinsam für jeweils gegebenenfalls substituiertes C₃-C₆-Alkandiyl oder C₃-C₆-Alkendiyl stehen, worin gegebenenfalls eine Methylengruppe durch eine Carbonylgruppe, Sauerstoff oder Schwefel ersetzt ist und
wobei als Substituenten jeweils in Frage kommen:
Halogen, Hydroxy, Mercapto oder jeweils gegebenenfalls durch Halogen substituiertes C₁-C₁₀-Alkyl, C₁-C₆-Alkoxy, C₁-C₆-Alkylthio, C₃-C₇-Cyaoalkyl, Phenyl oder Benzyloxy, oder eine weitere C₃-C₆-Alkandiylgruppierung, C₃-C₆-Alkendiylgruppierung oder eine Butadienylgruppierung, die gegebenenfalls durch C₁-C₆-Alkyl substituiert ist oder in der gegebenenfalls zwei benachbarte Substituenten mit den Kohlenstoffatomen, an die sie gebunden sind, einen weiteren gesättigten oder ungesättigten Cyclus mit 5 oder 6 Ringatomen bilden (im Fall der Verbindung der Formel (I-1) stehen A und D dann gemeinsam mit den Atomen, an die sie gebunden sind beispielsweise für die weiter unten genannten Gruppen AD-1 bis AD-10), der Sauerstoff oder Schwefel enthalten kann,
G für Wasserstoff (a) oder für eine der Gruppen 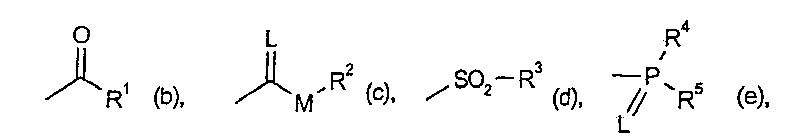 E(f) oder 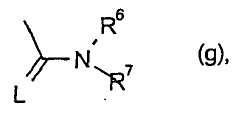 steht in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₁-C₈-alkyl oder gegebenenfalls durch Halogen, C₁-C₆-Alkyl oder C₁-C₆-Alkoxy substituiertes C₃-C₈-Cycloalkyl, in welchem gegebenenfalls ein oder mehrere nicht direkt benachbarte Ringglieder durch Sauerstoff und/oder Schwefel ersetzt sind,
für gegebenenfalls durch Halogen, Cyano, Nitro, C₁-C₆-Alkyl, C₁-C₆-Alkoxy, C₁-C₆-Halogenalkyl, C₁-C₆-Halogenalkoxy, C₁-C₆-Alkylthio oder C₁-C₆-Alkylsulfonyl substituiertes Phenyl,
für gegebenenfalls durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls durch Halogen, C₁-C₆-Alkyl, C₁-C₂-Halogenalkyl oder C₁-C₄-Alkoxy substituiertes 5- oder 6-gliedriges Hetaryl,
für gegebenenfalls durch Halogen oder C₁-C₆-Alkyl substituiertes Phenoxy-C₁-C₆-alkyl oder für gegebenenfalls durch Halogen, Amino oder C₁-C₆-Alkyl substituiertes 5- oder 6-gliedriges Hetaryloxy-C₁-C₆-alkyl steht,
R² für jeweils gegebenenfalls durch Halogen substituiertes C₁-C₂₀-Alkyl, C₂-C₂₀-Alkenyl, C₁-C₈-Alkoxy-C₂-C₈-alkyl, Poly-C₁-C₈-alkoxy-C₂-C₈-alkyl,
für gegebenenfalls durch Halogen, C₁-C₆-Alkyl oder C₁-C₆-Alkoxy substituiertes C₃-C₈-Cycloalkyl in welchem gegebenenfalls ein Ringatom durch Sauerstoff ersetzt ist, oder
für jeweils gegebenenfalls durch Halogen, Cyano, Nitro, C₁-C₆-Alkyl, C₁-C₆-Alkoxy, C₁-C₆-Halogenalkyl oder C₁-C₆-Halogenalkoxy substituiertes Phenyl oder Benzyl steht,
R³ für gegebenenfalls durch Halogen substituiertes C₁-C₈-Alkyl oder für jeweils gegebenenfalls durch Halogen, C₁-C₆-Alkyl, C₁-C₆-Alkoxy, C₁-C₄-Halogenalkyl, C₁-C₄-Halogenalkoxy, Cyano oder Nitro substituiertes Phenyl oder Benzyl steht,
R⁴ und R⁵ unabhängig voneinander für jeweils gegebenenfalls durch Halogen substituiertes C₁-C₈-Alkyl, C₁-C₈-Alkoxy, C₁-C₈-Alkylamino, Di-(C₁-C₈-alkyl)amino, C₁-C₈-Alkylthio, C₂-C₈-Alkenylthio, C₃-C₇-Cycloalkylthio oder für jeweils gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen,
R⁶ und R⁷ unabhängig voneinander für Wasserstoff, für jeweils gegebenenfalls durch Halogen substituiertes C₁-C₈-Alkyl, C₃-C₈-Cycloalkyl, C₁-C₈-Akoxy, C₃-C₈-Alkenyl, C₁-C₈-Alkoxy-C₁-C₈-alkyl, für gegebenenfalls durch Halogen, C₁-C₈-Halogenalkyl, C₁-C₈-Alkyl oder C₁-C₈-Alkoxy substituiertes Phenyl, gegebenenfalls durch Halogen, C₁-C₈-Alkyl, C₁-C₈-Halogenalkyl oder C₁-C₈-Alkoxy substituiertes Benzyl oder zusammen für einen gegebenenfalls durch C₁-C₄-Alkyl substituierten C₃-C₆-Alkylenrest stehen, in welchem gegebenenfalls ein Kohlenstoffatom durch Sauerstoff oder Schwefel ersetzt ist,
wobei die Verknüpfungsposition am Pyrazolylrest festgelegt wird auf folgende Strukturen:

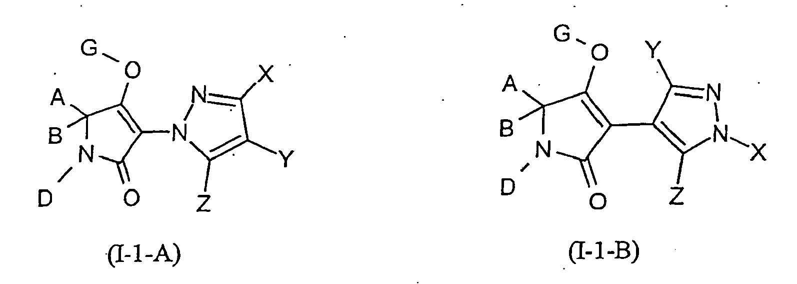

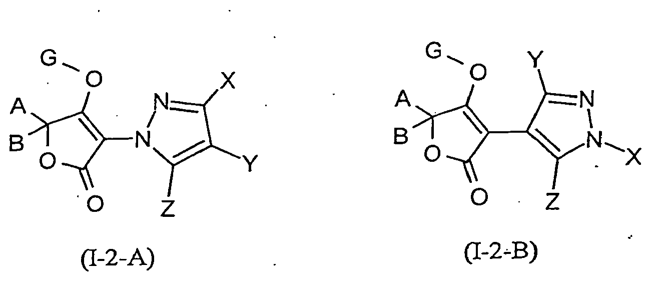

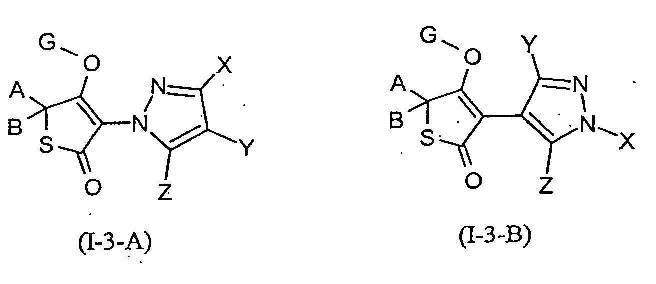

2. Verbindungen der Formel (I) gemäß Anspruch 1, in welcher
X für jeweils gegebenenfalls einfach bis dreifach durch Fluor, Chlor, Brom, C₁-C₄-Alkyl, C₁-C₄-Alkoxy, C₁-C₂-Halogenalkyl, C₁-C₂-Halogenalkoxy, Nitro oder Cyano substituiertes Phenyl, steht,
Y für Wasserstoff, Chlor, Brom oder C₁-C₄-Alkyl steht,
Z für C₁-C₄-Alkyl, Chlor, Brom, C₁-C₄-Alkoxy, C₁-C₄-Halogenalkoxy oder jeweils gegebenenfalls einfach bis zweifach durch C₁-C₄-Alkyl, C₁-C₄-Alkoxy, Fluor, Chlor, Brom, C₁-C₂-Halogenalkyl, C₁-C₂-Halogenalkoxy, Cyano oder Nitro substituiertes Benzyloxy steht,
Het für eine der Gruppen 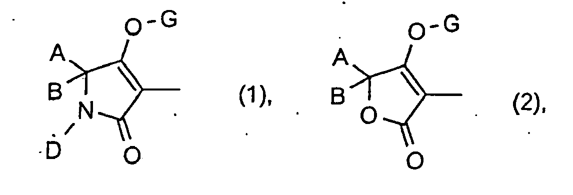 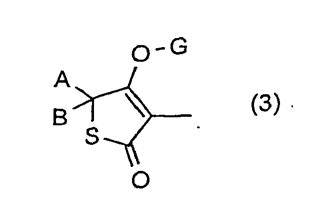 steht,
A für Wasserstoff, jeweils gegebenenfalls durch Fluor substituiertes C₁-C₁₀-Alkyl, C₁-C₈-Alkoxy-C₁-C₆-alkyl, gegebenenfalls durch Fluor, Chlor, C₁-C₄-Alkyl oder C₁-C₄-Alkoxy substituiertes C₃-C₇-Cycloalkyl, in welchem gegebenenfalls ein Ringglied durch Sauerstoff oder Schwefel ersetzt ist oder jeweils gegebenenfalls durch Fluor, Chlor, Brom, C₁-C₄-Alkyl, C₁-C₄-Halogenalkyl, C₁-C₄-Alkoxy, Cyano, Nitro oder C₁-C₄-Halogenalkoxy substituiertes Phenyl oder Phenyl-C₁-C₂-alkyl steht,
B für Wasserstoff oder C₁-C₁₀-Alkyl steht oder
A, B und das Kohlenstoffatom an das sie gebunden sind, für gesättigtes C₃-C₇-Cycloalkyl oder ungesättigtes C₅-C₇-Cycloalkyl stehen, worin gegebenenfalls ein Ringglied durch Sauerstoff oder Schwefel ersetzt ist und welches gegebenenfalls einfach oder zweifach durch C₁-C₆-Alkyl, C₅-C₈-Cycloalkyl, C₁-C₃-Halogenalkyl, C₁-C₆-Alkoxy, Fluor, Chlor oder Phenyl substituiert ist oder
A, B und das Kohlenstoffatom, an das sie gebunden sind, für C₅-C₆-Cycloalkyl stehen, welches durch eine gegebenenfalls ein oder zwei nicht direkt benachbarte Sauerstoff- oder Schwefelatome enthaltende Alkylendiyl- oder durch eine Alkylendioxyl- oder durch eine Alkylendithiolyl-Gruppe substituiert ist, die mit dem Kohlenstoffatom, an das sie gebunden ist, einen weiteren fünf- oder sechsgliedrigen Ring bildet der gegebenenfalls durch C₁-C₃-Alkyl substituiert sein kann, oder
A, B und das Kohlenstoffatom, an das sie gebunden sind, für C₅-C₆-Cycloalkyl oder C₅-C₆-Cycloalkenyl stehen, in welchen zwei Substituenten gemeinsam mit den Kohlenstoffatoinen, an die sie gebunden sind, für jeweils gegebenenfalls durch C₁-C₅-Alkyl, C₁-C₅-Alkoxy, Fluor, Chlor oder Brom substituiertes C₂-C₄-Alkandiyl, C₂-C₄-Alkendiyl, worin gegebenenfalls eine Methylengruppe durch Sauerstoff oder Schwefel ersetzt ist oder Butadiendiyl stehen,
D für Wasserstoff, für jeweils gegebenenfalls durch Fluor substituiertes C₁-C₁₀-Alkyl, C₃-C₆-Alkenyl, C₁-C₆-Alkoxy-C₂-C₄-alkyl oder C₁-C₆-Alkylthio-C₂-C₄-alkyl, für gegebenenfalls durch Fluor, C₁-C₄-Alkyl, C₁-C₄-Akoxy oder C₁-C₂-Halogenalkyl substituiertes C₃-C₇-Cycloalkyl, in welchem gegebenenfalls eine Methylengruppe durch Sauerstoff oder Schwefel ersetzt ist oder für jeweils gegebenenfalls durch Fluor, Chlor, Brom, C₁-C₄-Alkyl, C₁-C₄-Halogenalkyl, C₁-C₄-Alkoxy oder C₁-C₄-Halogenalkoxy substituiertes Phenyl, oder Phenyl-C₁-C₄-alkyl steht oder
A und D gemeinsam für C₃-C₅-Alkandiyl stehen, in welchem gegebenenfalls eine Methylengruppe durch Sauerstoff oder Schwefel ersetzt sein kann, wobei als Substituenten C₁-C₆-Alkyl oder C₁-C₄-Alkoxy in Frage kommen oder
A und D stehen (im Fall der Verbindungen der Formel (I-1)) gemeinsam mit den Atomen, an die sie gebunden sind, für eine der Gruppen AD-1 bis AD-10: 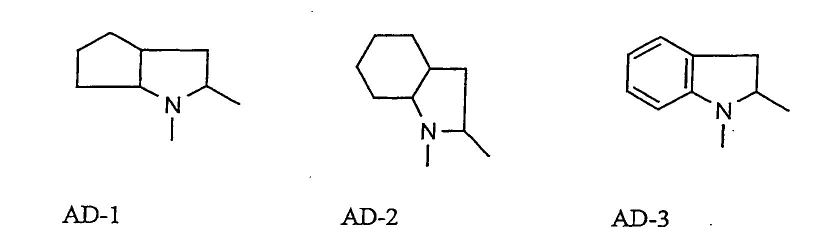 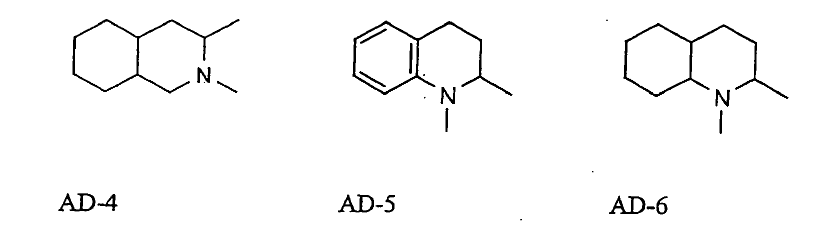 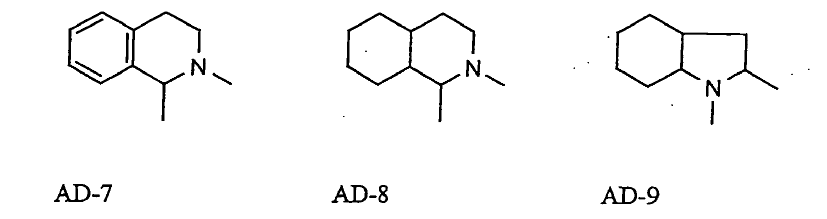 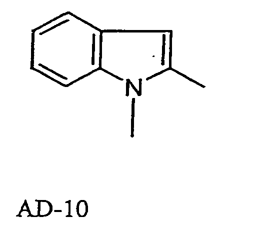 stehen,
G für Wasserstoff (a) oder für eine der Gruppen 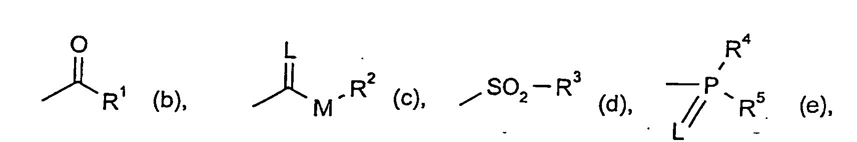 E (f) oder 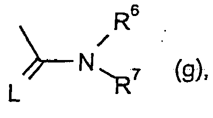 steht
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₆-Alkyl, -C₂-C₁₆-Alkenyl, C₁-C₆-Akoxy-C₁-C₄-alkyl, C₁-C₆-Alkylthio-C₁-C₄-alkyl oder gegebenenfalls durch Fluor, Chlor, C₁-C₅-Alkyl oder C₁-C₅-Alkoxy substituiertes C₃-C₇-Cyloalkyl, in welchem gegebenenfalls ein oder zwei nicht direkt benachbarte Ringglieder durch Sauerstoff und/oder Schwefel ersetzt sind,
für gegebenenfalls durch Fluor, Chlor, Brom, Cyano, Nitro, C₁-C₄-Alkyl, C₁-C₄-Alkoxy, C₁-C₃-Halogenalkyl, C₁-C₃-Halogenalkoxy, C₁-C₄-Alkylthio oder C₁-C₄-Alkylsulfonyl substituiertes Phenyl,
für gegebenenfalls durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls durch Fluor, Chlor, Brom, C₁-C₄-Alkyl, Trifluormethyl oder C₁-C₂-Alkoxy substituiertes Pyrazolyl, Thiazolyl, Pyridyl, Pyrimidyl, Furanyl oder Thienyl steht,
R² für jeweils gegebenenfalls durch Fluor substituiertes C₁-C₁₆-Alkyl, C₂-C₁₆-Alkenyl oder C₁-C₆-Alkoxy-C₂-C₆-alkyl,
für gegebenenfalls durch Fluor, Chlor, C₁-C₄-Alkyl oder C₁-C₄-Alkoxy substituiertes C₃-C₇-Cycloalkyl oder für jeweils gegebenenfalls durch Fluor, Chlor, Brom, Cyano, Nitro, C₁-C₄-Alkyl, C₁-C₃-Alkoxy, C₁-C₂-Halogenalkyl oder C₁-C₂-Halogenalkoxy substituiertes Phenyl oder Benzyl steht,
R³ für gegebenenfalls durch Fluor substituiertes C₁-C₆-Alkyl oder für jeweils gegebenenfalls durch Fluor, Chlor, Brom, C₁-C₄-Alkyl, C₁-C₄-Alkoxy, C₁-C₃-Halogenalkyl, C₁-C₃-Halogenalkoxy, Cyano oder Nitro substituiertes Phenyl steht,
R⁴ für C₁-C₆-Alkyl, C₁-C₆-Alkoxy, C₁-C₆-Alkylamino, Di-(C₁-C₆-alkyl)amino, C₁-C₆-Alkylthio, C₃-C₄-Alkenylthio, C₃-C₆-Cycloalkylthio oder für jeweils gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio steht,
R⁵ für C₁-C₆-Alkoxy oder C₁-C₆-Alkylthio steht,
R⁶ für C₁-C₆-Alkyl, C₃-C₆-Cycloalkyl, C₁-C₆-Alkoxy, C₃-C₆-Alkenyl, C₁-C₆-Alkoxy-C₁-C₆-alkyl, für gegebenenfalls durch Fluor, Chlor, Brom, C₁-C₃-Halogenalkyl, C₁-C₄-Alkyl oder C₁-C₄-Alkoxy substituiertes Phenyl, für gegebenenfalls durch Fluor, Chlor, Brom, C₁-C₄-Alkyl, C₁-C₃-Halogenalkyl oder C₁-C₄-Alkoxy substituiertes Benzyl steht,
R⁷ für Wasserstoff, C₁-C₆-Alkyl, C₃-C₆-Alkenyl steht,
R⁶ und R⁷ zusammen für einen gegebenenfalls durch Methyl oder Ethyl substituierten C₄-C₅-Alkylenrest stehen, in welchem gegebenenfalls eine Methylengruppe durch Sauerstoff oder Schwefel ersetzt ist,
wobei die Verknüpfungsposition am Pyrazolylrest festgelegt wird auf folgende Strukturen:

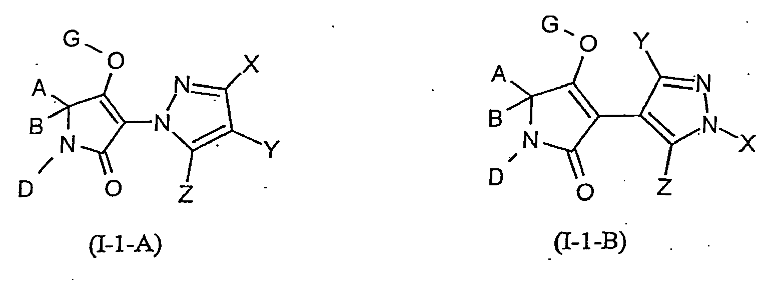

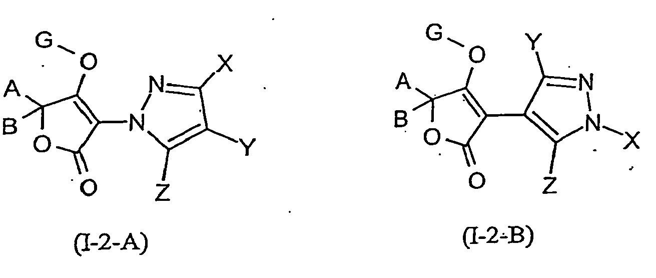

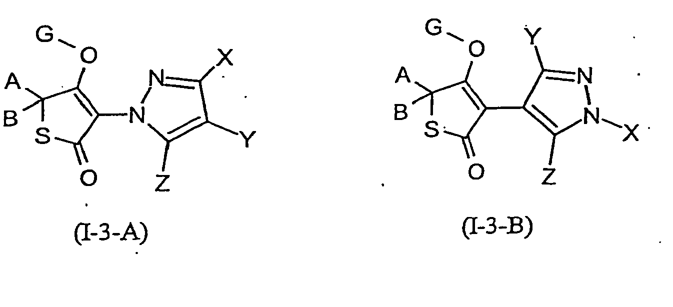

3. Verbindungen der Formel (I) gemäß Anspruch 1, in welcher
X für gegebenenfalls einfach bis zweifach durch Fluor, Chlor, Brom, Methyl, Ethyl, Trifluormethyl, Isopropyl, tert-Butyl, Trifluormethoxy, Methoxy, Ethoxy, Isopropoxy, tert.-Butoxy, Cyano oder Nitro substituiertes Phenyl steht,
Y für Wasserstoff, Methyl oder Ethyl steht,
Z für Methyl, Ethyl, Propyl, Isopropyl, Chlor, Methoxy, Ethoxy, Propoxy, Isopropoxy, Difluormethoxy oder Trifluorethoxy steht,
Het für eine der Gruppen 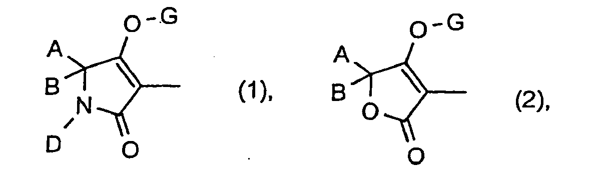 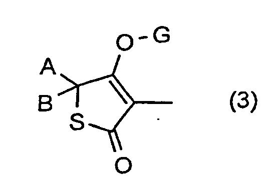 steht,
A für Wasserstoff, für jeweils gegebenenfalls durch Fluor substituiertes C₁-C₈-Alkyl oder C₁-C₆-Alkoxy-C₁-C₄-alkyl, gegebenenfalls durch Fluor, Methyl, Ethyl oder Methoxy substituiertes C₃-C₆-Cycloalkyl, in welchem gegebenenfalls ein Ringglied durch Sauerstoff oder Schwefel ersetzt ist oder für jeweils gegebenenfalls einfach bis zweifach durch Fluor, Chlor, Brom, Methyl, Ethyl, n-Propyl, iso-Propyl, Methoxy, Ethoxy, Trifluormethyl, Trifluormethoxy, Cyano oder Nitro substituiertes Phenyl oder Benzyl steht,
B für Wasserstoff oder C₁-C₆-Alkyl steht oder
A, B und das Kohlenstoffatom an das sie gebunden sind, für gesättigtes C₃-C₆-Cycloalkyl stehen, in welchem gegebenenfalls ein Ringglied durch Sauerstoff oder Schwefel ersetzt ist und welches gegebenenfalls einfach durch Methyl, Ethyl, Propyl, Isopropyl, Butyl, iso-Butyl, sec.-Butyl, tert.-Butyl, Trifluormethyl, Methoxy, Ethoxy, Propoxy, iso-Propoxy, Butoxy, iso-Butoxy, sek.-Butoxy, tert.-Butoxy, Fluor oder Chlor substituiert ist oder
A, B und das Kohlenstoffatom, an das sie gebunden sind, für C₆-Cycloalkyl stehen, welches durch eine gegebenenfalls einfach bis zweifach durch Methyl oder Ethyl substituierte Allcylendioxyl-Gruppe substituiert ist, die mit dem Kohlenstoffatom, an das sie gebunden ist, einen weiteren fünf- oder sechsgliedrigen Ring bildet,
A, B und das Kohlenstoffatom, an das sie gebunden sind, für C₅-C₆-Cycloalkyl oder C₅-C₆-Cycloalkenyl stehen, worin zwei Substituenten gemeinsam mit den Kohlenstoffatomen, an die sie gebunden sind, für C₂-C₄-Alkandiyl oder C₂-C₄-Alkendiyl, worin jeweils gegebenenfalls eine Methylengruppe durch Sauerstoff oder Schwefel ersetzt ist oder Butadiendiyl stehen,
D für Wasserstoff, für jeweils gegebenenfalls durch Fluor substituiertes C₁-C₈-Alkyl, C₃-C₄-Alkenyl, C₁-C₆-Alkoxy-C₂-C₄-alkyl, C₁-C₄-Alkylthio-C₂-C₄-alkyl oder für durch Trifluormethyl oder Fluor substituiertes C₃-C₆-Cycloalkyl, in welchem gegebenenfalls eine Methylengruppe durch Sauerstoff oder Schwefel ersetzt ist oder für jeweils gegebenenfalls einfach bis zweifach durch Fluor, Chlor, Methyl, Ethyl, n-Propyl, iso-Propyl, Methoxy, Ethoxy, Trifluormethyl oder Trifluormethoxy substituiertes Phenyl oder Benzyl steht,
oder
A und D gemeinsam für C₃-C₄-Alkandiyl stehen, worin gegebenenfalls ein Kohlenstoffatom durch Schwefel ersetzt ist und welches gegebenenfalls einfach bis zweifach durch Methyl, Ethyl, Methoxy oder Ethoxy substituiert ist oder
A und D (im Fall der Verbindungen der Formel (I-1)) gemeinsam mit den Atomen, an die sie gebunden sind, für eine der folgenden Gruppen AD: 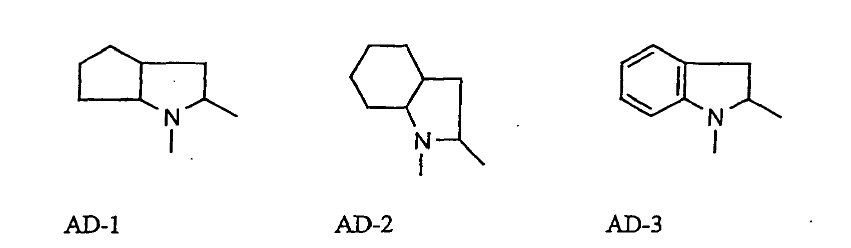 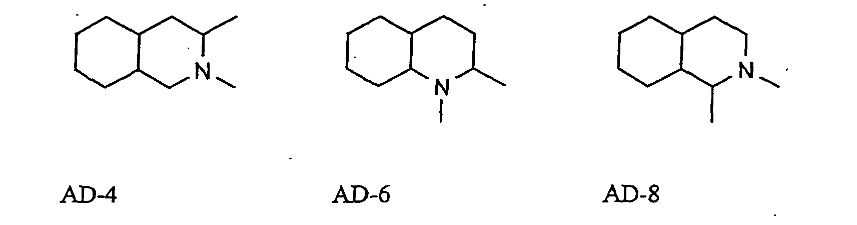 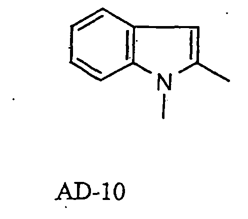 stehen,
G für Wasserstoff (a) oder für eine der Gruppen 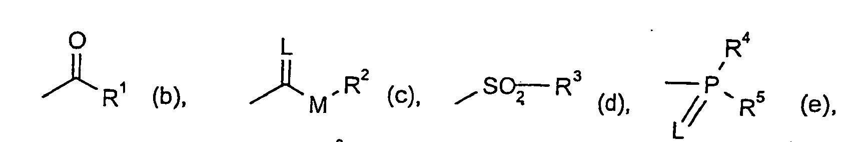 E (f) oder 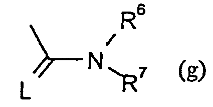 steht,
in welchen
E für ein Metallionäquivalent oder ein Ammoniumion steht,
L für Sauerstoff oder Schwefel steht und
M für Sauerstoff oder Schwefel steht,
R¹ für jeweils gegebenenfalls durch Fluor oder Chlor substituiertes C₁-C₁₄-Alkyl, C₂-C₁₄-Alkenyl, C₁-C₄-Alkoxy-C₁-C₂-alkyl, C₁-C₄-Alkylthio-C₁-C₂-alkyl oder gegebenenfalls einfach bis zweifach durch Fluor, Chlor, Methyl, Ethyl, Propyl, i-Propyl, Butyl, i-Butyl, tert.-Butyl, Methoxy oder Ethoxy, substituiertes C₃-C₆-Cycloalkyl, in welchem gegebenenfalls ein oder zwei nicht direkt benachbarte Ringglieder durch Sauerstoff und/oder Schwefel ersetzt sind,
für gegebenenfalls einfach bis zweifach durch Fluor, Chlor, Brom, Cyano, Nitro, Methyl, Ethyl, n-Propyl, i-Propyl, tert.-Butyl, Methoxy, Ethoxy, i-Propoxy, tert.-Butoxy, Trifluormethyl oder Trifluormethoxy substituiertes Phenyl, für jeweils gegebenenfalls einfach bis zweifach durch Fluor, Chlor, Brom, Methyl oder Ethyl substituiertes Furanyl, Thienyl oder Pyridyl steht,
R² für jeweils gegebenenfalls einfach bis dreifach durch Fluor substituiertes C₁-C₁₄-Alkyl, C₂-C₁₄-Alkenyl oder C₁-C₄-Alkoxy-C₂-C₆-alkyl,
für gegebenenfalls einfach bis zweifach durch Fluor, Chlor, Methyl, Ethyl, n-Propyl, iso-Propyl oder Methoxy substituiertes C₃-C₆-Cycloalkyl,
oder für jeweils gegebenenfalls einfach bis zweifach durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
R³ für jeweils gegebenenfalls einfach bis dreifach durch Fluor substituiertes Methyl, Ethyl, n-Propyl oder gegebenenfalls einfach bis zweifach durch Fluor, Chlor, Brom, Methyl, tert.-Butyl, Methoxy, Trifluormethyl, Trifluormethoxy, Cyano oder Nitro substituiertes Phenyl steht,
R⁴ für C₁-C₄-Alkyl, C₁-C₄-Alkoxy, C₁-C₄-Alkylamino, Di-(C₁-C₄-alkyl)amino, C₁-C₄-Alkylthio oder für jeweils gegebenenfalls einfach bis zweifach durch Fluor, Chlor, Brom, Nitro, Cyano, C₁-C₂-Alkoxy, C₁-C₂-Fluoralkoxy, C₁-C₂-Alkylthio, C₁-C₂-Fluoralkylthio oder C₁-C₃-Alkyl substituiertes Phenyl, Phenoxy oder Phenylthio steht,
R⁵ für Methoxy, Ethoxy, Methylthio oder Ethylthio steht,
R⁶ .für C₁-C₄-Alkyl, C₃-C₆-Cycloalkyl, C₁-C₄-Alkoxy, C₃-C₄-Alkenyl, C₁-C₄-Alkoxy-C₁-C₄-alkyl, für gegebenenfalls einfach bis zweifach durch Fluor, Chlor, Brom, Trifluormethyl, Methyl oder Methoxy substituiertes Phenyl, für gegebenenfalls einfach bis zweifach durch Fluor, Chlor, Brom, Methyl, Trifluormethyl oder Methoxy substituiertes Benzyl steht,
R⁷ für Wasserstoff, Methyl, Ethyl, Propyl oder Allyl steht,
R⁶ und R⁷ zusammen für einen C₅-C₆-Alkylenrest stehen, in welchem gegebenenfalls eine Methylengruppe durch Sauerstoff oder Schwefel ersetzt ist,
wobei die Verknüpfungsposition am Pyrazolylrest festgelegt wird auf folgende Strukturen:

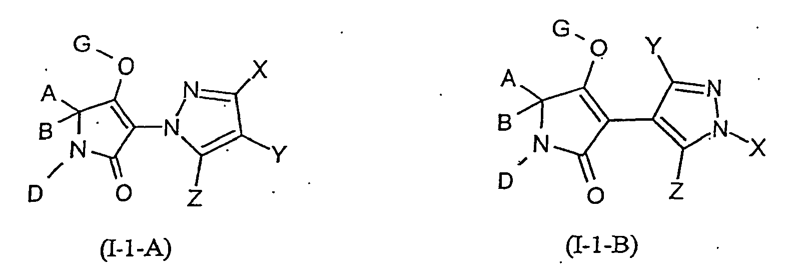

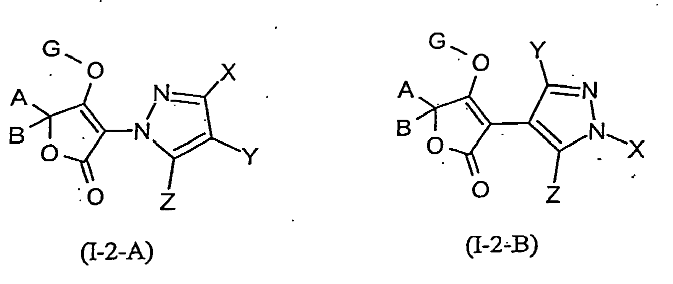

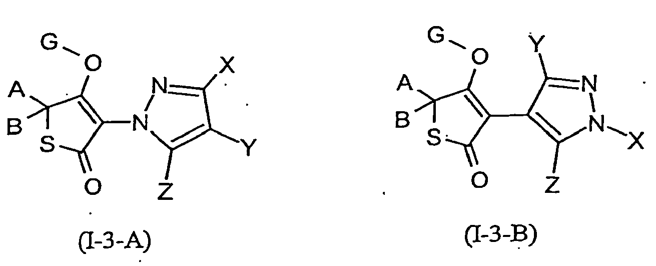

4. Verbindungen der Formel (I) gemäß Anspruch 1, in welcher
X für gegebenenfalls einfach bis zweifach durch Fluor, Chlor, Brom, Methyl, Trifluormethyl, Methoxy oder Trifluormethoxy substituiertes Phenyl steht,
Y für Wasserstoff oder Methyl steht,
Z für Methyl, Ethyl oder Propyl steht,
Het für eine der Gruppen 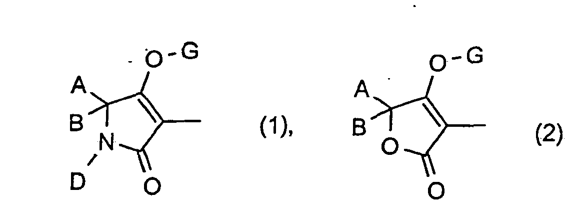 steht,
A für Wasserstoff oder C₁-C₆-Alkyl steht,
B für Wasserstoff oder C₁-C₆-Alkyl steht oder
A, B und das Kohlenstoffatom an das sie gebunden sind, für gesättigtes C₅-C₆-Cycloalkyl stehen, in welchem gegebenenfalls ein Ringglied durch Sauerstoff oder Schwefel ersetzt ist und welches gegebenenfalls einfach durch Methyl, Ethyl, Propyl, Isopropyl, Trifluormethyl, Methoxy, Ethoxy, Propoxy, iso-Propoxy, Butoxy oder iso-Butoxy substituiert ist oder
A, B und das Kohlenstoffatom an das sie gebunden sind für C₅-C₆-Cycloalkyl oder C₅-C₆-Cycloalkenyl stehen, worin zwei Substituenten gemeinsam mit den Kohlenstoffatomen, an die sie gebunden sind, für Butadienyl stehen,
D für Wasserstoff, Methyl, Ethyl oder Isopropyl oder für durch Trifluormethyl substituiertes Cyclohexyl steht,
A und D gemeinsam für C₃-C₄-Alkandiyl stehen,
G für Wasserstoff (a) oder für eine der Gruppen 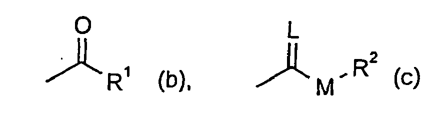 steht,
in welchen
L für Sauerstoff steht und
M für Sauerstoff oder Schwefel steht,
R¹ für jeweils gegebenenfalls einfach bis dreifach durch Fluor oder Chlor substituiertes C₁-C₆-Alkyl, C₂-C₆-Alkenyl, C₁-C₂-Alkoxy-C₁-C₂-alkyl, C₁-C₂-Alkylthio-C₁-C₂-alkyl oder gegebenenfalls einfach durch Fluor, Chlor, Methyl, Ethyl oder Methoxy substituiertes C₃-C₆-Cycloalkyl, in welchem gegebenenfalls ein Ringglied durch Sauerstoff oder Schwefel ersetzt ist,
für gegebenenfalls einfach durch Fluor, Chlor, Brom, Cyano, Nitro, Methyl, Ethyl, n-Propyl, i-Propyl, tert.-Butyl, Methoxy, Ethoxy, i-Propoxy, tert.-Butoxy, Trifluormethyl oder Trifluormethoxy substituiertes Phenyl,
für jeweils gegebenenfalls einfach durch Fluor, Chlor, Brom, Methyl oder Ethyl substituiertes Thienyl oder Pyridyl steht,
R² für jeweils gegebenenfalls einfach bis dreifach durch Fluor substituiertes C₁-C₆-Alkyl, C₂-C₆-Alkenyl oder C₁-C₄-Alkoxy-C₂-C₃-alkyl, für Cyclopentyl oder Cyclohexyl,
oder für jeweils gegebenenfalls einfach durch Fluor, Chlor, Cyano, Nitro, Methyl, Ethyl, n-Propyl, i-Propyl, Methoxy, Ethoxy, Trifluormethyl oder Trifluormethoxy substituiertes Phenyl oder Benzyl steht,
wobei die Verknüpfungsposition am Pyrazolylrest festgelegt wird auf folgende Strukturen:

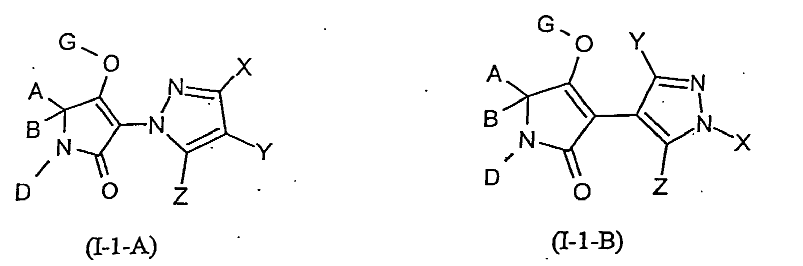

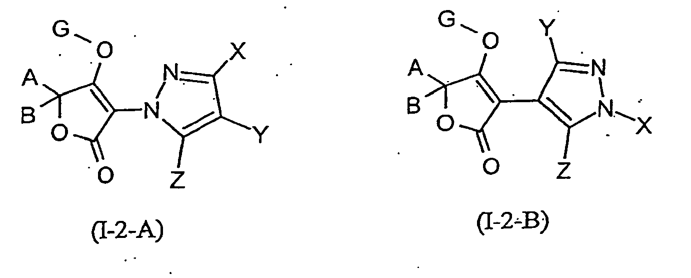

5. Verbindungen der Formel (I) gemäß Anspruch 1, in welcher der Pyrazolrest folgende Verknüpfungsposition hat: 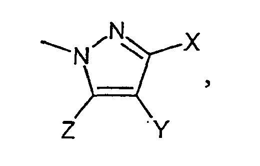 und
X für gegebenenfalls einfach bis zweifach durch Chlor, Brom oder Trifluormethyl substituiertes Phenyl steht,
Y für Wasserstoff steht,
Z für Methyl, Ethyl oder Propyl steht,
Het für eine der Gruppen 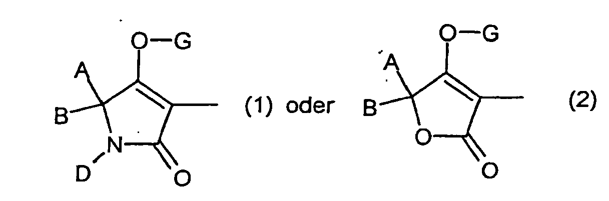 steht,
A für Wasserstoff oder C₁-C₆-Alkyl steht,
B für Wasserstoff oder C₁-C₆-Alkyl steht,
A, B und das Kohlenstoffatom, an das sie gebunden sind, für gesättigtes C₆-Cycloalkyl, in welchem gegebenenfalls ein Ringglied durch Sauerstoff ersetzt ist oder für gesättigtes C₆-Cycloalkyl steht, welches gegebenenfalls einfach durch Methyl, Trifluormethyl, Methoxy oder Ethoxy substituiert ist,
A, B und das Kohlenstoffatom an das sie gebunden sind für C₅-C₆-Cycloalkyl stehen, worin zwei Substituenten gemeinsam mit den Kohlenstoffatomen an die sie gebunden sind für Butadienyl stehen,
D für Wasserstoff oder für einfach durch Trifluormethyl substituiertes Cyclohexyl steht,
A und D für C₃-C₄-Alkandiyl stehen,
G für Wasserstoff(a) oder für eine der Gruppen 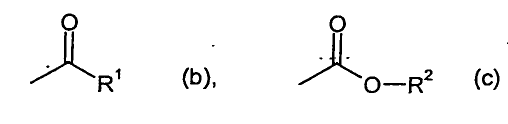 steht,
worin
R¹ für C₁-C₆-Alkyl, C₃-Cycloalkyl, für jeweils durch Chlor substituiertes Phenyl oder Pyridyl steht,
R² für C₁-C₆-Alkyl oder Benzyl steht.

6. Verbindungen der Formel (I) gemäß Anspruch 1, in welcher der Pyrazolrest folgende Verknüpfungsposition hat: 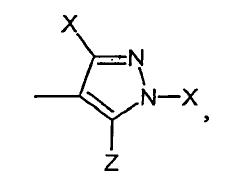 und
X für einfach durch Chlor oder Trifluormethyl substituiertes Phenyl steht,
Y für Methyl steht,
Z für Methyl steht,
Het für die Gruppe 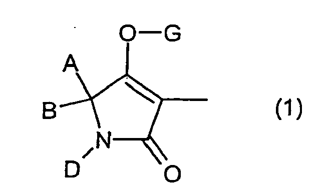 steht
A für Methyl steht,
B für Methyl steht,
A, B und das Kohlenstoffatom, an das sie gebunden sind, für C₆-Cycloalkyl stehen, welches einfach durch Methoxy substituiert ist,
D für Wasserstoff steht,
G für Wasserstoff oder für die Gruppe 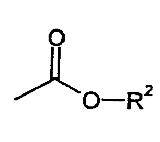 steht,
R² für C₁-C₆-Alkyl steht.

7. Verfahren zur Herstellung von Verbindungen der Formel (I) gemäß Anspruch 1, **dadurch gekennzeichnet, dass** man zum Erhalt von
(A) 3-Pyrazolyl-pyrrolidin-2,4-dione bzw. deren Enole der Formel (I-1-A-a) und (1-1-B-a) 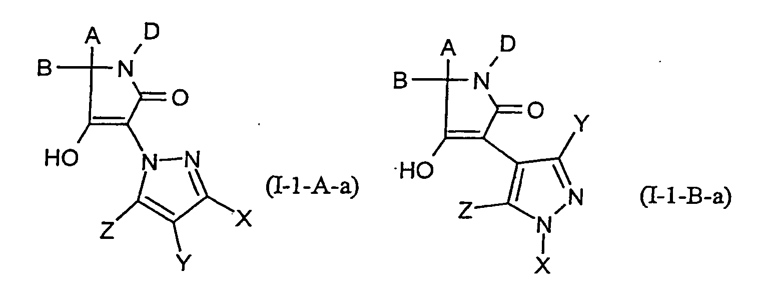 in welcher
A, B, D, X, Y und Z die oben angegebenen Bedeutungen haben,
N-Acylaminosäureester der Formel (II-A) und (II-B) 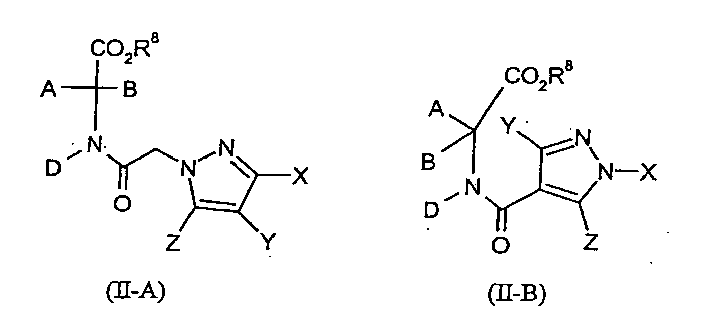 in welcher
A, B, D, X, Y und Z die oben angegebenen Bedeutungen haben, und
R⁸ für Alkyl steht,
in Gegenwart eines Verdünnungsmittels und in Gegenwart einer Base intramolekular kondensiert,
(B) substituierten 3-Pyrazolyl-4-hydroxy-Δ³-dihydrofuranon-Derivaten der Formel (I-2-A-a) und (I-2-B-a) 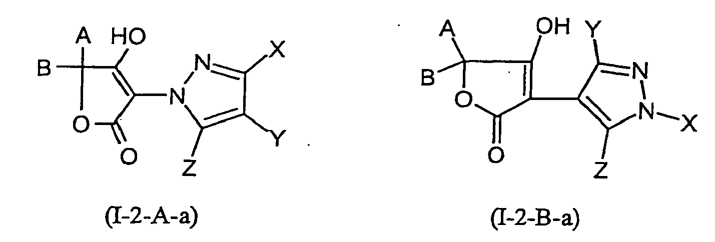 in welcher
A, B, X, Y und Z die oben angegebenen Bedeutungen haben,
Carbonsäureester der Formel (III-A) und (III-B) 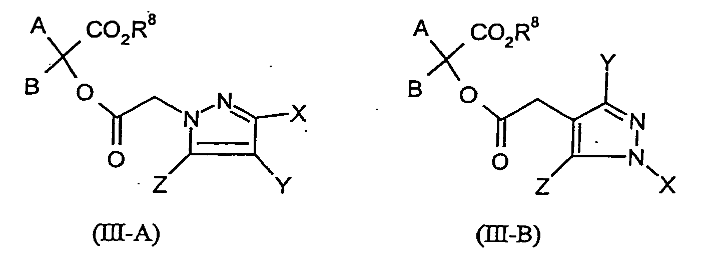 in welcher
A, B, X, Y, Z und R⁸ die oben angegebenen Bedeutungen haben,
in Gegenwart eines Verdünnungsmittels und in Gegenwart einer Base intramolekular kondensiert,
(C) substituierten 3-Thiazolyl-4-hydroxy-Δ³-dihydrothiophen-2-on-Derivaten der Formel (I-3-A-a) und (I-3-B-a) 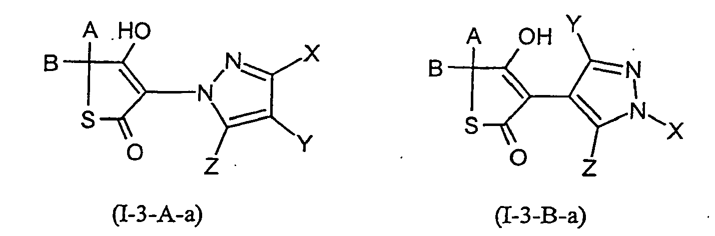 in welcher
A, B, X, Y und Z die oben angegebenen Bedeutungen haben,
β-Ketocarbonsäureester der Formel (IV-A) und (IV-B) 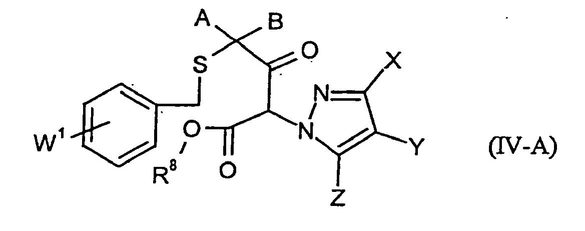 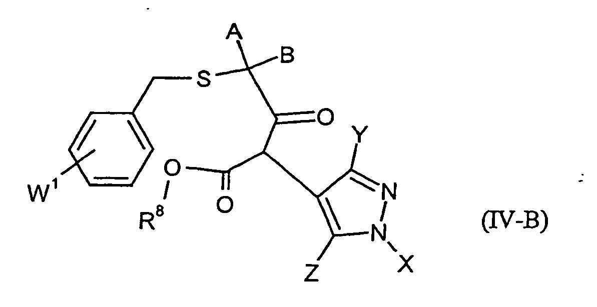 in welcher
A, B, X, Y, Z und R⁸ die oben angegebenen Bedeutungen haben und
W¹ für Wasserstoff, Halogen, Alkyl oder Alkoxy steht,
gegebenenfalls in Gegenwart eines Verdünnungsmittels und in Gegenwart einer Säure intramolekular cyclisiert,
D) Verbindungen der oben gezeigten Formeln (I-1-A-b) bis (I-3-B-b), in welchen A, B, D, R¹, X, Y und Z die oben angegebenen Bedeutungen haben, Verbindungen der oben gezeigten Formeln (I-1-A-a) bis (I-3-B-a), in welchen A, B, D, X, Y und Z die oben angegebenen Bedeutungen haben, jeweils
(α) mit Säurehalogeniden der Formel (V) 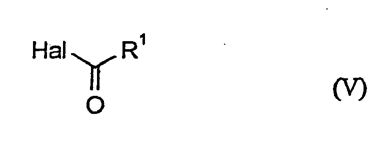 in welcher
R¹ die oben angegebene Bedeutung hat und
Hal für Halogen steht
oder
(β) mit Carbonsäureanhydriden der Formel (VI)
R¹-CO-O-CO-R¹ (VI)
in welcher
R¹ die oben angegebene Bedeutung hat,
gegebenenfalls in Gegenwart eines Verdünnungsmittels und gegebenenfalls in Gegenwart eines Säurebindemittels umsetzt;
(E) Verbindungen der oben gezeigten Formeln (I-1-A-c) bis (I-3-B-c), in welchen A, B, D, R², M, X, Y und Z die oben angegebenen Bedeutungen haben und L für Sauerstoff steht, Verbindungen der oben gezeigten Formeln (I-1-A-a) bis (I-3-B-a), in welchen A, B, D, X, Y und Z die oben angegebenen Bedeutungen haben, jeweils
mit Chlorameisensäureestem oder Chlorameisensäurethioestem der Formel (VII)
R²-M-CO-Cl (VII)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(F) Verbindungen der oben gezeigten Formeln (I-1-A-c) bis (I-3-B-c), in welchen A, B, D, R², M, X, Y und Z die oben angegebenen Bedeutungen haben und L für Schwefel steht, Verbindungen der oben gezeigten Formeln (I-1-A-a) bis (I-3-B-a), in welchen A, B, D, X, Y und Z die oben angegebenen Bedeutungen haben; jeweils mit Chlormonothioameisensäureestern oder Chlordithioameisensäureestem der Formel (VTII) 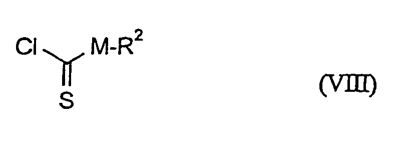 in welcher
M und R² die oben angegebenen Bedeutungen haben,
gegebenenfalls in Gegenwart eines Verdünnungsmittels und gegebenenfalls in Gegenwart eines Säurebindemittels umsetzt,
(G) Verbindungen der oben gezeigten Formeln (I-1-A-d) bis (I-3-B-d), in welchen A, B, D, R³, X, Y und Z die oben angegebenen Bedeutungen haben, Verbindungen der oben gezeigten Formeln (I-I-A-a) bis (I-3-B-a), in welchen A, B, D, X, Y und Z die oben angegebenen Bedeutungen haben, jeweils
mit Sulfionsäurechloäden der Formel (IX)
R³-SO₂-Cl (IX)
in welcher
R³ die oben angegebene Bedeutung hat,
gegebenenfalls in Gegenwart eines Verdünnungsmittels und gegebenenfalls in Gegenwart eines Säurebindemittels umsetzt,
(H) Verbindungen der oben gezeigten Formeln (I-1-A-e) bis (I-3-B-e), in welchen A, B, D, L, R⁴, R⁵, X, Y und Z die oben angegebenen Bedeutungen haben, Verbindungen der oben gezeigten Formeln (I-1-A-a) bis (I-3-B-a), in welchen A, B, D, X, Y und Z die oben angegebenen Bedeutungen haben, jeweils
mit Phosphorverbindungen der Formel (X) 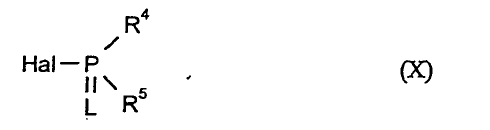 in welcher
L, R⁴ und R⁵ die oben angegebenen Bedeutungen haben und
Hal für Halogen steht,
gegebenenfalls in Gegenwart eines Verdünnungsmittels und gegebenenfalls in Gegenwart eines Säurebindemittels umsetzt,
(I) Verbindungen der oben gezeigten Formeln (I-1-A-f) bis (I-3-B-f), in welchen A, B, D, E, X, Y und Z die oben angegebenen Bedeutungen haben, Verbindungen der oben gezeigten Formeln (I-1-A-a) bis (I-3-B-a), in welchen A, B, D, X, Y und Z die oben angegebenen Bedeutungen haben, jeweils
mit Metallverbindungen oder Aminen der Formeln (XI) oder (XII)
ME(OR¹⁰)ₜ (XI) 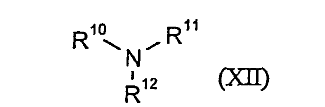
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(J) Verbindungen der oben gezeigten Formeln (I-1-A-g) bis (I-3-B-g), in welchen A, B, D, L, R⁶, R⁷, X, Y und Z die oben angegebenen Bedeutungen haben, Verbindungen der oben gezeigten Formeln (I-1-A-a) bis (I-3-B-a), in welchen A, B, D, X, Y und Z die oben angegebenen Bedeutungen haben, jeweils
(α) mit Isocyanaten oder Isothiocyanaten der Formel (XIII)
R⁶-N=C=L (XIII)
in welcher
R⁶ und L die oben angegebenen Bedeutungen haben,
gegebenenfalls in Gegenwart eines Verdünnungsmittels und gegebenenfalls in Gegenwart eines Katalysators umsetzt oder
(β) mit Carbamidsäurechloriden oder Thiocarbamidsäurechloriden der Formel (XIV) 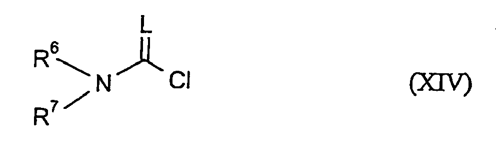
in welcher
L, R⁶ und R⁷ die oben angegebenen Bedeutungen haben,
gegebenenfalls in Gegenwart eines Verdünnungsmittels und gegebenenfalls in Gegenwart eines Säurebindemittels, umsetzt.

8. Verbindungen der Formel (II-A) und (II-B) 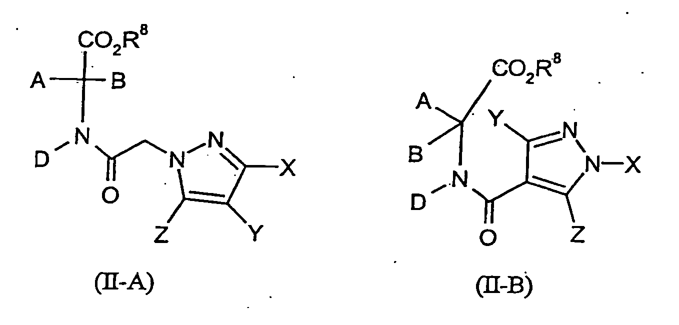 in welcher
A, B, D, X, Y und Z die oben angegebenen Bedeutungen haben und
R⁸ für Alkyl steht.

9. Verbindungen der Formel (III-A) und (III-B) 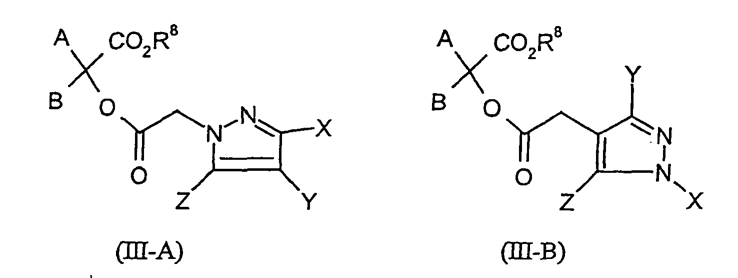 in welcher
A, B, X, Y, Z und R⁸ die oben angegebenen Bedeutungen haben.

10. Verbindungen der Formel (IV-A) und (IV-B) 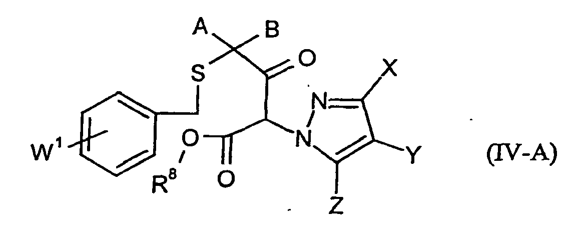 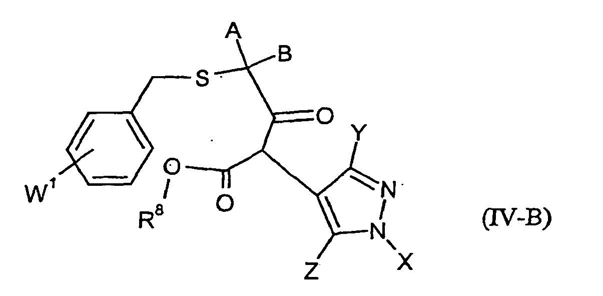 in welcher
A, B, X, Y, Z und R⁸ die oben angegebenen Bedeutungen haben und
W¹ für Wasserstoff, Halogen, Alkyl oder Alkoxy
steht.

11. Verbindungen der Formel (XIX-A) und (XIX-B) 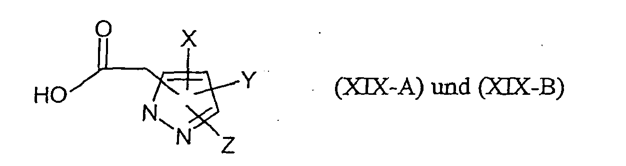 in welcher
X, Y und Z die oben angegebenen Bedeutungen haben, wobei im Fall der Verbindungen der Formel (XIX-B) Y nur für Wasserstoff steht und ausgenommen die Verbindung:

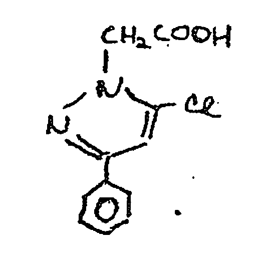

12. Schädlingsbekämpfungsmittel, Mikrobizide und Herbizide, **gekennzeichnet durch** einen Gehalt an mindestens einer Verbindung der Formel (I) gemäß Anspruch 1.

13. Verfahren zur Bekämpfung von tierischen Schädlingen, unerwünschten Pflanzenbewuchs und Pilzen, **dadurch gekennzeichnet; dass** man Verbindungen der Formel (I) gemäß Anspruch 1 auf Schädlinge, unerwünschte Pflanzen, Pilze und/oder ihren Lebensraum einwirken läßt.

14. Verwendung von Verbindungen der Formel (I) gemäß Anspruch 1 zur Bekämpfung von tierischen Schädlingen, unerwünschtem Pflanzenbewuchs und Pilzen.

15. Verfahren zur Herstellung von Schädlingsbekämpfungsmitteln, Mikrobiziden und Herbiziden, **dadurch gekennzeichnet, dass** man Verbindungen der Formel (I) gemäß Anspruch 1 mit Streckmitteln und/oder oberflächenaktiven Stoffen vermischt.

16. Verwendung von Verbindungen der Formel (I) gemäß Anspruch 1 zur Herstellung von Schädlingsbekämpfungsmitteln, Mirkobiziden und Herbiziden.

## Claims

1. Compounds of the formula (I) 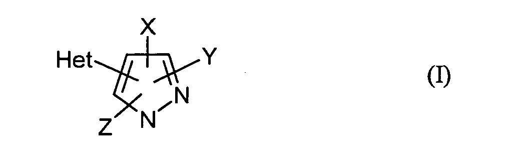 in which
X represents in each case optionally halogen-, C₁-C₆-alkyl-, C₁-C₆-alkoxy-, C₁-C₄-haloalkyl-, C₁-C₄-haloalkoxy-, nitro- or cyano-substituted phenyl,
Y represents hydrogen, halogen or C₁-C₆-alkyl,
Z represents C₁-C₆-alkyl, halogen, hydroxyl, C₁-C₆-alkoxy, C₁-C₆-haloalkoxy or in each case optionally C₁-C₆-alkyl-, C₁-C₆-alkoxy-, halogen-, C₁-C₄-haloalkyl-, C₁-C₆-haloalkoxy-, cyano- or nitro-substituted phenyl-C₁-C₂-alkoxy or optionally C₁-C₂-alkyl- or halogen-substituted C₃-C₆-cycloalkyl,
Het represents one of the groups 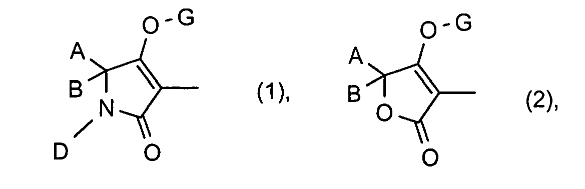 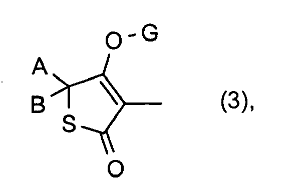
A represents hydrogen or in each case optionally fluorine- or chlorine-substituted C₁-C₁₂-alkyl, C₃-C₈-alkenyl, C₁-C₁₀-alkoxy-C₁-C₈-alkyl, poly-C₁-C₈-alkoxy-C₁-C₈-alkyl, C₁-C₁₀-alkylthio-C₁-C₆-alkyl, optionally fluorine-, chlorine-, C₁-C₆-alkyl- or C₁-C₆-alkoxy-substituted C₃-C₈-cycloalkyl in which optionally one or two not directly adjacent ring members are replaced by oxygen and/or sulphur or represents in each case optionally halogen-, C₁-C₆-alkyl-, C₁-C₆-haloalkyl-, C₁-C₆-alkoxy-, C₁-C₆-haloalkoxy-, cyano- or nitro-substituted C₆- or C₁₀-aryl, or C₆- or C₁₀-aryl-C₁-C₆-alkyl,
B represents hydrogen, C₁-C₁₂-alkyl or C₁-C₈-alkoxy-C₁-C₆-alkyl, or
A, B and the carbon atom to which they are attached represent saturated C₃-C₁₀-cycloalkyl or unsaturated C₅-C₁₀-cycloalkyl in which optionally one ring member is replaced by oxygen or sulphur and which are optionally mono- or disubstituted by C₁-C₈-alkyl, C₃-C₁₀-cycloalkyl, C₁-C₈-haloalkyl, C₁-C₈-alkoxy, C₁-C₈-alkylthio, halogen or phenyl, or
A, B and the carbon atom to which they are attached represent C₅-C₆-cycloalkyl which is substituted by an alkylenediyl group which optionally contains one or two not directly adjacent oxygen and/or sulphur atoms or by an alkylenedioxyl or an alkylenedithioyl group which, together with the carbon atom to which it is attached, forms a further five- to eight-membered ring which may optionally be substituted by C₁-C₄-alkyl, or
A, B and the carbon atom to which they are attached represent C₃-C₈-cycloalkyl or C₅-C₈-cycloalkenyl in which two substituents together with the carbon atoms to which they are attached represent in each case optionally C₁-C₆-alkyl-, C₁-C₆-alkoxy- or halogen-substituted C₂-C₆-alkanediyl, C₂-C₆-alkenediyl or C₄-C₆-alkanedienediyl in which optionally one methylene group is replaced by oxygen or sulphur,
D represents hydrogen, in each case optionally halogen-substituted C₁-C₁₂-alkyl, C₃-C₈-alkenyl, C₃-C₈-alkynyl, C₁-C₁₀-alkoxy-C₂-C₈-alkyl, poly-C₁-C₈-alkoxy-C₂-C₈-alkyl, C₁-C₁₀-alkylthio-C₂-C₈-alkyl, optionally halogen-, C₁-C₄-alkyl-, C₁-C₄-alkoxy- or C₁-C₄-haloalkyl-substituted C₃-C₈-cycloalkyl in which optionally one ring member is replaced by oxygen or sulphur, or represents in each case optionally halogen-, C₁-C₆-alkyl-, C₁-C₆-haloalkyl-, C₁-C₆-alkoxy-, C₁-C₆-haloalkoxy-, cyano- or nitro-substituted phenyl, phenyl-C₁-C₆-alkyl, or
A and D together represent in each case optionally substituted C₃-C₆-alkanediyl or C₃-C₆-alkenediyl in which optionally one methylene group is replaced by a carbonyl group, oxygen or sulphur, possible substituents being in each case:
halogen, hydroxyl, mercapto or in each case optionally halogensubstituted C₁-C₁₀-alkyl, C₁-C₆-alkoxy, C₁-C₆-alkylthio, C₃-C₇-cycloalkyl, phenyl or benzyloxy, or a further C₃-C₆-alkanediyl grouping, C₃-C₆-alkenediyl grouping or a butadienyl grouping which is optionally substituted by C₁-C₆-alkyl or in which optionally two adjacent substituents together with the carbon atoms to which they are attached form a further saturated or unsaturated cycle having 5 or 6 ring atoms (in the case of the compounds of the formula (I-1), A and D together with the atoms to which the are attached then represent, for example, the groups AD-1 to AD-10 mentioned further below), which cycle may contain oxygen or sulphur,
G represents hydrogen (a) or represents one of the groups 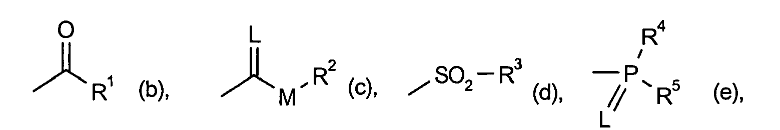 E (f) or 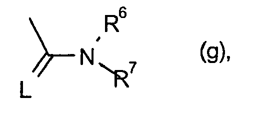 in which
E represents a metal ion equivalent or an ammonium ion,
L represents oxygen or sulphur and
M represents oxygen or sulphur,
R¹ represents in each case optionally halogen-substituted C₁-C₂₀-alkyl, C₂-C₂₀-alkenyl, C₁-C₈-alkoxy-C₁-C₈-alkyl, C₁-C₈-alkylthio-C₁-C₈-alkyl, poly-C₁-C₈-alkoxy-C₁-C₈-alkyl or optionally halogen-, C₁-C₆-alkyl- or C₁-C₆-alkoxy-substituted C₃-C₈-cycloalkyl in which optionally one or more not directly adjacent ring members are replaced by oxygen and/or sulphur,
represents optionally halogen-, cyano-, nitro-, C₁-C₆-alkyl-, C₁-C₆-alkoxy-, C₁-C₆-haloalkyl-, C₁-C₆-haloalkoxy-, C₁-C₆-alkylthio- or C₁-C₆-alkylsulphonyl-substituted phenyl,
represents optionally halogen-, nitro-, cyano-, C₁-C₆-alkyl-, C₁-C₆-alkoxy-, C₁-C₆-haloalkyl- or C₁-C₆-haloalkoxy-substituted phenyl-C₁-C₆-alkyl,
represents optionally halogen-, C₁-C₆-alkyl-, C₁-C₂-haloalkyl- or C₁-C₄-alkoxy-substituted 5- or 6-membered hetaryl,
represents optionally halogen- or C₁-C₆-alkyl-substituted phenoxy-C₁-C₆-alkyl or
represents optionally halogen-, amino- or C₁-C₆-alkyl-substituted 5-or 6-membered hetaryloxy-C₁-C₆-alkyl,
R² represents in each case optionally halogen-substituted C₁-C₂₀-alkyl, C₂-C₂₀-alkenyl, C₁-C₈-alkoxy-C₂-C₈-alkyl, poly-C₁-C₈-alkoxy-C₂-C₈-alkyl,
represents optionally halogen-, C₁-C₆-alkyl- or C₁-C₆-alkoxy-substituted C₃-C₈-cycloalkyl in which optionally one ring atom is replaced by oxygen, or
represents in each case optionally halogen-, cyano-, nitro-, C₁-C₆-alkyl-, C₁-C₆-alkoxy-, C₁-C₆-haloalkyl- or C₁-C₆-haloalkoxy-substituted phenyl or benzyl,
R³ represents optionally halogen-substituted C₁-C₈-alkyl or represents in each case optionally halogen-, C₁-C₆-alkyl-, C₁-C₆-alkoxy-, C₁-C₄-haloalkyl-, C₁-C₄-haloalkoxy-, cyano- or nitro-substituted phenyl or benzyl,
R⁴ and R⁵ independently of one another represent in each case optionally halogen-substituted C₁-C₈-alkyl, C₁-C₈-alkoxy, C₁-C₈-alkylamino, di-(C₁-C₈-alkyl)amino, C₁-C₈-alkylthio, C₂-C₈-alkenylthio, C₃-C₇-cyaoalkylthio or represent in each case optionally halogen-, nitro-, cyano-, C₁-C₄-alkoxy-, C₁-C₄-haloalkoxy-, C₁-C₄-alkylthio-, C₁-C₄-haloalkylthio-, C₁-C₄-alkyl- or C₁-C₄-haloalkyl-substituted phenyl, phenoxy or phenylthio,
R⁶ and R⁷ independently of one another represent hydrogen, represent in each case optionally halogen-substituted C₁-C₈-alkyl, C₃-C₈-cycloalkyl, C₁-C₈-alkoxy, C₃-C₈-alkenyl, C₁-C₈-alkoxy-C₁-C₈-alkyl, represent optionally halogen-, C₁-C₈-haloalkyl-, C₁-C₈-alkyl-or C₁-C₈-alkoxy-substituted phenyl, optionally halogen-, C₁-C₈-alkyl-, C₁-C₈-haloalkyl- or C₁-C₈-alkoxy-substituted benzyl or together represent an optionally C₁-C₄-alkyl-substituted C₃-C₆-alkylene radical in which optionally one carbon atom is replaced by oxygen or sulphur,
the point of attachment on the pyrazolyl radical being established on the following structures:

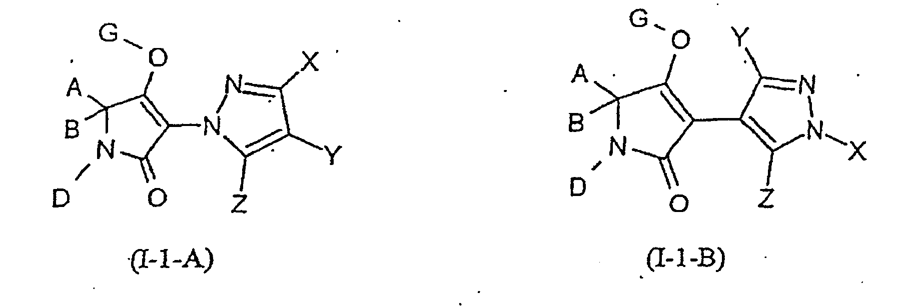

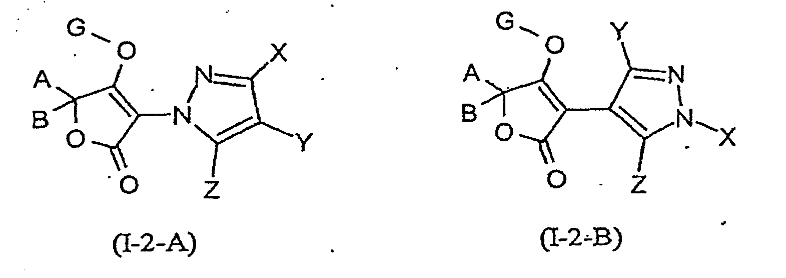

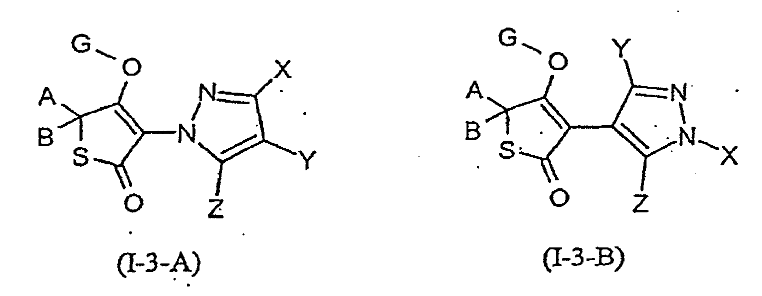

2. Compounds of the formula (I) according to Claim 1 in which
X represents phenyl which is optionally mono- to trisubstituted by fluorine, chlorine, bromine, C₁-C₄-alkyl, C₁-C₄-alkoxy, C₁-C₂-haloalkyl, C₁-C₂-haloalkoxy, nitro or cyano,
Y represents hydrogen, chlorine, bromine or C₁-C₄-alkyl,
Z represents C₁-C₄-alkyl, chlorine, bromine, C₁-C₄-alkoxy, C₁-C₄-haloalkoxy or represents benzyloxy which is optionally mono- or disubstituted by C₁-C₄-alkyl, C₁-C₄-alkoxy, fluorine, chlorine, bromine, C₁-C₂-haloalkyl, C₁-C₂-haloalkoxy, cyano or nitro,
Het represents one of the groups 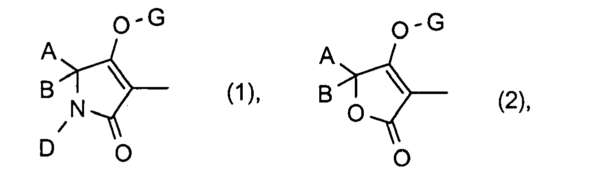 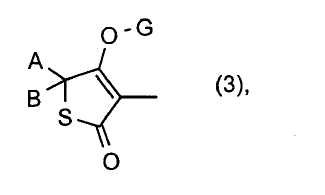
A represents hydrogen, in each case optionally fluorine-substituted C₁-C₁₀-alkyl, C₁-C₈-alkoxy-C₁-C₆-alkyl, optionally fluorine-, chlorine-, C₁-C₄-alkyl- or C₁-C₄-alkoxy-substituted C₃-C₇-cycloalkyl in which optionally one ring member is replaced by oxygen or sulphur or represents phenyl or phenyl-C₁-C₂-alkyl, each of which is optionally substituted by fluorine, chlorine, bromine, C₁-C₄-alkyl, C₁-C₄-haloalkyl, C₁-C₄-alkoxy, cyano, nitro or C₁-C₄-haloalkoxy,
B represents hydrogen or C₁-C₁₀-alkyl or
A, B and the carbon atom to which they are attached represent saturated C₃-C₇-cycloalkyl or unsaturated C₅-C₇-cycloalkyl in which optionally one ring member is replaced by oxygen or sulphur and which is optionally mono- or disubstituted by C₁-C₆-alkyl, C₅-C₈-cycloalkyl, C₁-C₃-haloalkyl, C₁-C₆-alkoxy, fluorine, chlorine or phenyl, or
A, B and the carbon atom to which they are attached represent C₅-C₆-cycloalkyl which is substituted by an alkylenediyl group which optionally contains one or two not directly adjacent oxygen or sulphur atoms or by an alkylenedioxyl or by an alkylenedithiolyl group which, together with the carbon atom to which it is attached, forms a further five- or six-membered ring which may optionally be substituted by C₁-C₃-alkyl, or
A, B and the carbon atom to which they are attached represent C₅-C₆-cycloalkyl or C₅-C₆-cycloalkenyl in which two substituents together with the carbon atoms to which they are attached represent C₂-C₄-alkanediyl, C₂-C₄-alkenediyl in which optionally one methylene group is replaced by oxygen or sulphur, each of which radicals is optionally substituted by C₁-C₅-alkyl, C₁-C₅-alkoxy, fluorine, chlorine or bromine, or represent butadienediyl,
D represents hydrogen, represents in each case optionally fluorine-substituted C₁-C₁₀-alkyl, C₃-C₆-alkenyl, C₁-C₆-alkoxy-C₂-C₄-alkyl or C₁-C₆-alkylthio-C₂-C₄-alkyl, represents optionally fluorine-, C₁-C₄-alkyl-, C₁-C₄-alkoxy- or C₁-C₂-haloalkyl-substituted C₃-C₇-cycloalkyl in which optionally one methylene group is replaced by oxygen or sulphur or represents phenyl or phenyl-C₁-C₄-alkyl, each of which is optionally substituted by fluorine, chlorine, bromine, C₁-C₄-alkyl, C₁-C₄-haloalkyl, C₁-C₄-akoxy or C₁-C₄-haloalkoxy, or
A and D together represent C₃-C₅-alkanediyl in which optionally one methylene group may be replaced by oxygen or sulphur, possible substituents being C₁-C₆-alkyl and C₁-C₄-alkoxy, or
A and D represent (in the case of compounds of the formula (I-1)), together with the atoms to which they are attached, one of the groups AD-1 to AD-10: 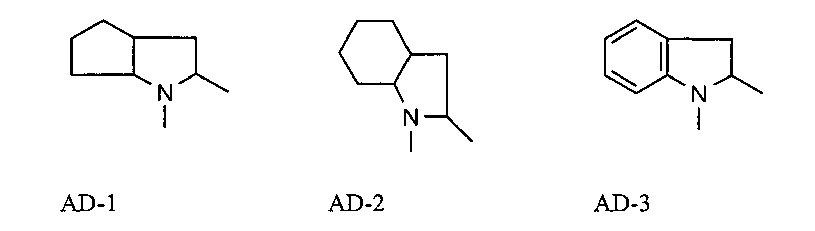 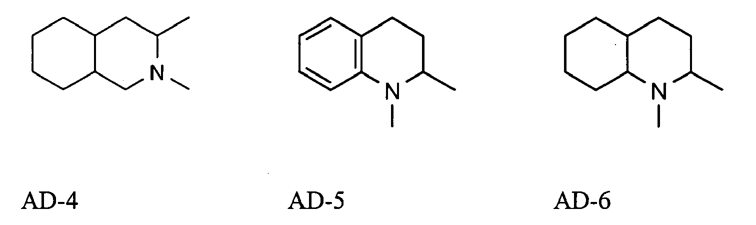 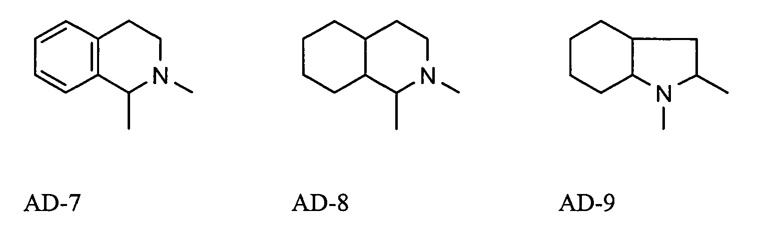 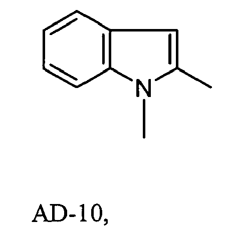
G represents hydrogen (a) or represents one of the groups 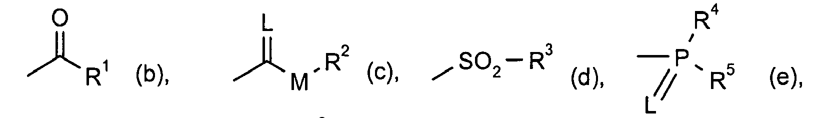 E (f) or 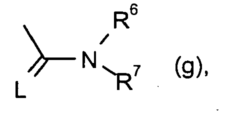 in which
E represents a metal ion equivalent or an ammonium ion,
L represents oxygen or sulphur and
M represents oxygen or sulphur,
R¹ represents in each case optionally fluorine- or chlorine-substituted C₁-C₁₆-alkyl, C₂-C₁₆-alkenyl, C₁-C₆-alkoxy-C₁-C₄-alkyl, C₁-C₆-alkylthio-C₁-C₄-alkyl or optionally fluorine-, chlorine-, C₁-C₅-alkyl- or C₁-C₅-alkoxy-substituted C₃-C₇-cycloalkyl in which optionally one or two not directly adjacent ring members are replaced by oxygen and/or sulphur,
represents optionally fluorine-, chlorine-, bromine-, cyano-, nitro-, C₁-C₄-alkyl-, C₁-C₄-alkoxy-, C₁-C₃-haloalkyl-, C₁-C₃-haloalkoxy-, C₁-C₄-alkylthio- or C₁-C₄-alkylsulphonyl-substituted phenyl,
represents optionally fluorine-, chlorine-, bromine-, C₁-C₄-alkyl-, C₁-C₄-alkoxy-, C₁-C₃-haloalkyl- or C₁-C₃-haloalkoxy-substituted phenyl-C₁-C₄-alkyl, represents in each case optionally fluorine-, chlorine-, bromine-, C₁-C₄-alkyl-, trifluoromethyl- or C₁-C₂-alkoxy-substituted pyrazolyl, thiazolyl, pyridyl, pyrimidyl, furanyl or thienyl,
R² represents in each case optionally fluorine-substituted C₁-C₁₆-alkyl, C₂-C₁₆-alkenyl or C₁-C₆-alkoxy-C₂-C₆-alkyl,
represents optionally fluorine-, chlorine-, C₁-C₄-alkyl- or C₁-C₄-alkoxy-substituted C₃-C₇-cycloalkyl or
represents in each case optionally fluorine-, chlorine-, bromine-, cyano-, nitro-, C₁-C₄-alkyl-, C₁-C₃-alkoxy-, C₁-C₂-haloalkyl- or C₁-C₂-haloalkoxy-substituted phenyl or benzyl,
R³ represents optionally fluorine-substituted C₁-C₆-alkyl or represents in each case optionally fluorine-, chlorine-, bromine-, C₁-C₄-alkyl-, C₁-C₄-alkoxy-, C₁-C₃-haloalkyl-, C₁-C₃-haloalkoxy-, cyano- or nitro-substituted phenyl,
R⁴ represents C₁-C₆-alkyl, C₁-C₆-alkoxy, C₁-C₆-alkylamino, di-(C₁-C₆-alkyl)amino, C₁-C₆-alkylthio, C₃-C₄-alkenylthio, C₃-C₆-cycloalkylthio or represents in each case optionally fluorine-, chlorine-, bromine-, nitro-, cyano-, C₁-C₃-alkoxy-, C₁-C₃-haloalkoxy-, C₁-C₃-alkylthio-, C₁-C₃-haloalkylthio-, C₁-C₃-alkyl-or C₁-C₃-haloalkyl-substituted phenyl, phenoxy or phenylthio,
R⁵ represents C₁-C₆-alkoxy or C₁-C₆-alkylthio,
R⁶ represents C₁-C₆-alkyl, C₃-C₆-cycloalkyl, C₁-C₆-alkoxy, C₃-C₆-alkenyl, C₁-C₆-alkoxy-C₁-C₆-alkyl, represents optionally fluorine-, chlorine-, bromine-, C₁-C₃-haloalkyl-, C₁-C₄-alkyl- or C₁-C₄-alkoxy-substituted phenyl, represents optionally fluorine-, chlorine-, bromine-, C₁-C₄-alkyl-, C₁-C₃-haloalkyl- or C₁-C₄-alkoxy-substituted benzyl,
R⁷ represents hydrogen, C₁-C₆-alkyl, C₃-C₆-alkenyl,
R⁶ and R⁷ together represent an optionally methyl- or ethyl-substituted C₄-C₅-alkylene radical in which optionally one methylene group is replaced by oxygen or sulphur,
the point of attachment on the pyrazolyl radical being established on the following structures:

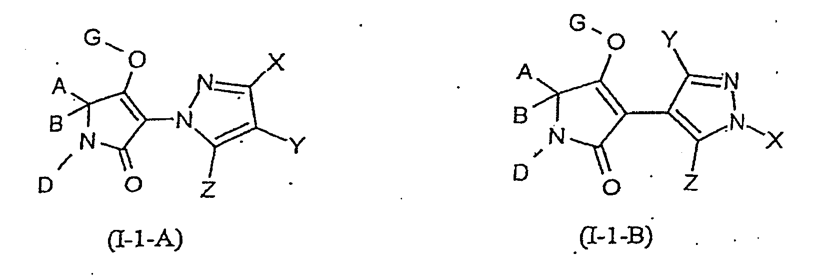

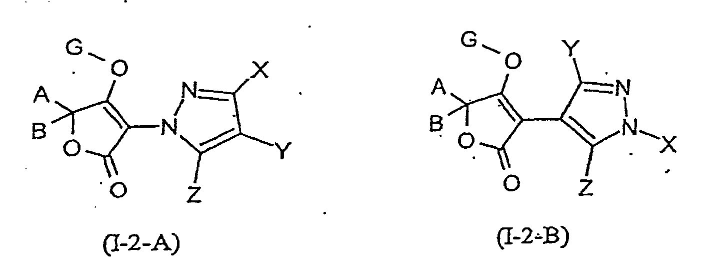

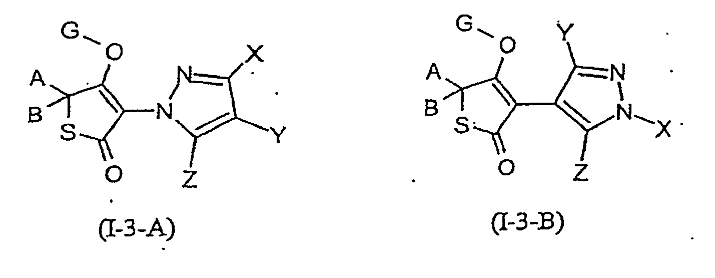

3. Compounds of the formula (I) according to Claim 1 in which
X represents phenyl which is optionally mono- or disubstituted by fluorine, chlorine, bromine, methyl, ethyl, trifluoromethyl, isopropyl, tert-butyl, trifluoromethoxy, methoxy, ethoxy, isopropoxy, tert-butoxy, cyano or nitro,
Y represents hydrogen, methyl or ethyl,
Z represents methyl, ethyl, propyl, isopropyl, chlorine, methoxy, ethoxy, propoxy, isopropoxy, difluoromethoxy or trifluoroethoxy,
Het represents one of the groups 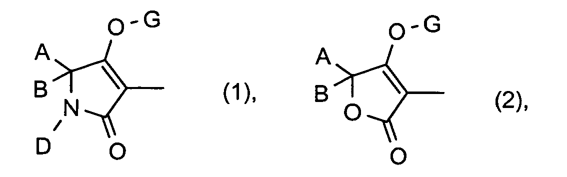 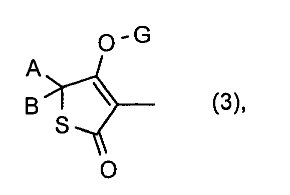
A represents hydrogen, represents in each case optionally fluorine-substituted C₁-C₈-alkyl or C₁-C₆-alkoxy-C₁-C₄-alkyl, optionally fluorine-, methyl-, ethyl- or methoxy-substituted C₃-C₆-cycloalkyl in which optionally one ring member is replaced by oxygen or sulphur, or represents phenyl or benzyl, each of which is optionally mono- or disubstituted by fluorine, chlorine, bromine, methyl, ethyl, n-propyl, isopropyl, methoxy, ethoxy, trifluoromethyl, trifluoromethoxy, cyano or nitro,
B represents hydrogen or C₁-C₆-alkyl or
A, B and the carbon atom to which they are attached represent saturated C₃-C₆-cycloalkyl in which optionally one ring member is replaced by oxygen or sulphur and which is optionally monosubstituted by methyl, ethyl, propyl, isopropyl, butyl, isobutyl, sec-butyl, tert-butyl, trifluoromethyl, methoxy, ethoxy, propoxy, isopropoxy, butoxy, isobutoxy, sec-butoxy, tert-butoxy, fluorine or chlorine, or
A, B and the carbon atom to which they are attached represent C₆-cycloalkyl which is substituted by an alkylenedioxyl group which is optionally mono- or disubstituted by methyl or ethyl and which, together with the carbon atom to which it is attached, forms a further five- or six-membered ring, or
A, B and the carbon atom to which they are attached represent C₅-C₆-cycloalkyl or C₅-C₆-cycloalkenyl in which two substituents together with the carbon atoms to which they are attached represent C₂-C₄-alkanediyl or C₂-C₄-alkenediyl in which in each case optionally one methylene group is replaced by oxygen or sulphur, or represent butadienediyl,
D represents hydrogen, represents in each case optionally fluorine-substituted C₁-C₈-alkyl, C₃-C₄-alkenyl, C₁-C₆-alkoxy-C₂-C₄-alkyl, C₁-C₄-alkylthio-C₂-C₄-alkyl or represents trifluoromethyl- or fluorine-substituted C₃-C₆-cycloalkyl in which optionally one methylene group is replaced by oxygen or sulphur or represents phenyl or benzyl, each of which is optionally mono- or disubstituted by fluorine, chlorine, methyl, ethyl, n-propyl, isopropyl, methoxy, ethoxy, trifluoromethyl or trifluoromethoxy,
or
A and D together represent C₃-C₄-alkanediyl in which optionally one carbon atom is replaced by sulphur and which is optionally mono- or disubstituted by methyl, ethyl, methoxy or ethoxy, or
A and D (in the case of the compounds of the formula (I-1)) together with the atoms to which they are attached represent one of the groups AD below: 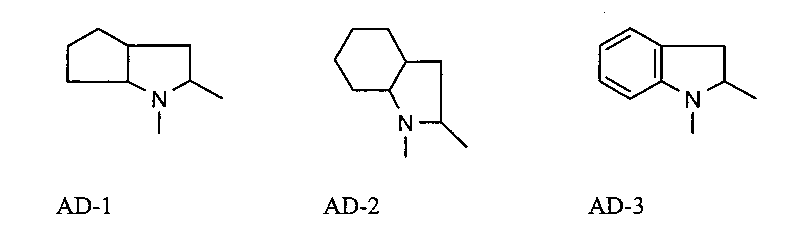 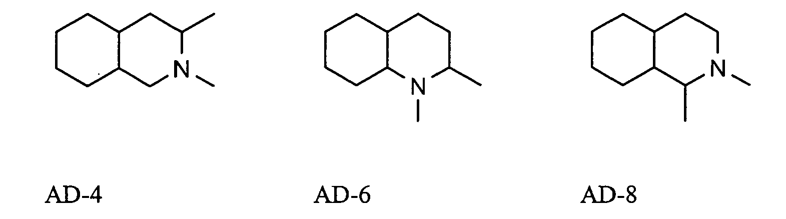 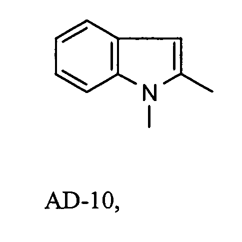
G represents hydrogen (a) or represents one of the groups 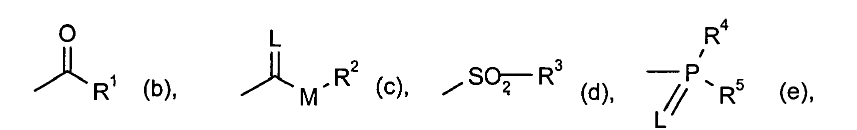 E (f) or 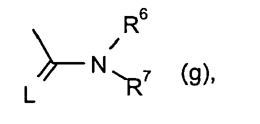 in which
E represents a metal ion equivalent or an ammonium ion,
L represents oxygen or sulphur and
M represents oxygen or sulphur,
R¹ represents in each case optionally fluorine- or chlorine-substituted C₁-C₁₄-alkyl, C₂-C₁₄-alkenyl, C₁-C₄-alkoxy-C₁-C₂-alkyl, C₁-C₄-alkylthio-C₁-C₂-alkyl or represents C₃-C₆-cycloalkyl which is optionally mono- or disubstituted by fluorine, chlorine, methyl, ethyl, propyl, isopropyl, butyl, isobutyl, tert-butyl, methoxy or ethoxy and in which optionally one or two not directly adjacent ring members are replaced by oxygen and/or sulphur,
represents phenyl which is optionally mono- or disubstituted by fluorine, chlorine, bromine, cyano, nitro, methyl, ethyl, n-propyl, i-propyl, tert-butyl, methoxy, ethoxy, i-propoxy, tert-butoxy, trifluoromethyl or trifluoromethoxy,
represents furanyl, thienyl or pyridyl, each of which is optionally mono- or disubstituted by fluorine, chlorine, bromine, methyl or ethyl,
R² represents C₁-C₁₄-alkyl, C₂-C₁₄-alkenyl or C₁-C₄-alkoxy-C₂-C₆-alkyl, each of which is optionally mono- to trisubstituted by fluorine,
represents C₃-C₆-cycloalkyl which is optionally mono- or disubstituted by fluorine, chlorine, methyl, ethyl, n-propyl, isopropyl or methoxy,
or represents phenyl or benzyl, each of which is optionally mono- or disubstituted by fluorine, chlorine, cyano, nitro, methyl, ethyl, n-propyl, i-propyl, methoxy, ethoxy, trifluoromethyl or trifluoromethoxy,
R³ represents methyl, ethyl, n-propyl, each of which is optionally mono- to trisubstituted by fluorine or represents phenyl which is optionally mono- or disubstituted by fluorine, chlorine, bromine, methyl, tert-butyl, methoxy, trifluoromethyl, trifluoromethoxy, cyano or nitro,
R⁴ represents C₁-C₄-alkyl, C₁-C₄-alkoxy, C₁-C₄-alkylamino, di-(C₁-C₄-alkyl)amino, C₁-C₄-alkylthio or represents phenyl, phenoxy or phenylthio, each of which is optionally mono- or disubstituted by fluorine, chlorine, bromine, nitro, cyano, C₁-C₂-alkoxy, C₁-C₂-fluoroalkoxy, C₁-C₂-alkylthio, C₁-C₂-fluoroalkylthio or C₁-C₃-alkyl,
R⁵ represents methoxy, ethoxy, methylthio or ethylthio,
R⁶ represents C₁-C₄-alkyl, C₃-C₆-cycloalkyl, C₁-C₄-alkoxy, C₃-C₄-alkenyl, C₁-C₄-alkoxy-C₁-C₄-alkyl, represents phenyl which is optionally mono- or disubstituted by fluorine, chlorine, bromine, trifluoromethyl, methyl or methoxy, represents benzyl which is optionally mono- or disubstituted by fluorine, chlorine, bromine, methyl, trifluoromethyl or methoxy,
R⁷ represents hydrogen, methyl, ethyl, propyl or allyl,
R⁶ and R⁷ together represent a C₅-C₆-alkylene radical in which optionally one methylene group is replaced by oxygen or sulphur,
the point of attachment on the pyrazolyl radical being established on the following structures:

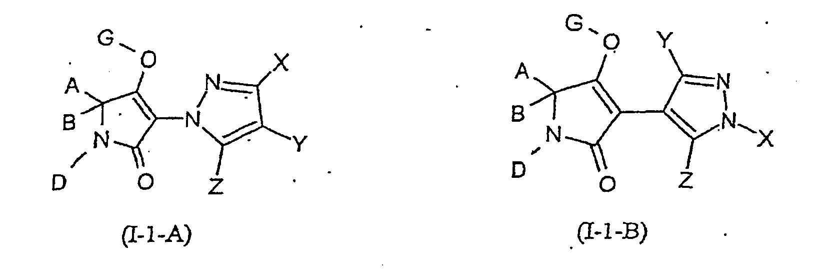

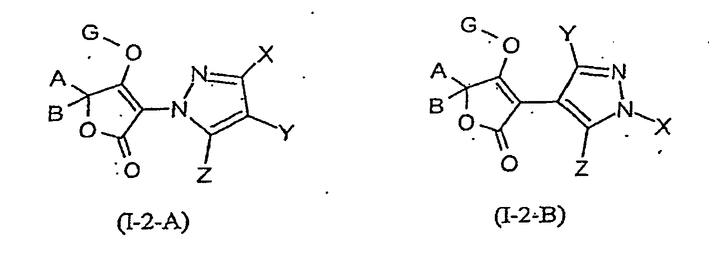

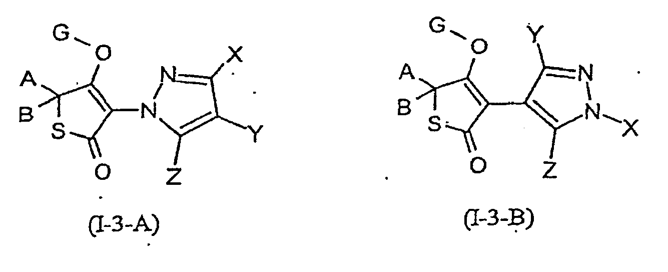

4. Compounds of the formula (I) according to Claim 1 in which
X represents phenyl which is optionally mono- or disubstituted by fluorine, chlorine, bromine, methyl, trifluoromethyl, methoxy or trifluoromethoxy,
Y represents hydrogen or methyl,
Z represents methyl, ethyl or propyl,
Het represents one of the groups 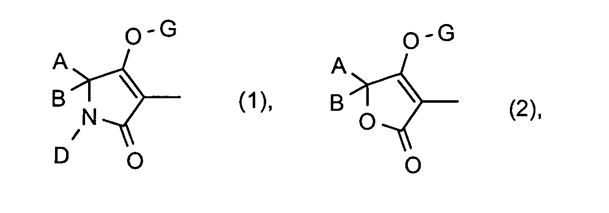
A represents hydrogen or C₁-C₆-alkyl,
B represents hydrogen or C₁-C₆-alkyl, or
A, B and the carbon atom to which they are attached represent saturated C₅-C₆-cycloalkyl in which optionally one ring member is replaced by oxygen or sulphur and which is optionally monosubstituted by methyl, ethyl, propyl, isopropyl, trifluoromethyl, methoxy, ethoxy, propoxy, isopropoxy, butoxy or isobutoxy, or
A, B and the carbon atom to which they are attached represent C₅-C₆-cycloalkyl or C₅-C₆-cycloalkenyl in which two substituents together with the carbon atoms to which they are attached represent butadienyl,
D represents hydrogen, methyl, ethyl or isopropyl or represents trifluoromethyl-substituted cyclohexyl,
A and D together represent C₃-C₄-alkanediyl,
G represents hydrogen (a) or represents one of the groups 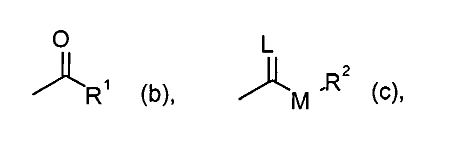 in which
L represents oxygen and
M represents oxygen or sulphur,
R¹ represents C₁-C₆-alkyl, C₂-C₆-alkenyl, C₁-C₂-alkoxy-C₁-C₂-alkyl, C₁-C₂-alkylthio-C₁-C₂-alkyl, each of which is optionally mono- to trisubstituted by fluorine or chlorine, or represents C₃-C₆-cycloalkyl which is optionally monosubstituted by fluorine, chlorine, methyl, ethyl or methoxy and in which optionally one ring member is replaced by oxygen or sulphur,
represents phenyl which is optionally monosubstituted by fluorine, chlorine, bromine, cyano, nitro, methyl, ethyl, n-propyl, i-propyl, tert-butyl, methoxy, ethoxy, i-propoxy, tert-butoxy, trifluoromethyl or trifluoromethoxy,
represents thienyl or pyridyl, each of which is optionally monosubstituted by fluorine, chlorine, bromine, methyl or ethyl,
R² represents C₁-C₆-alkyl, C₂-C₆-alkenyl or C₁-C₄-alkoxy-C₂-C₃-alkyl, each of which is optionally mono- to trisubstituted by fluorine, represents cyclopentyl or cyclohexyl,
or represents phenyl or benzyl, each of which is optionally monosubstituted by fluorine, chlorine, cyano, nitro, methyl, ethyl, n-propyl, i-propyl, methoxy, ethoxy, trifluoromethyl or trifluoromethoxy,
the point of attachment on the pyrazolyl radical being established on the following structures:

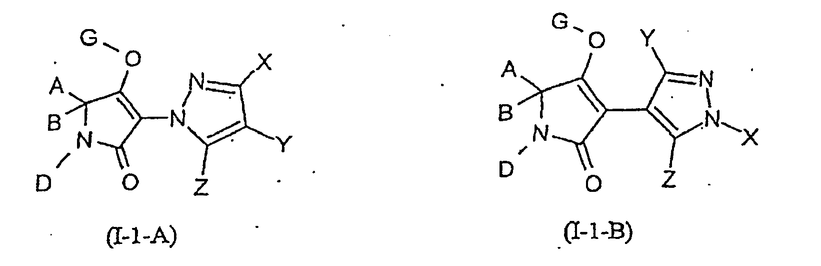

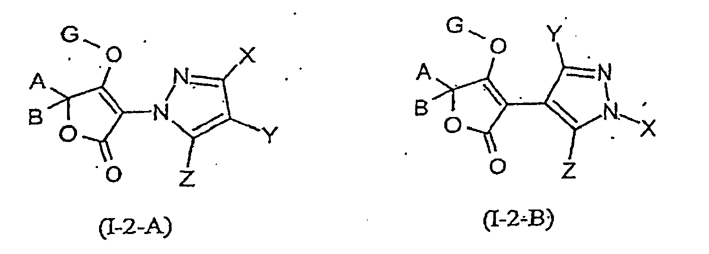

5. Compounds of the formula (I) according to Claim 1 in which the pyrazole radical has the following point of attachment: 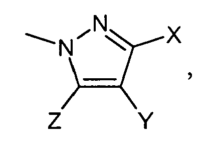 and
X represents phenyl which is optionally mono- or disubstituted by chlorine, bromine or trifluoromethyl,
Y represents hydrogen,
Z represents methyl, ethyl or propyl,
Het represents one of the groups 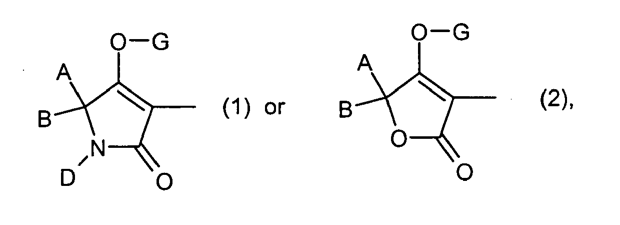
A represents hydrogen or C₁-C₆-alkyl,
B represents hydrogen or C₁-C₆-alkyl,
A, B and the carbon atom to which they are attached represent saturated C₆-cycloalkyl in which optionally one ring member is replaced by oxygen or represent saturated C₆-cycloalkyl which is optionally monosubstituted by methyl, trifluoromethyl, methoxy or ethoxy,
A, B and the carbon atom to which they are attached represent C₅-C₆-cycloalkyl in which two substituents together with the carbon atoms to which they are attached represent butadienyl,
D represents hydrogen or represents cyclohexyl which is monosubstituted by trifluoromethyl,
A and D represent C₃-C₄-alkanediyl,
G represents hydrogen (a) or represents one of the groups 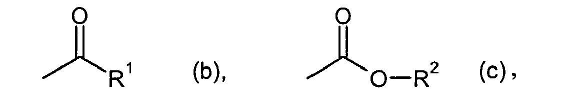 in which
R¹ represents C₁-C₆-alkyl, C₃-cycloalkyl, represents in each case chlorine-substituted phenyl or pyridyl,
R² represents C₁-C₆-alkyl or benzyl.

6. Compounds of the formula (I) according to Claim 1 in which the pyrazole radical has the following point of attachment: 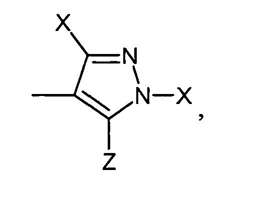 and
X represents phenyl which is monosubstituted by chlorine or trifluoromethyl,
Y represents methyl,
Z represents methyl,
Het represents the group 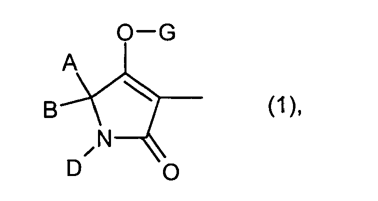
A represents methyl,
B represents methyl,
A, B and the carbon atom to which they are attached represent C₆-cycloalkyl which is monosubstituted by methoxy,
D represents hydrogen,
G represents hydrogen or the group 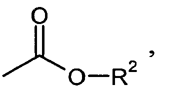
R² represents C₁-C₆-alkyl.

7. Process for preparing compounds of the formula (I) according to Claim 1, **characterized in that**, to obtain
(A) 3-pyrazolylpyrrolidine-2,4-diones or their enols of the formulae (I-1-A-a) and (I-1-B-a) 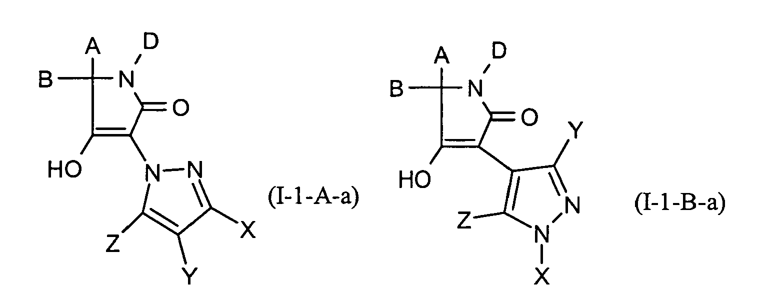 in which
A, B, D, X, Y and Z are as defined above,
N-acylamino acid esters of the formulae (II-A) and (II-B) 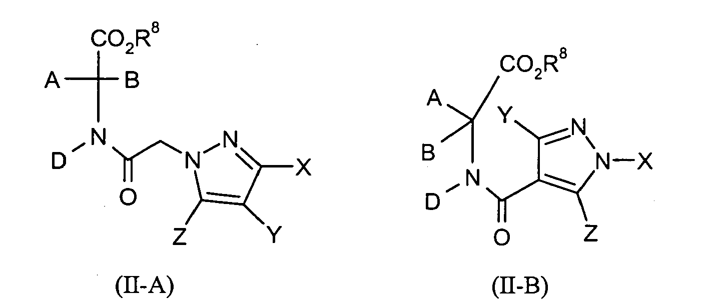 in which
A, B, D, X, Y and Z are as defined above
and
R⁸ represents alkyl
are condensed intramolecularly in the presence of a diluent and in the presence of a base,
(B) substituted 3-pyrazolyl-4-hydroxy-Δ³-dihydrofuranone derivatives of the formulae (I-2-A-a) and (I-2-B-a) 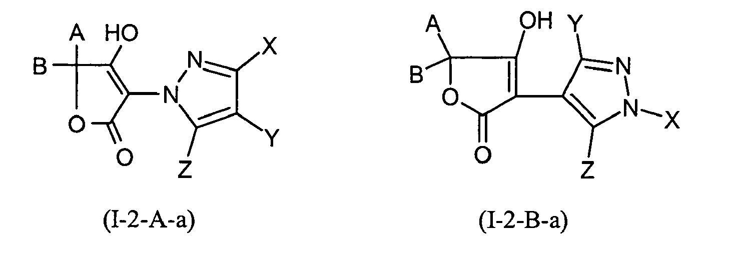 in which
A, B, X, Y and Z are as defined above,
carboxylic acid esters of the formulae (III-A) and (III-B) 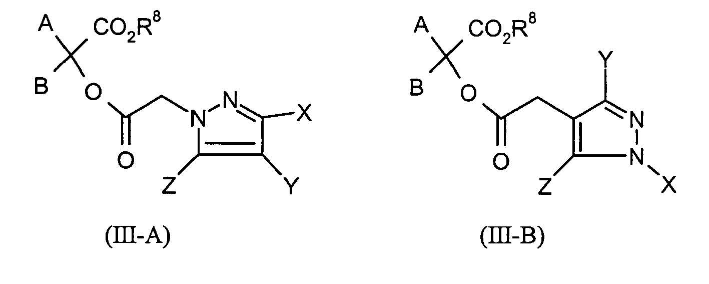 in which
A, B, X, Y, Z and R⁸ are as defined above
are condensed intramolecularly in the presence of a diluent and in the presence of a base,
(C) substituted 3-thiazolyl-4-hydroxy-Δ³-dihydrothiophen-2-one derivatives of the formulae (I-3-A-a) and (I-3-B-a) 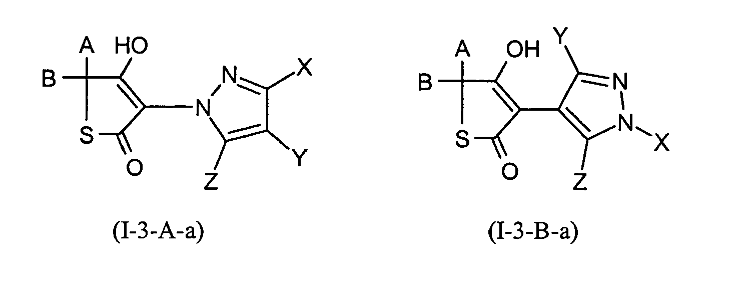 in which
A, B, X, Y and Z are as defined above
β-ketocarboxylic acid esters of the formulae (IV-A) and (IV-B) 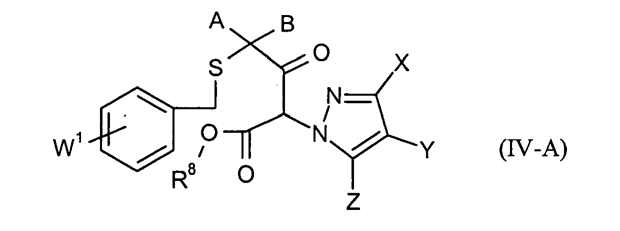 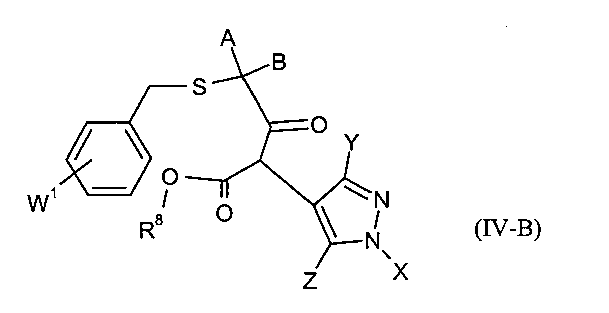 in which
A, B, X, Y, Z and R⁸ are as defined above and
W¹ represents hydrogen, halogen, alkyl or alkoxy,
are cyclized intramolecularly, if appropriate in the presence of a diluent and in the presence of an acid,
D) compounds of the formulae (I-1-A-b) to (I-3-B-b) shown above in which A, B, D, R¹, X, Y and Z are as defined above, compounds of the formulae (I-1-A-a) to (I-3-B-a) shown above in which A, B, D, X, Y and Z are as defined above are in each case reacted
(α) with acid halides of the formula (V) 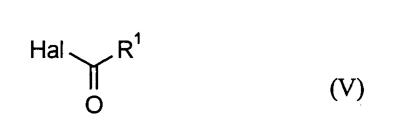 in which
R¹ is as defined above and
Hal represents halogen
or
(β) with carboxylic anhydrides of the formula (VI)
R¹-CO-O-CO-R¹ (VI)
in which
R¹ is as defined above,
if appropriate in the presence of a diluent and if appropriate in the presence of an acid binder;
(E) compounds of the formulae (I-1-A-c) to (I-3-B-c) shown above in which A, B, D, R², M, X, Y and Z are as defined above and L represents oxygen, compounds of the formulae (I-1-A-a) to (I-3-B-a) shown above in which A, B, D, X, Y and Z are as defined above are in each case reacted
with chloroformic acid esters or chloroformic acid thioesters of the formula (VII)
R²-M-CO-Cl (VII)
in which
R² and M are as defined above,
if appropriate in the presence of a diluent and if appropriate in the presence of an acid binder;
(F) compounds of the formulae (I-1-A-c) to (I-3-B-c) shown above in which A, B, D, R², M, X, Y and Z are as defined above and L represents sulphur, compounds of the formulae (I-1-A-a) to (1-3-B-a) shown above in which A, B, D, X, Y and Z are as defined above are in each case reacted
with chloromonothioformic acid esters or chlorodithioformic acid esters of the formula (VIII) 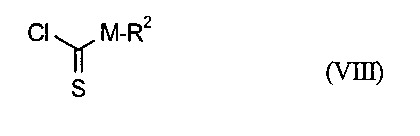 in which
M and R² are as defined above,
if appropriate in the presence of a diluent and if appropriate in the presence of an acid binder;
(G) compounds of the formulae (I-1-A-d) to (I-3-B-d) shown above in which A, B, D, R³, X, Y and Z are as defined above, compounds of the formulae (I-1-A-a) to (I-3-B-a) shown above in which A, B, D, X, Y and Z are as defined above are in each case reacted
with sulphonyl chlorides of the formula (IX)
R³-SO₂-Cl (IX)
in which
R³ is as defined above,
if appropriate in the presence of a diluent and if appropriate in the presence of an acid binder,
(H) compounds of the formulae (I-1-A-e) to (I-3-B-e) shown above in which A, B, D, L, R⁴, R⁵, X, Y and Z are as defined above, compounds of the formulae (I-1-A-a) to (I-3-B-a) shown above in which A, B, D, X, Y and Z are as defined above are in each case reacted with phosphorus compounds of the formula (X) 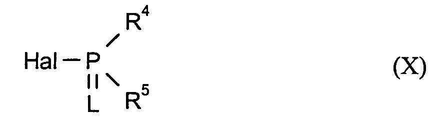 in which
L, R⁴ and R⁵ are as defined above and
Hal represents halogen,
if appropriate in the presence of a diluent and if appropriate in the presence of an acid binder,
(I) compounds of the formulae (I-1-A-f) to (I-3-B-f) shown above in which A, B, D, E, X, Y and Z are as defined above, compounds of the formulae (I-1-A-a) to (I-3-B-a) shown above in which A, B, D, X, Y and Z are as defined above are in each case reacted
with metal compounds or amines of the formulae (XI) and (XII), respectively,
Me(OR¹⁰)ₜ (XI) 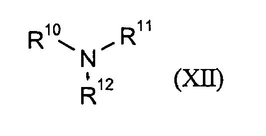
in which
Me represents a mono- or divalent metal,
t represents the number 1 or 2 and
R¹⁰, R¹¹, R¹² independently of one another represent hydrogen or alkyl,
if appropriate in the presence of a diluent,
(J) compounds of the formulae (I-1-A-g) to (I-3-B-g) shown above in which A, B, D, L, R⁶, R⁷, X, Y and Z are as defined above, compounds of the formulae (I-1-A-a) to (I-3-B-a) shown above in which A, B, D, X, Y and Z are as defined above are in each case reacted
(α) with isocyanates or isothiocyanates of the formula (XIII)
R⁶-N=C=L (XIII)
in which
R⁶ and L are as defined above,
if appropriate in the presence of a diluent and if appropriate in the presence of a catalyst, or
(β) with carbamoyl chlorides or thiocarbamoyl chlorides of the formula (XIV) 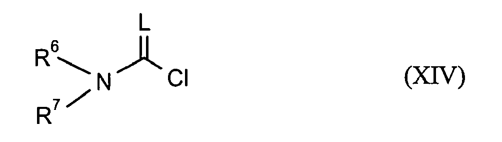
in which
L, R⁶ and R⁷ are as defined above,
if appropriate in the presence of a diluent and if appropriate in the presence of an acid binder.

8. Compounds of the formulae (II-A) and (II-B) 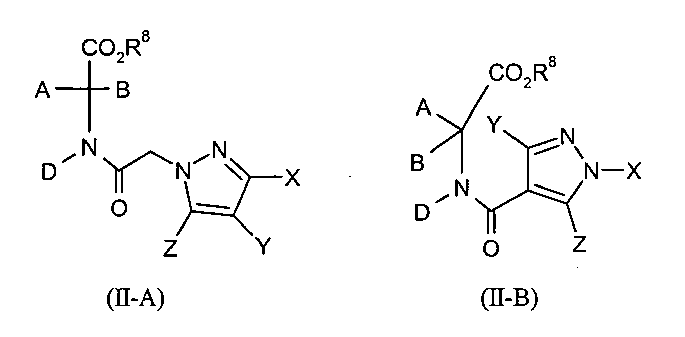 in which
A, B, D, X, Y and Z are as defined above and
R⁸ represents alkyl.

9. Compounds of the formulae (III-A) and (III-B) 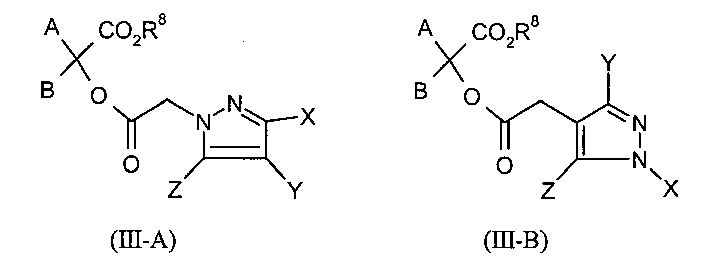 in which
A, B, X, Y, Z and R⁸ are as defined above.

10. Compounds of the formula (IV-A) and (IV-B) 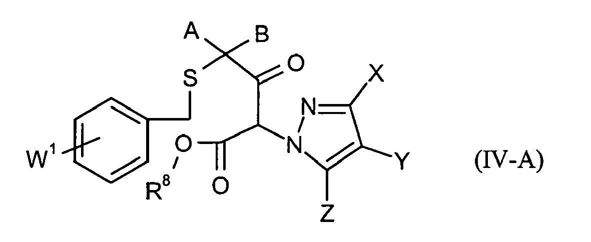 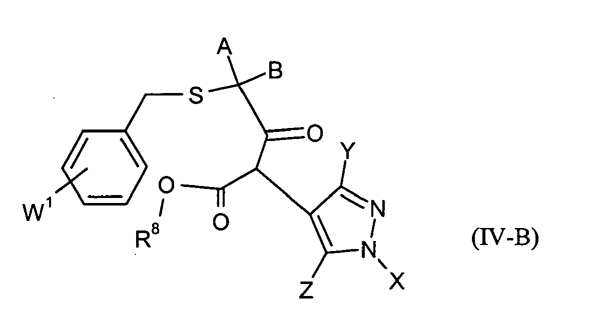 in which
A, B, X, Y, Z and R⁸ are as defined above and
W¹ represents hydrogen, halogen, alkyl or alkoxy.

11. Compounds of the formulae (XIX-A) and (XIX-B) 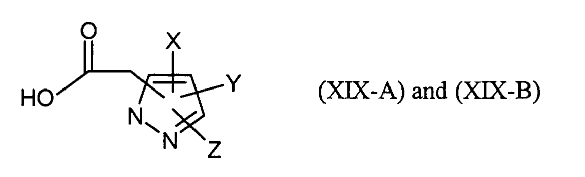 in which
X, Y and Z are as defined above, wherein in the case of the compounds of the formula (XIX-B) Y only represents hydrogen and with the exception of the compound

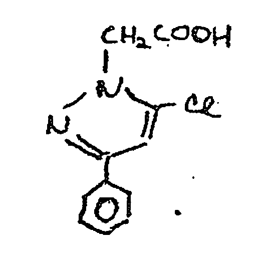

12. Pesticides, microbicides and herbicides, **characterized in that** they comprise at least one compound of the formula (I) according to Claim 1.

13. Method for controlling animal pests, unwanted vegetation and fungi, **characterized in that** compounds of the formula (I) according to Claim 1 are allowed to act on pests, unwanted plants, fungi and/or their habitat.

14. Use of compounds of the formula (I) according to Claim 1 for controlling animal pests, unwanted vegetation and fungi.

15. Process for preparing pesticides, microbicides and herbicides, **characterized in that** compounds of the formula (I) according to Claim 1 are mixed with extenders and/or surfactants.

16. Use of compounds of the formula (I) according to Claim 1 for preparing pesticides, microbicides and herbicides.

## Revendications

1. Composés de formule (I) 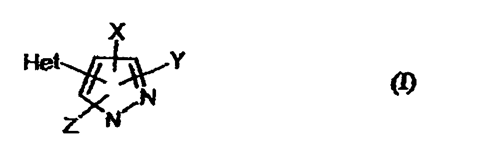 dans laquelle
X représente un reste phényle éventuellement substitué par un halogène, un radical alkyle en C₁ à C₆, alkoxy en C₁ à C₆, halogénalkyle en C₁ à C₄, halogénalkoxy en C₁ à C₄, nitro ou cyano,
Y représente l'hydrogène, un halogène ou un reste alkyle en C₁ à C₆,
Z représente un reste alkyle en C₁ à C₆, un halogène, un groupe hydroxy, un reste alkoxy en C₁ à C₆, halogénalkoxy en C₁ à C₆ ou un reste phényl-(alkoxy en C₁ ou C₂) éventuellement substitué par un radical alkyle en C₁ à C₆, alkoxy en C₁ à C₆, halogéno, halogénalkyle en C₁ à C₄, halogénalkoxy en C₁ à C₆, cyano ou nitro
ou bien un reste cycloalkyle en C₃ à C₆ éventuellement substitué par un radical alkyle en C₁ ou C₂ ou halogéno,
Het représente l'un des groupes 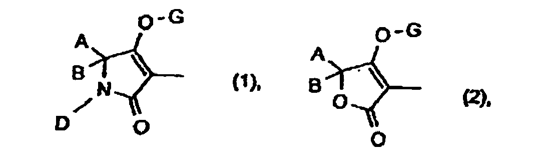 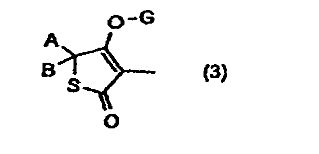
A représente l'hydrogène ou un reste alkyle en C₁ à C₁₂, alcényle en C₃ à C₈, (alkoxy en C₁ à C₁₀) - (alkyle en C₁ à C₈), poly(alkoxy en C₁ à C₈)-(alkyle en C₁ à C₈), (alkylthio en C₁ à C₁₀) - (alkyle en C₁ à C₆), chacun éventuellement substitué par du fluor ou par du chlore, un reste cycloalkyle en C₃ à C₈ éventuellement substitué par du fluor, du chlore, un radical alkyle en C₁ à C₆ ou alkoxy en C₁ à C₆, dans le noyau duquel, le cas échéant, un ou deux chaînons non directement contigus sont remplacés par de l'oxygène et/ou par du soufre, ou bien un reste aryle en C₆ ou C₁₀ ou un reste (aryle en C₆ ou C₁₀) - (alkyle en C₁ à C₆) chacun éventuellement substitué par un radical halogéno, alkyle en C₁ à C₆, halogénalkyle en C₁ à C₆, alkoxy en C₁ à C₆, halogénalkoxy en C₁ à C₆, cyano ou nitro,
B représente l'hydrogène, un reste alkyle en C₁ à C₁₂ ou (alkoxy en C₁ à C₈)-(alkyle en C₁ à C₆),
ou
A, B et l'atome de carbone auquel ils sont liés représentent un reste cycloalkyle en C₃ à C₁₀ saturé ou un reste cycloalkyle en C₅ à C₁₀ non saturé, où le cas échéant un chaînon est remplacé par de l'oxygène ou par du soufre et qui sont éventuellement substitués une ou deux fois par un radical alkyle en C₁ à C₈, cycloalkyle en C₃ à C₁₀, halogénalkyle en C₁ à C₈, alkoxy en C₁ à C₈, alkylthio en C₁ à C₈, halogéno ou phényle, ou bien
A, B et l'atome de carbone auquel ils sont liés représentent un reste cycloalkyle en C₅ ou C₆ qui est substitué par un groupe alkylènediyle comprenant éventuellement un ou deux atomes d'oxygène et/ou de soufre non directement contigus, ou par un groupe alkylènedioxyle ou par un groupe alkylènedithioyle qui forme avec l'atome de carbone auquel il est lié un autre noyau pentagonal à octogonal qui peut éventuellement être substitué par un radical alkyle en C₁ à C₄, ou bien
A, B et l'atome de carbone auquel ils sont liés représentent un reste cycloalkyle en C₃ à C₈ ou cycloalcényle en C₅ à C₈, dans lesquels deux substituants représentent, conjointement avec les atomes de carbone auxquels ils sont liés, un reste alcanediyle en C₂ à C₆, alcènediyle en C₂ à C₆ ou alcanediènediyle en C₄ à C₆, chacun éventuellement substitué par un radical alkyle en C₁ à C₆, alkoxy en C₁ à C₆ ou halogéno, où le cas échéant un groupe méthylène est remplacé par de l'oxygène ou par du soufre,
D représente l'hydrogène, un reste alkyle en C₁ à C₁₂, alcényle en C₃ à C₈, alcynyle en C₃ à C₈, (alkoxy en C₁ à C₁₀)-(alkyle en C₂ à C₈), poly(alkoxy en C₁ à C₈)-(alkyle en C₂ à C₈), (alkylthio en C₁ à C₁₀)-(alkyle en C₂ à C₈), chacun éventuellement substitué par un halogène, un reste cycloalkyle en C₃ à C₈, éventuellement substitué par un radical halogéno, alkyle en C₁ à C₄, alkoxy en C₁ à C₄ ou halogénalkyle en C₁ à C₄ et dans lequel, le cas échéant, un chaînon du noyau est remplacé par de l'oxygène ou par du soufre, ou bien un reste phényle ou phényl-(alkyle en C₁ à C₆) éventuellement substitué par un radical halogéno, alkyle en C₁ à C₆, halogénalkyle en C₁ à C₆, alkoxy en C₁ à C₆, halogénalkoxy en C₁ à C₆, cyano ou nitro, ou
A et D forment ensemble un reste alcanediyle en C₃ à C₆ ou alcènediyle en C₃ à C₆, chacun éventuellement substitué, où le cas échéant un groupe méthylène est remplacé par un groupe carbonyle, de l'oxygène ou du soufre et
les substituants suivants étant considérés dans chaque cas :
halogéno, hydroxy, mercapto ou alkyle en C₁ à C₁₀, alkoxy en C₁ à C₆, alkylthio en C₁ à C₆, cycloalkyle en C₃ à C₇, phényle ou benzyloxy, chacun éventuellement substitué par un halogène, ou bien un autre groupement alcanediyle en C₃ à C₆, alcènediyle en C₃ à C₆ ou un groupement butadiényle qui est éventuellement substitué par un radical alkyle en C₁ à C₆ ou dans lequel, le cas échéant, deux substituants contigus forment avec les atomes de carbone auxquels ils sont liés un autre cycle saturé ou non saturé pentagonal ou hexagonal (dans le cas du composé de formule (I-1), A et D forment alors conjointement avec les atomes auxquels ils sont liés, par exemple les groupes AD-1 à AD-10 mentionnés plus loin) qui peut contenir de l'oxygène ou du soufre,
G représente l'hydrogène (a) ou l'un des groupes 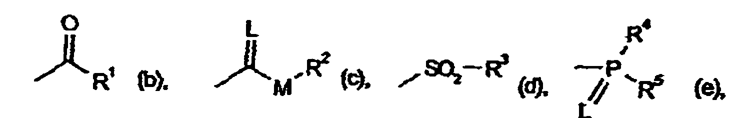 E (f) ou 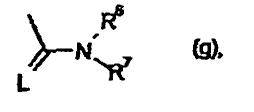 dans lesquels
E représente un équivalent d'ions métalliques ou un ion ammonium,
L représente l'oxygène ou le soufre et
M représente l'oxygène ou le soufre,
R¹ représente un reste alkyle en C₁ à C₂₀, alcényle en C₂ à C₂₀, (alkoxy en C₁ à C₈) - (alkyle en C₁ à C₈), (alkylthio en C₁ à C₈) -(alkyle en C₁ à C₈), poly(alkoxy en C₁ à C₈) - (alkyle en C₁ à C₈), chacun éventuellement substitué par un radical halogéno ou un reste cycloalkyle en C₃ à C₈ éventuellement substitué par un radical halogéno, alkyle en C₁ à C₆ ou alkoxy en C₁ à C₆, dans lequel le cas échéant ou plusieurs chaînons non directement contigus sont remplacés par de l'oxygène et/ou par du soufre
un reste phényle éventuellement substitué par un radical halogéno, cyano, nitro, alkyle en C₁ à C₆, alkoxy en C₁ à C₆, halogénalkyle en C₁ à C₆, halogénalkoxy en C₁ à C₆, alkylthio en C₁ à C₆ ou alkylsulfonyle en C₁ à C₆,
un reste phényl-(alkyle en C₁ à C₆) éventuellement substitué par un radical halogéno, nitro, cyano, alkyle en C₁ à C₆, alkoxy en C₁ à C₆, halogénalkyle en C₁ à C₆ ou halogénalkoxy en C₁ à C₆,
un reste hétaryle pentagonal ou hexagonal éventuellement substitué par un radical halogéno, alkyle en C₁ à C₆, halogénalkyle en C₁ ou C₂ ou alkoxy en C₁ à C₄,
un reste phénoxy-(alkyle en C₁ à C₆) éventuellement substitué par un radical halogéno ou alkyle en C₁ à C₆,
un reste (hétaryloxy pentagonal ou hexagonal)-(alkyle en C₁ à C₆) éventuellement substitué par un radical halogéno, amino ou alkyle en C₁ à C₆,
R² représente un reste alkyle en C₁ à C₂₀, alcényle en C₂ à C₂₀, (alkoxy en C₁ à C₈)-(alkyle en C₂ à C₈), poly(alkoxy en C₁ à C₈)-(alkyle en C₂ à C₈), chacun éventuellement substitué par un halogène,
un reste cycloalkyle en C₃ à C₈, éventuellement substitué par un radical halogéno, alkyle en C₁ à C₆ ou alkoxy en C₁ à C₆, dans lequel le cas échéant un atome du noyau est remplacé par de l'oxygène, ou bien
un reste phényle ou benzyle chacun éventuellement substitué par un radical halogéno, cyano, nitro, alkyle en C₁ à C₆, alkoxy en C₁ à C₆, halogénalkyle en C₁ à C₆ ou halogénalkoxy en C₁ à C₆,
R³ représente un reste alkyle en C₁ à C₈ éventuellement substitué par un halogène ou un reste phényle ou benzyle chacun éventuellement substitué par un radical halogéno, alkyle en C₁ à C₆, alkoxy en C₁ à C₆, halogénalkyle en C₁ à C₄, halogénalkoxy en C₁ à C₄, cyano ou nitro,
R⁴ et R⁵ représentent indépendamment l'un de l'autre un reste alkyle en C₁ à C₈, alkoxy en C₁ à C₈, alkylamino en C₁ à C₈, di(alkyle en C₁ à C₈)amino, alkylthio en C₁ à C₈, alcénylthio en C₂ à C₈, cycloalkylthio en C₃ à C₇, chacun éventuellement substitué par un halogène ou un reste phényle, phénoxy ou phénylthio, chacun éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄,
R⁶ et R⁷ représentent, indépendamment l'un de l'autre, l'hydrogène, un reste alkyle en C₁ à C₈, cycloalkyle en C₃ à C₈, alkoxy en C₁ à C₈, alcényle en C₃ à C₈, (alkoxy en C₁ à C₈)-(alkyle en C₁ à C₈), chacun éventuellement substitué par un halogène, un reste phényle éventuellement substitué par un radical halogéno, halogénalkyle en C₁ à C₈, alkyle en C₁ à C₈ ou alkoxy en C₁ à C₈, un reste benzyle éventuellement substitué par un radical halogéno, alkyle en C₁ à C₈, halogénalkyle en C₁ à C₈ ou alkoxy en C₁ à C₈, ou forment ensemble un reste alkylène en C₃ à C₆ éventuellement substitué par un radical alkyle en C₁ à C₄ et dans lequel, le cas échéant, un atome de carbone est remplacé par de l'oxygène ou par du soufre,
la position de liaison au reste pyrazolyle étant établie sur les structures suivantes :

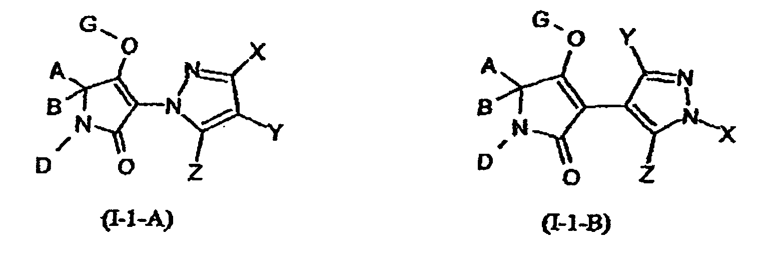

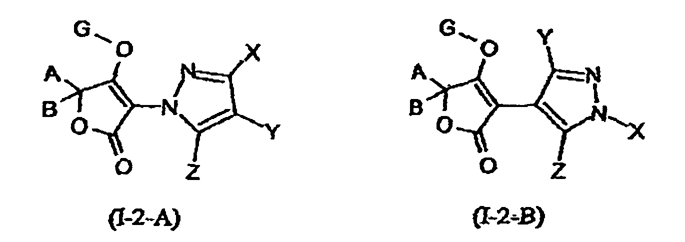

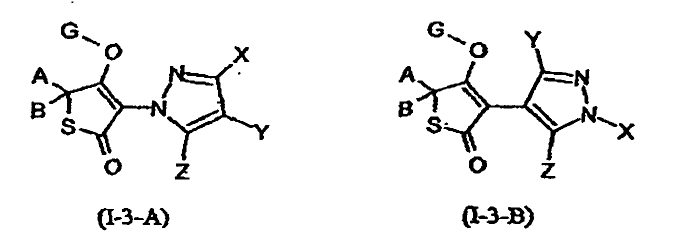

2. Composés de formule (I) suivant la revendication 1, formule dans laquelle
X représente un reste phényle éventuellement substitué une à trois fois par un radical fluoro, chloro, bromo, alkyle en C₁ à C₄, alkoxy en C₁ à C₄, halogénalkyle en C₁ ou C₂, halogénalkoxy en C₁ ou C₂, nitro ou cyano,
Y représente l'hydrogène, le chlore, le brome ou un reste alkyle en C₁ à C₄,
Z représente un reste alkyle en C₁ à C₄, le chlore, le brome, un reste alkoxy en C₁ à C₄, halogénalkoxy en C₁ à C₄, ou bien un reste benzyloxy éventuellement substitué une ou deux fois par un radical alkyle en C₁ à C₄, alkoxy en C₁ à C₄, fluoro, chloro, bromo, halogénalkyle en C₁ ou C₂, halogénalkoxy en C₁ ou C₂, cyano ou nitro
Het représente l'un des groupes 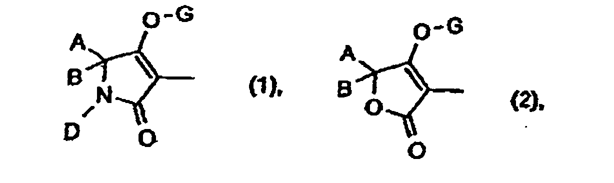 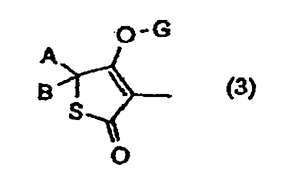
A représente l'hydrogène, un reste alkyle en C₁ à C₁₀, (alkoxy en C₁ à C₈) - (alkyle en C₁ à C₆), chacun éventuellement substitué par du fluor, un reste cycloalkyle en C₃ à C₇ éventuellement substitué par un radical fluoro, chloro, alkyle en C₁ à C₄ ou alkoxy en C₁ à C₄, et dans lequel le cas échéant un chaînon du noyau est remplacé par de l'oxygène ou par du soufre,
ou bien un reste phényle ou phényl-(alkyle en C₁ ou C₂), chacun éventuellement substitué par un radical fluoro, chloro, bromo, alkyle en C₁ à C₄, halogénalkyle en C₁ à C₄, alkoxy en C₁ à C₄, cyano, nitro ou halogénalkoxy en C₁ à C₄,
B représente l'hydrogène ou un reste alkyle en C₁ à C₁₀, ou bien
A, B et l'atome de carbone auquel ils sont liés forment un reste cycloalkyle saturé en C₃ à C₇ ou un reste cycloalkyle non saturé en C₅ à C₇, où le cas échéant un chaînon du noyau est remplacé par de l'oxygène ou par du soufre et qui est éventuellement substitué une ou deux fois par un radical alkyle en C₁ à C₆, cycloalkyle en C₅ à C₈, halogénalkyle en C₁ à C₃, alkoxy en C₁ à C₆, fluoro, chloro ou phényle, ou bien
A, B et l'atome de carbone auquel ils sont liés représentent un reste cycloalkyle en C₅ ou C₆ qui est substitué par un groupe alkylènediyle contenant éventuellement un ou deux atomes d'oxygène ou de soufre non directement contigus ou par un groupe alkylènedioxyle ou par un groupe alkylènedithiolyle, qui forme avec l'atome de carbone auquel il est lié un autre noyau pentagonal ou hexagonal qui peut éventuellement être substitué par un radical alkyle en C₁ à C₃,
A, B et l'atome de carbone auquel ils sont liés représentent un reste cycloalkyle en C₅ ou C₆ ou cycloalcényle en C₅ ou C₆ dans lesquels deux substituants représentent, conjointement avec les atomes de carbone auxquels ils sont liés, un reste alcanediyle en C₂ à C₄ ou alcènediyle en C₂ à C₄, chacun éventuellement substitué par un radical alkyle en C₁ à C₅, alkoxy en C₁ à C₅, fluoro, chloro ou bromo, et où un groupe méthylène est éventuellement remplacé par de l'oxygène ou du soufre, ou bien un reste butadiènediyle,
D représente l'hydrogène, un reste alkyle en C₁ à C₁₀, alcényle en C₃ à C₆, (alkoxy en C₁ à C₆) - (alkyle en C₂ à C₄) ou (alkylthio en C₁ à C₆) - (alkyle en C₂ à C₄), chacun éventuellement substitué par du fluor, un reste cycloalkyle en C₃ à C₇ éventuellement substitué par un radical fluoro, alkyle en C₁ à C₄, alkoxy en C₁ à C₄, ou halogénalkyle en C₁ ou C₂, et dans lequel, le cas échéant, un groupe méthylène est remplacé par de l'oxygène ou par du soufre, ou bien un reste phényle ou phényl-(alkyle en C₁ à C₄), chacun éventuellement substitué par un radical fluoro, chloro, bromo, alkyle en C₁ à C₄, halogénalkyle en C₁ à C₄, alkoxy en C₁ à C₄ ou halogénalkoxy en C₁ à C₄, ou bien
A et D forment ensemble un reste alcanediyle en C₃ à C₅ dans lequel, le cas échéant, un groupe méthylène peut être remplacé par de l'oxygène ou du soufre, en considérant comme substituants un radical alkyle en C₁ à C₆ ou alkoxy en C₁ à C₄, ou bien
A et D représentent (dans le cas des composés de formule (I-1)), conjointement avec les atomes auxquels ils sont liés, l'un des groupes AD-1 à AD-10 : 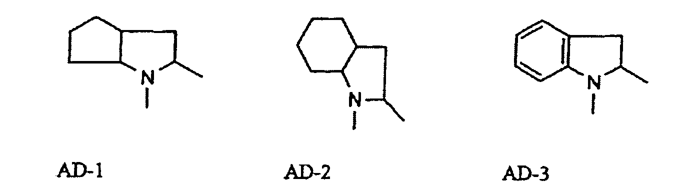 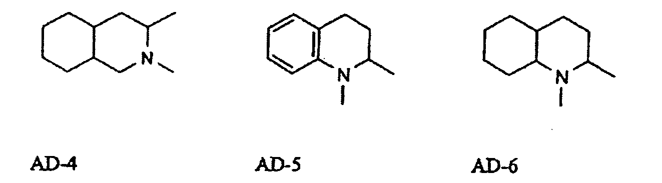 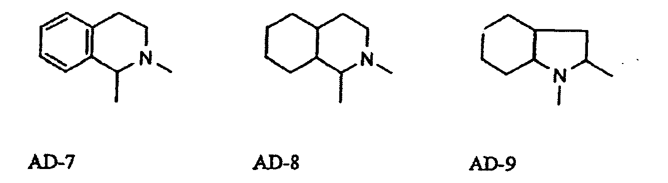 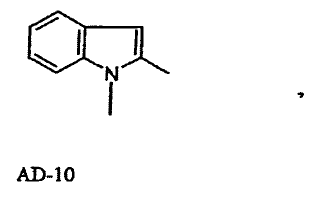
G représente l'hydrogène (a) ou l'un des groupes 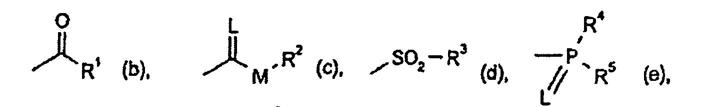 E (f) ou 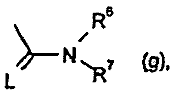 dans lesquels
E représente un équivalent d'ion métallique ou un ion ammonium,
L représente l'oxygène ou le soufre et
M représente l'oxygène ou le soufre,
R¹ représente un reste alkyle en C₁ à C₁₆, alcényle en C₂ à C₁₆, (alkoxy en C₁ à C₆) - (alkyle en C₁ à C₄), (alkylthio en C₁ à C₆) - (alkyle en C₁ à C₄), chacun éventuellement substitué par du fluor ou par du chlore, ou bien un reste cycloalkyle en C₃ à C₇ éventuellement substitué par un radical fluoro, chloro, alkyle en C₁ à C₅ ou alkoxy en C₁ à C₅, dans le noyau duquel un ou deux chaînons non directement contigus peuvent être remplacés par de l'oxygène et/ou du soufre,
un reste phényle éventuellement substitué par un radical fluoro, chloro, bromo, cyano, nitro, alkyle en C₁ à C₄, alkoxy en C₁ à C₄, halogénalkyle en C₁ à C₃, halogénalkoxy en C₁ à C₃, alkylthio en C₁ à C₄ ou alkylsulfonyle en C₁ à C₄, un reste phényl-(alkyle en C₁ à C₄) éventuellement substitué par un radical fluoro, chloro, bromo, alkyle en C₁ à C₄, alkoxy en C₁ à C₄, halogénalkyle en C₁ à C₃
ou halogénalkoxy en C₁ à C₃,
un reste pyrazolyle, thiazolyle, pyridyle, pyrimidyle, furannyle ou thiényle, chacun éventuellement substitué par un radical fluoro, chloro, bromo, alkyle en C₁ à C₄, trifluorométhyle ou alkoxy en C₁ ou C₂,
R² représente un reste alkyle en C₁ à C₁₆, alcényle en C₂ à C₁₆ ou (alkoxy en C₁ à C₆) - (alkyle en C₂ à C₆), chacun éventuellement substitué par du fluor,
un reste cycloalkyle en C₃ à C₇ éventuellement substitué par un radical fluoro, chloro, alkyle en C₁ à C₄ ou alkoxy en C₁ à C₄, ou bien
un reste phényle ou benzyle, chacun éventuellement substitué par un radical fluoro, chloro, bromo, cyano, nitro, alkyle en C₁ à C₄, alkoxy en C₁ à C₃, halogénalkyle en C₁ ou C₂ ou halogénalkoxy en C₁ ou C₂,
R³ représente un reste alkyle en C₁ à C₆ éventuellement substitué par du fluor ou un reste phényle éventuellement substitué par un radical fluoro, chloro, bromo, alkyle en C₁ à C₄, alkoxy en C₁ à C₄, halogénalkyle en C₁ à C₃, halogénalkoxy en C₁ à C₃, cyano ou nitro,
R⁴ représente un reste alkyle en C₁ à C₆, alkoxy en C₁ à C₆, alkylamino en C₁ à C₆, di-(alkyle en C₁ à C₆)amino, alkylthio en C₁ à C₆, alcénylthio en C₃ ou C₄, cycloalkylthio en C₃ à C₆ ou un reste phényle, phénoxy ou phénylthio, chacun éventuellement substitué par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃,
R⁵ représente un reste alkoxy en C₁ à C₆ ou alkylthio en C₁ à C₆,
R⁶ représente un reste alkyle en C₁ à C₆, cycloalkyle en C₃ à C₆, alkoxy en C₁ à C₆, alcényle en C₃ à C₆, (alkoxy en C₁ à C₆)-(alkyle en C₁ à C₆), un reste phényle éventuellement substitué par un radical fluoro, chloro, bromo, halogénalkyle en C₁ à C₃, alkyle en C₁ à C₄ ou alkoxy en C₁ à C₄, un reste benzyle éventuellement substitué par un radical fluoro, chloro, bromo, alkyle en C₁ à C₄, halogénalkyle en C₁ à C₃ ou alkoxy en C₁ à C₄,
R⁷ représente l'hydrogène, un reste alkyle en C₁ à C₆, alcényle en C₃ à C₆,
R⁶ et R⁷ forment ensemble un reste alkylène en C₄ ou C₅ éventuellement substitué par un radical méthyle ou éthyle et dans lequel, le cas échéant, un groupe méthylène est remplacé par de l'oxygène ou du soufre,
la position de liaison au reste pyrazolyle étant établie sur les structures suivantes :

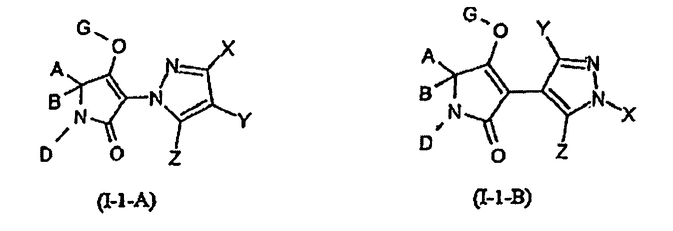

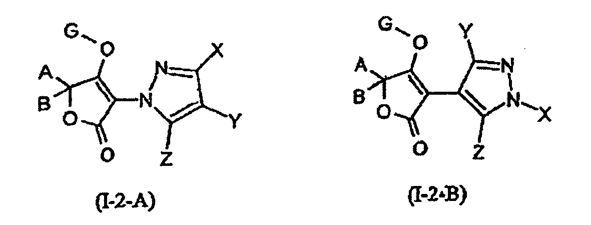

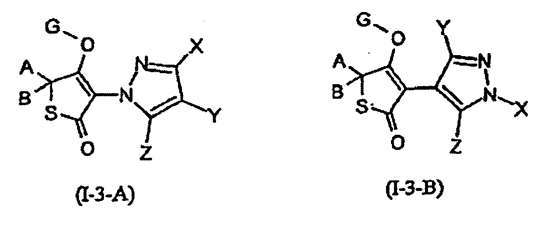

3. Composés de formule (I) suivant la revendication 1, formule dans laquelle
X représente un reste phényle éventuellement substitué une ou deux fois par un radical fluoro, chloro, bromo, méthyle, éthyle, trifluorométhyle, isopropyle, tertiobutyle, trifluorométhoxy, méthoxy, éthoxy, isopropoxy, tertiobutoxy, cyano ou nitro,
Y représente l'hydrogène, un reste méthyle ou éthyle,
Z représente un reste méthyle, éthyle, propyle, isopropyle, chloro, méthoxy, éthoxy, propoxy, isopropoxy, difluorométhoxy ou trifluoréthoxy,
Het représente l'un des groupes 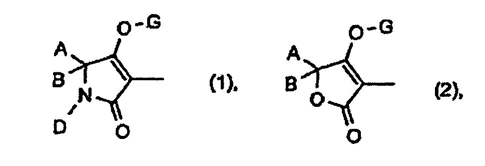 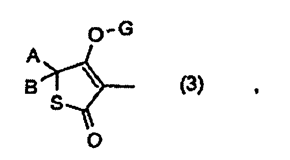
A représente l'hydrogène, un reste alkyle en C₁ à C₈ ou (alkoxy en C₁ à C₆) - (alkyle en C₁ à C₄), chacun éventuellement substitué par du fluor, un reste cycloalkyle en C₃ à C₆ éventuellement substitué par un radical fluoro, méthyle, éthyle ou méthoxy, et dans lequel le cas échéant un chaînon du noyau est remplacé par de l'oxygène ou par du soufre, ou bien un reste phényle ou benzyle, chacun substitué une ou deux fois par un radical fluoro, chloro, bromo, méthyle, éthyle, n-propyle, isopropyle, méthoxy, éthoxy, trifluorométhyle, trifluorométhoxy, cyano ou nitro,
B représente l'hydrogène ou un reste alkyle en C₁ à C₆, ou bien
A, B et l'atome de carbone auquel ils sont liés représentent un reste cycloalkyle saturé en C₃ à C₆, dans lequel le cas échéant un chaînon du noyau est remplacé par de l'oxygène ou par du soufre et qui est éventuellement substitué une fois par un radical méthyle, éthyle, propyle, isopropyle, butyle, isobutyle, sec.-butyle, tertiobutyle, trifluorométhyle, méthoxy, éthoxy, propoxy, isopropoxy, butoxy, isobutoxy, sec.-butoxy, tertiobutoxy, fluoro ou chloro, ou bien
A, B et l'atome de carbone auquel ils sont liés représentent un reste cycloalkyle en C₆ qui est substitué par un groupe alkylènedioxyle éventuellement substitué une ou deux fois par un radical méthyle ou éthyle et qui forme avec l'atome de carbone auquel il est lié un autre noyau pentagonal ou hexagonal,
A, B et l'atome de carbone auquel ils sont liés représentent un reste cycloalkyle en C₅ ou C₆ ou cycloalcényle en C₅ ou C₆, où deux substituants représentent conjointement avec les atomes de carbone auxquels ils sont liés un reste alcanediyle en C₂ à C₄ ou alcènediyle en C₂ à C₄ où dans chaque cas, le cas échéant, un groupe méthylène est remplacé par de l'oxygène ou par du soufre, ou bien un groupe butadiènediyle,
D représente l'hydrogène, un reste alkyle en C₁ à C₈, alcényle en C₃ ou C₄, (alkoxy en C₁ à C₆)-(alkyle en C₂ à C₄), (alkylthio en C₁ à C₄)-(alkyle en C₂ à C₄) ou un reste cycloalkyle en C₃ à C₆, substitué par un radical trifluorométhyle ou fluoro, dans lequel le cas échéant un groupe méthylène est remplacé par de l'oxygène ou par du soufre, ou représente un reste phényle ou benzyle, chacun éventuellement substitué une ou deux fois par un radical fluoro, chloro, méthyle, éthyle, n-propyle, isopropyle, méthoxy, éthoxy, trifluorométhyle ou trifluorométhoxy,
ou bien
A et D forment ensemble un reste alcanediyle en C₃ ou C₄, dans lequel, le cas échéant, un atome de carbone est remplacé par du soufre et qui est éventuellement substitué une ou deux fois par un radical méthyle, éthyle, méthoxy ou éthoxy, ou bien
A et D (dans le cas des composés de formule (I-1)) forment, conjointement avec les atomes auxquels ils sont liés, l'un des groupes AD suivants : 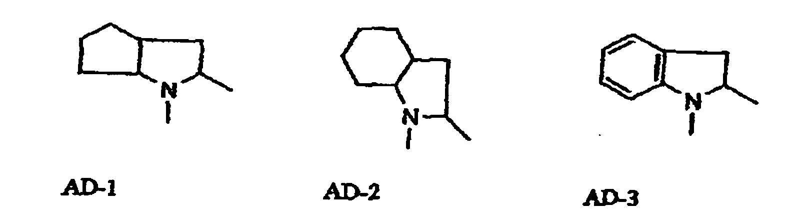 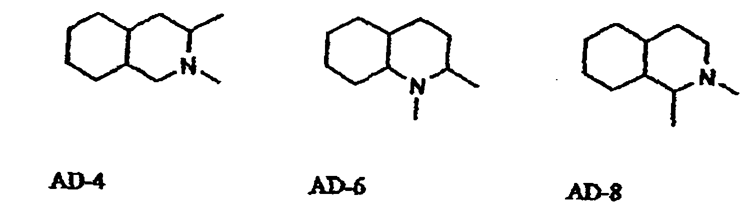 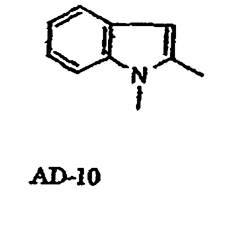
G représente l'hydrogène (a) ou l'un des groupes 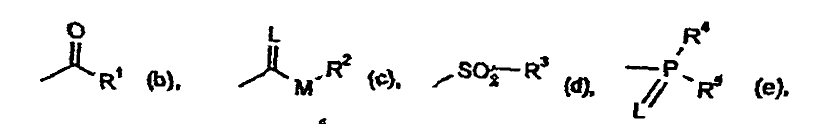 E (f) ou 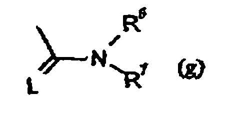 dans lesquels
E représente un équivalent d'ions métalliques ou un ion ammonium,
L représente l'oxygène ou le soufre et
M représente l'oxygène ou le soufre,
R¹ représente un reste alkyle en C₁ à C₁₄, alcényle en C₂ à C₁₄, (alkoxy en C₁ à C₄)-(alkyle en C₁ ou C₂), (alkylthio en C₁ à C₄)-(alkyle en C₁ ou C₂), chacun éventuellement substitué par du fluor ou par du chlore ou un reste cycloalkyle en C₃ à C₆, éventuellement substitué une ou deux fois par un radical fluoro, chloro, méthyle, éthyle, propyle, isopropyle, butyle, isobutyle, tertiobutyle, méthoxy ou éthoxy, dans lequel le cas échéant, un ou deux chaînons non directement contigus du noyau sont remplacés par de l'oxygène et/ou par du soufre,
un reste phényle éventuellement substitué une ou deux fois par un radical fluoro, chloro, bromo, cyano, nitro, méthyle, éthyle, n-propyle, isopropyle, tertiobutyle, méthoxy, éthoxy, isopropoxy, tertiobutoxy, trifluorométhyle ou trifluorométhoxy,
un reste furannyle, thiényle ou pyridyle, chacun éventuellement substitué une ou deux fois par du fluor, du chlore, du brome, un radical méthyle ou éthyle,
R² représente un reste alkyle en C₁ à C₁₄, alcényle en C₂ à C₁₄ ou (alkoxy en C₁ à C₄) -(alkyle en C₂ à C₆), chacun éventuellement substitué une à trois fois par du fluor,
un reste cycloalkyle en C₃ à C₆ éventuellement substitué une ou deux fois par un radical fluoro, chloro, méthyle, éthyle, n-propyle, isopropyle ou méthoxy,
ou bien un reste phényle ou benzyle, chacun éventuellement substitué une ou deux fois par un radical fluoro, chloro, cyano, nitro, méthyle, éthyle, n-propyle, isopropyle, méthoxy, éthoxy, trifluorométhyle ou trifluorométhoxy,
R³ représente un reste méthyle, éthyle, n-propyle, chacun éventuellement substitué une ou deux fois par du fluor, ou bien un reste phényle éventuellement substitué une ou deux fois par un radical fluoro, chloro, bromo, méthyle, tertiobutyle, méthoxy, trifluorométhyle, trifluorométhoxy, cyano ou nitro,
R⁴ représente un reste alkyle en C₁ à C₄, alkoxy en C₁ à C₄, alkylamino en C₁ à C₄, di(alkyle en C₁ à C₄)amino, alkylthio en C₁ à C₄ ou un reste phényle, phénoxy ou phénylthio chacun éventuellement substitué une ou deux fois par un radical fluoro, chloro, bromo, nitro, cyano, alkoxy en C₁ ou C₂, fluoralkoxy en C₁ ou C₂, alkylthio en C₁ ou C₂, fluoralkylthio en C₁ ou C₂ ou alkyle en C₁ à C₃,
R⁵ désigne un reste méthoxy, éthoxy, méthylthio ou éthylthio,
R⁶ représente un reste alkyle en C₁ à C₄, cycloalkyle en C₃ à C₆, alkoxy en C₁ à C₄, alcényle en C₃ ou C₄, (alkoxy en C₁ à C₄) - (alkyle en C₁ à C₄), un reste phényle éventuellement substitué une ou deux fois par un radical fluoro, chloro, bromo, trifluorométhyle, méthyle ou méthoxy, un reste benzyle éventuellement substitué une ou deux fois par un radical fluoro, chloro, bromo, méthyle, trifluorométhyle ou méthoxy,
R⁷ représente l'hydrogène, un reste méthyle, éthyle, propyle ou allyle,
R⁶ et R⁷ forment ensemble un reste alkylène en C₅ ou C₆ dans lequel le cas échéant un groupe méthylène est remplacé par de l'oxygène ou par du soufre,
la position de liaison au reste pyrazolyle étant établie sur les structures suivantes :

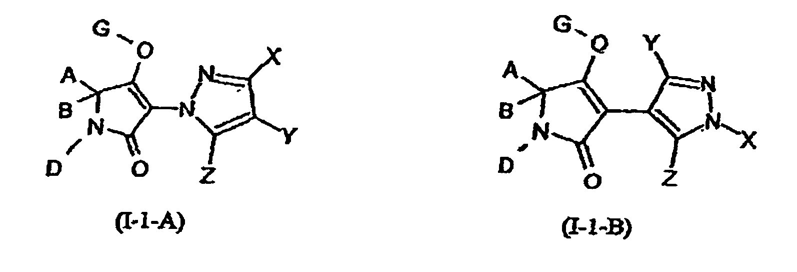

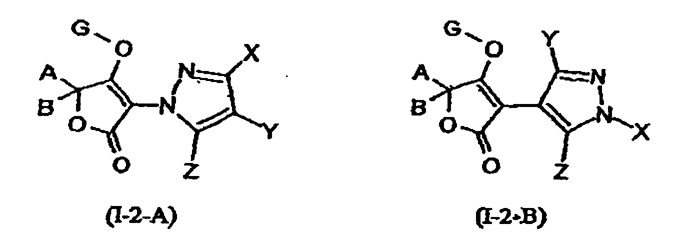

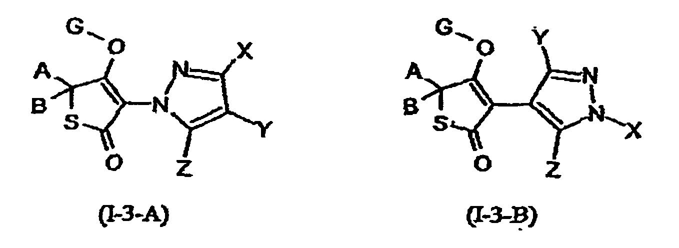

4. Composés de formule (I) suivant la revendication 1, formule dans laquelle
X représente un reste phényle éventuellement substitué une ou deux fois par un radical fluoro, chloro, bromo, méthyle, trifluorométhyle, méthoxy ou trifluorométhoxy,
Y représente l'hydrogène ou un reste méthyle,
Z représente un reste méthyle, éthyle ou propyle,
Het représente l'un des groupes 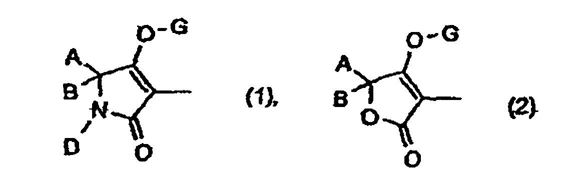
A représente l'hydrogène ou un reste alkyle en C₁ à C₆,
B représente l'hydrogène ou un reste alkyle en C₁ à C₆, ou bien
A, B et l'atome de carbone auquel ils sont liés représentent un reste cycloalkyle en C₅ ou C₆ dans lequel, le cas échéant, un chaînon du noyau est remplacé par de l'oxygène ou par du soufre et qui est éventuellement substitué une fois par un radical méthyle, éthyle, propyle, isopropyle, trifluorométhyle, méthoxy, éthoxy, propoxy, isopropoxy, butoxy ou isobutoxy, ou bien
A, B et l'atome de carbone auquel ils sont liés représentent un reste cycloalkyle en C₅ ou C₆ ou cycloalcényle en C₅ ou C₆, dans lequel deux substituants forment conjointement avec les atomes de carbone auxquels ils sont liés un reste butadiényle,
D représente l'hydrogène, un reste méthyle, éthyle ou isopropyle ou un reste cyclohexyle substitué par un radical trifluorométhyle,
A et D D forment ensemble un groupe alcanediyle en C₃ ou C₄,
G représente l'hydrogène (a) ou l'un des groupes 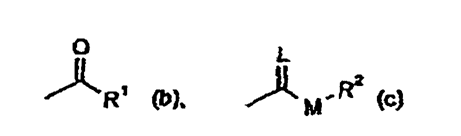 dans lesquels
L représente l'oxygène et
M représente l'oxygène ou le soufre,
R¹ représente un reste alkyle en C₁ à C₆, alcényle en C₂ à C₆, (alkoxy en C₁ ou C₂) - (alkyle en C₁ ou C₂), chacun éventuellement substitué une à trois fois par du fluor ou par du chlore, un reste (alkylthio en C₁ ou C₂)-(alkyle en C₁ ou C₂) ou un reste cycloalkyle en C₃ à C₆, éventuellement substitué par du fluor, du chlore, un radical méthyle, éthyle ou méthoxy, dans lequel le cas échéant un chaînon du noyau est remplacé par de l'oxygène ou par du soufre,
un reste phényle éventuellement substitué une fois par un radical fluoro, chloro, bromo, cyano, nitro, méthyle, éthyle, n-propyle, isopropyle, tertiobutyle, méthoxy, éthoxy, isopropoxy, tertiobutoxy, trifluorométhyle ou trifluorométhoxy,
un reste thiényle ou pyridyle, chacun éventuellement substitué une fois soit par un radical fluoro, chloro, bromo, méthyle ou éthyle,
R² représente un reste alkyle en C₁ à C₆, alcényle en C₂ à C₆ ou (alkoxy en C₁ à C₄)-(alkyle en C₂ ou C₃), chacun éventuellement substitué par du fluor, ou bien un reste cyclopentyle ou cyclohexyle,
ou bien un reste phényle ou benzyle, chacun éventuellement substitué par un radical fluoro, chloro, cyano, nitro, méthyle, éthyle, n-propyle, isopropyle, méthoxy, éthoxy, trifluorométhyle ou trifluorométhoxy,
la position de liaison au reste pyrazolyle étant établie sur les structures suivantes :

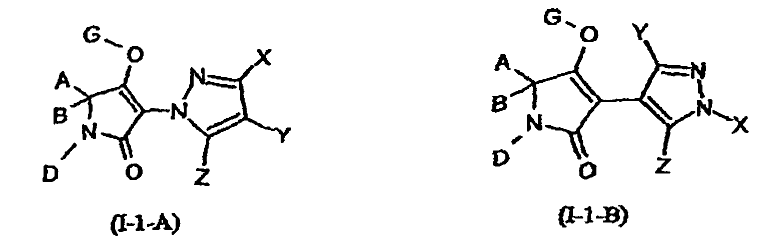

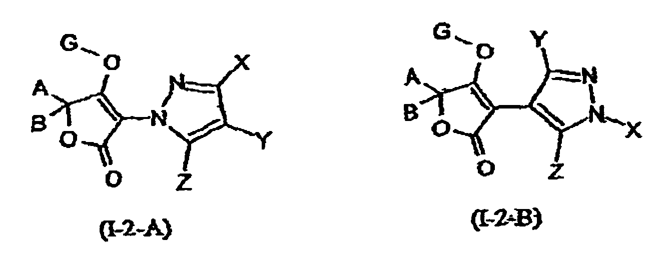

5. Composés de formule (I) suivant la revendication 1, formule dans laquelle le reste pyrazole a la position de liaison suivante : 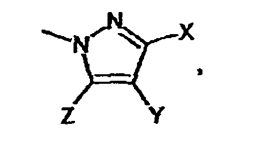 et
X représente un reste phényle éventuellement substitué une ou deux fois par un radical chloro, bromo ou trifluorométhyle,
Y représente l'hydrogène,
Z représente un reste méthyle, éthyle ou propyle,
Het représente l'un des groupes 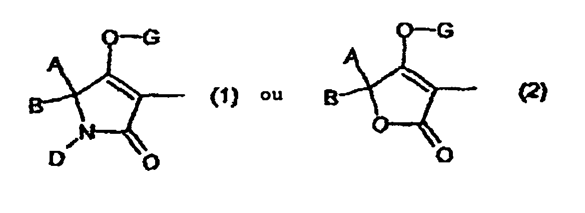
A représente l'hydrogène ou un reste alkyle en C₁ à C₆,
B représente l'hydrogène ou un reste alkyle en C₁ à C₆,
A, B et l'atome de carbone auquel ils sont liés représentent un groupe cycloalkyle en C₆ saturé dans lequel le cas échéant un chaînon du noyau est remplacé par de l'oxygène, ou bien un reste cycloalkyle en C₆ saturé qui est éventuellement substitué une fois par un radical méthyle, trifluorométhyle, méthoxy ou éthoxy,
A, B et l'atome de carbone auquel ils sont liés représentent un reste cycloalkyle en C₅ ou C₆ dans lequel deux substituants forment un groupe butadiényle conjointement aux atomes de carbone avec lesquels ils sont liés,
D représente l'hydrogène ou un reste cyclohexyle substitué une fois par un radical trifluorométhyle,
A et D représentent un reste alcanediyle en C₃ ou C₄,
G représente l'hydrogène (a) ou l'un des groupes 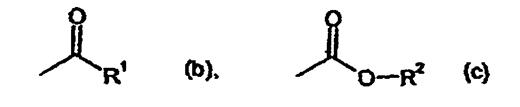 où
R¹ représente un reste alkyle en C₁ à C₆, cycloalkyle en C₃, un reste phényle ou pyridyle, chacun substitué par du chlore,
R² représente un reste alkyle en C₁ à C₆ ou benzyle.

6. Composés de formule (I) suivant la revendication 1, formule dans laquelle le reste pyrazole a la position de liaison suivante : 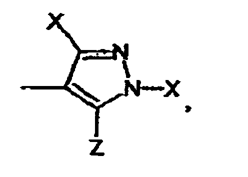 et
X représente un reste phényle substitué une fois par un radical chloro ou trifluorométhyle,
Y représente un reste méthyle,
Z représente un reste méthyle,
Het représente le groupe 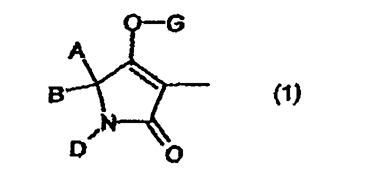
A est un reste méthyle,
B est un reste méthyle,
A, B et l'atome de carbone auquel ils sont liés forment un reste cycloalkyle en C₆ qui est substitué une fois par un radical méthoxy,
D représente l'hydrogène,
G représente l'hydrogène ou le groupe 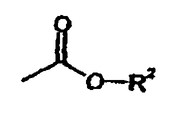
R² est un reste alkyle en C₁ à C₆.

7. Procédé de production de composés de formule (I) suivant la revendication 1, **caractérisé en ce que**
(A) pour obtenir des 3-pyrazolylpyrrolidine-2,4-diones ou leurs énols de formules (I-1-A-a) et (I-1-B-a) 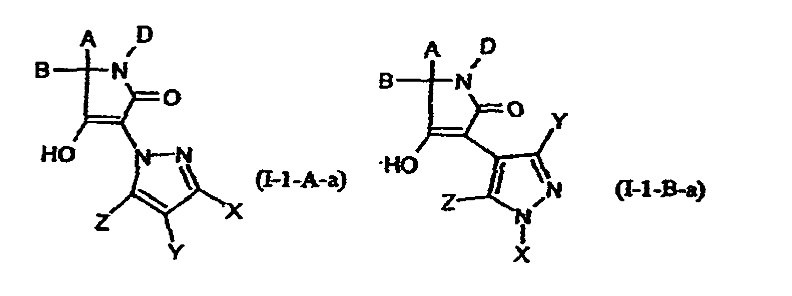 où
A, B, D, X, Y et Z ont les définitions indiquées ci-dessus,
on effectue la condensation intramoléculaire d'esters d'acides N-acylaminoacétiques de formules (II-A) et (II-B) 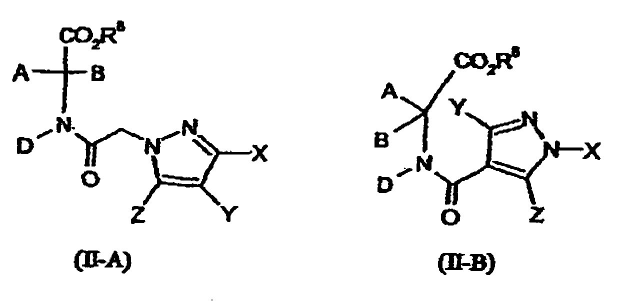 où
A, B, D, X, Y et Z ont les définitions indiquées ci-dessus,
et
R⁸ est un reste alkyle,
en présence d'un diluant et en présence d'une base,
(B) pour obtenir des dérivés substitués de 3-pyrazolyl-4-hydroxy-Δ³-dihydrofurannone de formules (I-2-A-a) et (I-2-B-a) 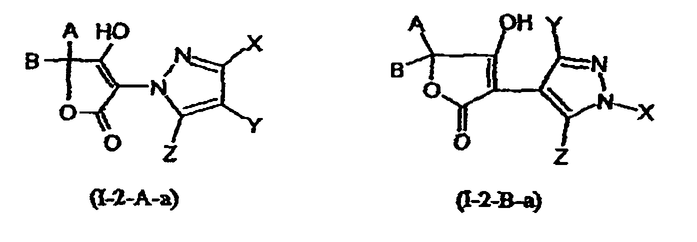 dans lesquelles
A, B, X, Y et Z ont les définitions indiquées ci-dessus,
on effectue la condensation intramoléculaire d'esters d'acides carboxyliques de formules (III-A) et (III-B) 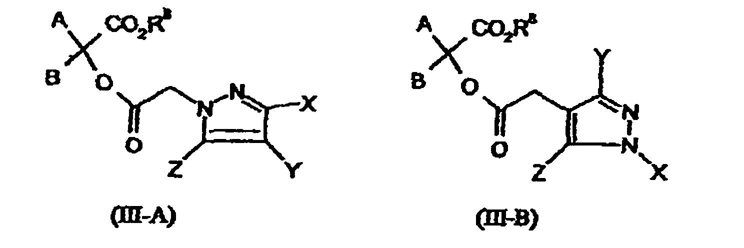 dans lesquelles
A, B, X, Y, Z et R⁸ ont les définitions indiquées ci-dessus,
en présence d'un diluant et en présence d'une base,
(C) pour obtenir des dérivés substitués de 3-thiazolyle-4-hydroxy-Δ³-dihydrothiophène-2-one de formules (I-3-A-a) et (I-3-B-a) 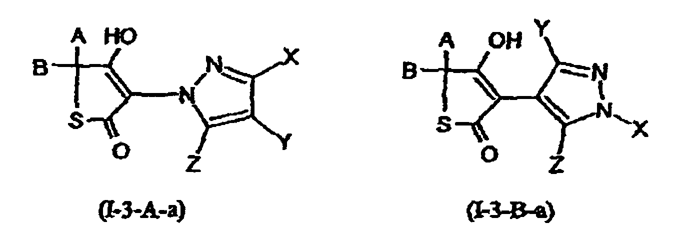 dans lesquelles
A, B, X, Y et Z ont les définitions indiquées ci-dessus
on effectue la cyclisation intramoléculaire d'esters d'acides β-cétocarboxyliques de formules (IV-A) et (IV-B) 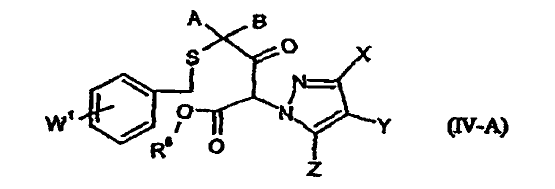 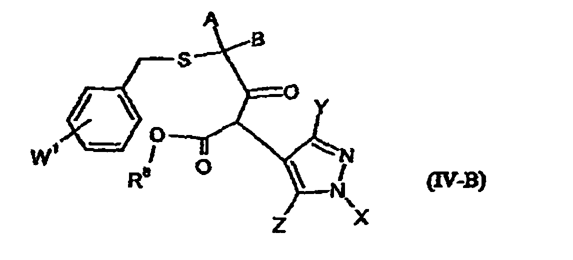 dans lesquelles
A, B, X, Y, Z et R⁸ ont les définitions indiquées ci-dessus et
W¹ représente l'hydrogène, un halogène, un reste alkyle ou alkoxy,
éventuellement en présence d'un diluant et, le cas échéant, en présence d'un acide,
D) pour obtenir des composés de formules (I-1-A-b) et (I-3-B-b) indiquées ci-dessus, dans lesquelles A, B, D, R¹, X, Y et Z ont les définitions indiquées ci-dessus, on fait réagir des composés de formules (I-1-A-a) à (I-3-B-a) indiquées ci-dessus, dans lesquelles A, B, D, X, Y et Z ont les définitions indiquées ci-dessus, dans chaque cas
(α) avec des halogénures d'acides de formule (V) 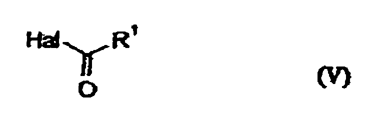 dans laquelle
R¹ a la définition indiquée ci-dessus et
Hal représente un halogène,
ou bien
(β) avec des anhydrides d'acides carboxyliques de formule (VI)
R¹-CO-O-CO-R¹ (VI)
dans laquelle
R¹ a la définition indiquée ci-dessus,
éventuellement en présence d'un diluant et, le cas échéant, en présence d'un accepteur d'acide ;
(E) on obtient des composés de formules (I-1-A-c) à (I-3-B-c) indiquées ci-dessus, dans lesquelles A, B, D, R², M, X, Y et Z ont les définitions indiquées ci-dessus et L représente l'oxygène, on fait réagir des composés de formules (I-1-A-a) à (I-3-B-a) indiquées ci-dessus,
dans lesquelles A, B, D, X, Y et Z ont les définitions indiquées ci-dessus, dans chaque cas
avec des esters d'acide chloroformique ou des thioesters d'acide chloroformique de formule (VII)
R²-M-CO-Cl (VII)
dans laquelle
R² et M ont les définitions indiquées ci-dessus,
éventuellement en présence d'un diluant et, le cas échéant, en présence d'un accepteur d'acide ;
(F) on obtient des composés de formules (I-1-A-c) à (I-3-B-c) indiquées ci-dessus, dans lesquelles A, B, D, R², M, X, Y et Z ont les définitions indiquées ci-dessus et L représente le soufre, en faisant réagir des composés de formules (I-1-A-a) à (I-3-B-a) indiquées ci-dessus, dans lesquelles A, B, D, X, Y et Z ont les définitions indiquées ci-dessus, dans chaque cas
avec des esters d'acide chloromonothioformique ou des esters d'acide chlorodithioformique de formule (VIII) 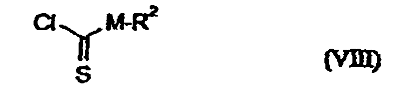 dans laquelle
M et R² ont les définitions indiquées ci-dessus,
éventuellement en présence d'un diluant et, le cas échéant, en présence d'un accepteur d'acide ;
(G) on obtient des composés de formules (I-1-A-d) à (I-3-B-d) indiquées ci-dessus, dans lesquelles A, B, D, R³, X, Y et Z ont les définitions indiquées ci-dessus, en faisant réagir dans chaque cas des composés de formules (I-1-A-a) à (I-3-B-a) indiquées ci-dessus
dans lesquelles A, B, D, X, Y et Z ont les définitions indiquées ci-dessus,
avec des chlorures d'acides sulfoniques de formule (IX)
R³-SO₂-Cl (IX)
dans laquelle
R³ a la définition indiquée ci-dessus,
éventuellement en présence d'un diluant et, le cas échéant, en présence d'un accepteur d'acide,
(H) on obtient des composés de formules (I-1-A-e) à (I-3-B-e) indiquées ci-dessus, dans lesquelles A, B, D, L, R⁴, R⁵, X, Y et Z ont les définitions indiquées ci-dessus, en faisant réagir des composés de formules (I-1-A-a) à (I-3-B-a) indiquées ci-dessus, dans lesquelles A, B, D, X, Y et Z ont les définitions indiquées ci-dessus, dans chaque cas
avec des composés phosphorés de formule (X) 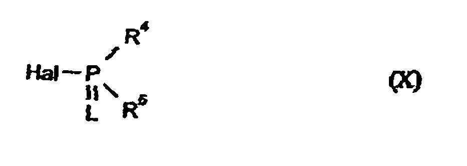 dans laquelle
L, R⁴ et R⁵ ont les définitions indiquées ci-dessus, et
Hal représente un halogène,
éventuellement en présence d'un diluant et, le cas échéant, en présence d'un accepteur d'acide,
(I) on obtient des composés de formules générales (I-1-A-f) à (I-3-B-f) indiquées ci-dessus, dans lesquelles A, B, D, E, X, Y et Z ont les définitions indiquées ci-dessus en faisant réagir des composés de formules (I-1-A-a) à (I-3-B-a) indiquées ci-dessus, dans lesquelles A, B, D, X, Y et Z ont les définitions indiquées ci-dessus, dans chaque cas,
avec des composés métalliques ou des amines de formule (XI) ou (XII)
Me(OR¹⁰)ₜ (XI) 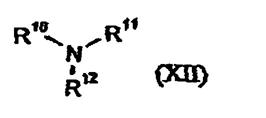
dans lesquelles
Me désigne un métal monovalent ou divalent,
t représente le nombre 1 ou 2 et
R¹⁰, R¹¹, R¹² représentent indépendamment les uns des autres l'hydrogène ou un reste alkyle,
éventuellement en présence d'un diluant,
(J) on obtient des composés de formules (I-1-A-g) à (I-3-B-g) indiquées ci-dessus, dans lesquelles A, B, D, L, R⁶, R⁷, X, Y et Z ont les définitions indiquées ci-dessus, en faisant réagir des composés de formules (I-1-A-a) à (I-3-B-a) indiquées ci-dessus, dans lesquelles A, B, D, X, Y et Z ont les définitions indiquées ci-dessus, dans chaque cas
(α) avec des isocyanates ou des isothiocyanates de formule (XIII)
R⁶-N=C=L (XIII)
dans laquelle
R⁶ et L ont les définitions indiquées ci-dessus,
éventuellement en présence d'un diluant et, le cas échéant, en présence d'un catalyseur, ou bien
(β) avec des chlorures d'acides carbamiques ou des chlorures d'acides thiocarbamiques de formule (XIV) 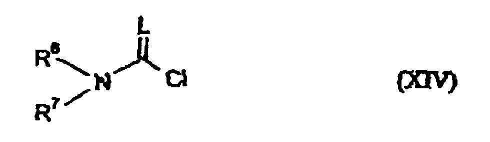 dans laquelle
L, R⁶ et R⁷ ont les définitions indiquées ci-dessus,
éventuellement en présence d'un diluant et, le cas échéant, en présence d'un accepteur d'acide.

8. Composés de formules (II-A) et (II-B) 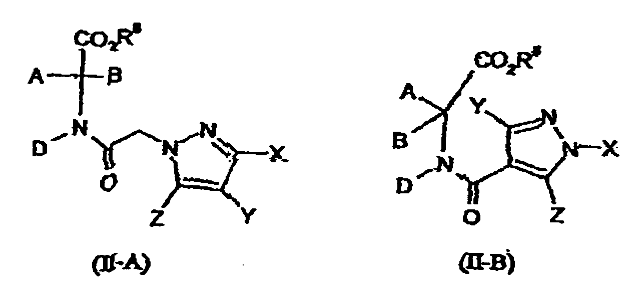 dans lesquelles
A, B, D, X, Y et Z ont les définitions indiquées ci-dessus et
R⁸ représente un reste alkyle.

9. Composés de formules (III-A) et (III-B) 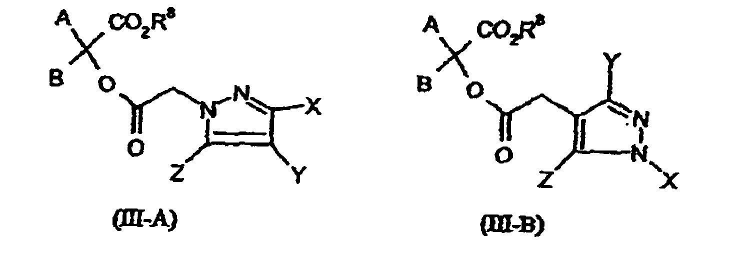 dans lesquelles
A, B, X, Y, Z et R⁸ ont les définitions indiquées ci-dessus.

10. Composés de formules (IV-A) et (IV-B) 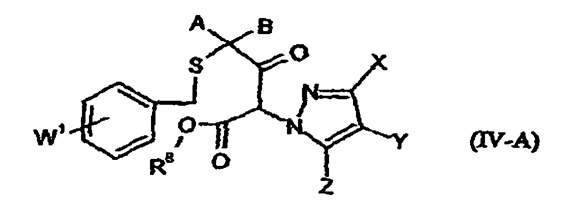 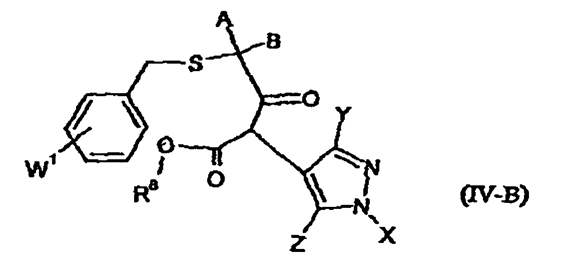 dans lesquelles
A, B, X, Y, Z et R⁸ ont les définitions indiquées ci-dessus et
W¹ représente l'hydrogène, un halogène, un reste alkyle ou alkoxy.

11. Composés de formules (XIX-A) et (XIX-B) 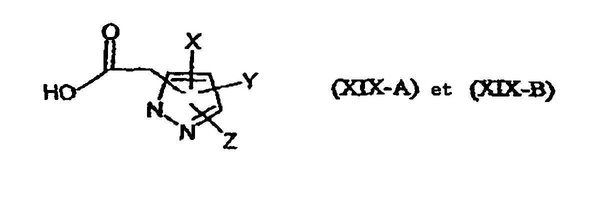 dans lesquelles
X, Y et Z ont les définitions indiquées ci-dessus, et dans le cas de composés de formule (XIX-B), Y ne représente que l'hydrogène, à l'exception du composé de formule

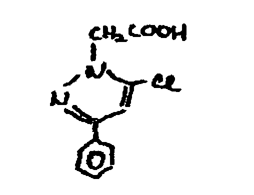

12. Compositions pesticides, microbicides et herbicides, **caractérisées par** une teneur en au moins un composé de formule (I) suivant la revendication 1.

13. Procédé pour combattre des parasites animaux, la croissance indésirable de végétaux et des champignons, **caractérisé en ce qu'**on fait agir des composés de formule (I) suivant la revendication 1 sur les parasites, la végétation indésirable et les champignons et/ou sur leur milieu.

14. Utilisation de composés de formule (I) suivant la revendication 1 pour combattre des parasites animaux, la croissance de plantes indésirables et des champignons.

15. Procédé de préparation de compositions pesticides, de microbicides et d'herbicides, **caractérisé en ce qu'**on mélange des composés de formule (I) suivant la revendication 1 avec des diluants et/ou des agents tensioactifs.

16. Utilisation de composés de formule (I) suivant la revendication 1 pour la préparation de compositions pesticides, de microbicides et d'herbicides.
